# EUROPEAN PATENT APPLICATION

(11) **EP 4 786 499 A1**
(43) Date of publication of application: **05.08.2026**
(21) Application number: 24876038.1
(22) Date of filing: 29.05.2024
(51) Int. Cl.: C07K 19/00, C12N 15/13, A61K 38/20

(54) **ANTI-PD1-IL10 FUSION PROTEIN AND USE THEREOF**

(30) Priority: 08.10.2023 CN 202311297567
(71) Applicant: Guangdong Fapon Biopharma Inc., Dongguan, Guangdong 523808 (CN)
(72) Inventor: GU, Ce, Zhuhai, Guangdong 519000 (CN); LU, Di, Zhuhai, Guangdong 519000 (CN); WU, Xiaodong, Zhuhai, Guangdong 519000 (CN); OU, Yingye, Zhuhai, Guangdong 519000 (CN); REN, Baiguang, Zhuhai, Guangdong 519000 (CN); GUO, Jian, Zhuhai, Guangdong 519000 (CN); HAN, Zhe, Zhuhai, Guangdong 519000 (CN); DING, Jiaojiao, Zhuhai, Guangdong 519000 (CN); DENG, Wentao, Zhuhai, Guangdong 519000 (CN); YE, Linhui, Zhuhai, Guangdong 519000 (CN)
(74) Representative: Fuchs Patentanwälte Partnerschaft mbB
(86) International application number: PCT/CN2024/096038
(87) International publication number: WO 2025/077212

(57) **Abstract**

The present disclosure belongs to the technical field of biology. Provided are an anti-PD1-IL10 fusion protein and the use thereof. Specifically, the present disclosure provides a fusion protein, including a PD1 binding unit and an Interleukin-10 (IL10) unit. The fusion protein has a good targeting effect, anti-tumor effect, and safety, and can be used for treating tumors or inflammatory diseases.

## Description

### Cross-Reference to Related Application

This application claims priority to Chinese Patent Application No. 202311297567.5 filed on October 8, 2023 and entitled "Anti-PD1-IL10 Fusion Protein and Use Thereof", the disclosure of which is hereby incorporated by reference in its entirety.

### Technical Field

The present disclosure relates to the technical field of biology, and specifically to an anti-PD1-IL10 fusion protein and use thereof.

### Background

The programmed cell death protein 1 (also referred to as PD1 or PD-1) is a member of the CD28 family and is expressed on activated B cells, T cells, and myeloid cells. Human PD1 is encoded by the Pdcd1 gene, located at chromosomal locus 2q37.3, has a full length of 9.6 kb, and consists of 5 exons and 4 introns, with upstream containing a 663-bp promoter. The PD1 is a 55 KDa type I transmembrane protein, a molecular structure consists of an extracellular region, a transmembrane region, and an intracellular region, the extracellular region includes an immunoglobulin variable region IgV structural domain, and the intracellular region includes an immunoreceptor tyrosine-based inhibitory motif (ITIM) and an immunoreceptor tyrosine-based switch motif (ITSM). A PD-1 extracellular region amino acid sequence has 24% homology to CTLA-4, and 28% homology to CD28. After the T cells are activated, the PD-1 mainly recruits tyrosine phosphatase SHP2 via the ITIM, leading to the dephosphorylation of downstream effector molecules. The PD-1 has two ligands: PD-L1 and PD-L2. Both the PD-L1 and PD-L2 are B7 homologues, the PDL gene is located at a 9P24.2 locus on human chromosome 9 and spans 42 kb. Its molecular structure includes an immunoglobulin-like variable structural domain, a constant region structural domain, a transmembrane region, and a short cytoplasmic tail. After the PD-1 binds to the PD-L1 and the PD-L2, the activation of the T cells may be down-regulated. The PD-L1 is expressed on the surfaces of various tumor cells. These tumor cells include lung cancer, gastric cancer, liver cancer, esophageal cancer, kidney cancer, ovarian cancer, cervical cancer, breast cancer, skin cancer, colon cancer, bladder cancer, glioma, head and neck cancer, and oral squamous cell carcinoma. Moreover, large numbers of CD8+T cells expressing the PD-1 are found in the peritumoral regions of these cancers. Clinical result statistics indicate that high levels of PD-L1 expression on the tumor cells correlate with a poor prognosis in cancer patients.

Exhausted CD8+T cells play a crucial role in the process of drug resistance during immune checkpoint therapy. Under sustained antigenic stimulation and the action of various inhibitory molecules, the T cells transition from an activated state to an exhausted state. The exhausted CD8+T cells show significantly reduced proliferative capacity, cytokine-secreting capacity, and tumor-killing ability, and thus cannot provide effective antitumor immunity. The CD8+T cells in the exhausted cannot be returned to the activated state even when treated with an anti-PD-1 antibody to block a PD-1/PD-L1 pathway.

### Summary

The present disclosure provides a fusion protein, including a PD1 binding unit portion and an Interleukin-10 (IL10) unit portion.

In some embodiments, disclosed is a fusion protein, comprising a PD1 binding unit portion and an IL10 unit portion. The PD1 binding unit portion is an anti-PD1 antibody, and the IL10 unit portion is an IL10 polypeptide. The anti-PD1 antibody includes a heavy chain variable region and a light chain variable region. The heavy chain variable region includes HCDR1, HCDR2, and HCDR3 in a variable region having at least 90% (e.g., 90%, 91%, 92%, 93%, 94%, 95%, 96%, 97%, 98%, 99%, or 100%) sequence identity to SEQ ID NO:1, 3, 5, or 7, and/or the light chain variable region comprises LCDR1, LCDR2, and LCDR3 in a variable region having at least 90% (e.g., 90%, 91%, 92%, 93%, 94%, 95%, 96%, 97%, 98%, 99%, or 100%) sequence identity to SEQ ID NO:2, 4, 6, or 8.

In some embodiments of any one of the fusion proteins described above, the anti-PD1 antibody is selected from the group consisting of the following:
**A.** an antibody comprising a heavy chain variable region comprising the HCDR1, HCDR2, and HCDR3 as set forth in SEQ ID NO:1, and a light chain variable region comprising the LCDR1, LCDR2, and LCDR3 as set forth in SEQ ID NO:2;
**B.** an antibody comprising a heavy chain variable region comprising the HCDR1, HCDR2, and HCDR3 as set forth in SEQ ID NO:3, and a light chain variable region comprising the LCDR1, LCDR2, and LCDR3 as set forth in SEQ ID NO:4;
**C.** an antibody comprising a heavy chain variable region comprising the HCDR1, HCDR2, and HCDR3 as set forth in SEQ ID NO:5, and a light chain variable region comprising the LCDR1, LCDR2, and LCDR3 as set forth in SEQ ID NO:6; or
**D.** an antibody comprising a heavy chain variable region comprising the HCDR1, HCDR2, and HCDR3 as set forth in SEQ ID NO:7, and a light chain variable region comprising the LCDR1, LCDR2, and LCDR3 as set forth in SEQ ID NO:8.

In some embodiments of any one of the fusion proteins described above, the HCDR1, HCDR2, HCDR3, LCDR1, LCDR2, and LCDR3 for the anti-PD1 antibody are defined by an IMGT numbering system, or defined by a Kabat numbering system, or defined by a Chothia numbering system, or defined by a Contact numbering system, or defined by an AbM numbering system.

In some embodiments of any one of the fusion proteins described above, the HCDR1, HCDR2, HCDR3, LCDR1, LCDR2, and LCDR3 for the anti-PD1 antibody are defined by the Kabat numbering system.

In some embodiments of any one of the fusion proteins described above, the HCDR1, HCDR2, HCDR3, LCDR1, LCDR2, and LCDR3 for the anti-PD1 antibody are defined by the IMGT numbering system.

In some embodiments of any one of the fusion proteins described above, for the anti-PD1 antibody, the HCDR1 of the heavy chain variable region of the anti-PD1 antibody includes an amino acid sequence of SEQ ID NO:9, the HCDR2 includes an amino acid sequence of SEQ ID NO:10, 15, or 16, and the HCDR3 includes an amino acid sequence of SEQ ID NO:11; and the LCDR1 of the light chain variable region includes an amino acid sequence of SEQ ID NO:12, the LCDR2 includes an amino acid sequence of SEQ ID NO:13, and the LCDR3 includes an amino acid sequence of SEQ ID NO:14.

In some embodiments of any one of the fusion proteins described above, the anti-PD1 antibody comprises the HCDR1, HCDR2, and HCDR3 as set forth in SEQ ID NO:9, SEQ ID NO:10, and SEQ ID NO:11, respectively, and the LCDR1, LCDR2, and LCDR3 as set forth in SEQ ID NO:12, SEQ ID NO:13, and SEQ ID NO:14, respectively.

In some embodiments of any one of the fusion proteins described above, the anti-PD1 antibody comprises the HCDR1, HCDR2, and HCDR3 shown as set forth in SEQ ID NO:9, SEQ ID NO:15, and SEQ ID NO:11, respectively, and the LCDR1, LCDR2, and LCDR3 as set forth in SEQ ID NO:12, SEQ ID NO:13, and SEQ ID NO:14, respectively.

In some embodiments of any one of the fusion proteins described above, the anti-PD1 antibody comprises the HCDR1, HCDR2, and HCDR3 as set forth in SEQ ID NO:9, SEQ ID NO:16, and SEQ ID NO:11, respectively, and the LCDR1, LCDR2, and LCDR3 as set forth in SEQ ID NO:12, SEQ ID NO:13, and SEQ ID NO:14, respectively.

In some embodiments of any one of the fusion proteins described above, the anti-PD1 antibody is a humanized antibody, a mouse-derived antibody, or a chimeric antibody.

In some embodiments of any one of the fusion proteins described above, the heavy chain variable region of the anti-PD1 antibody comprises an amino acid sequence having at least 90% (e.g., 90%, 91%, 92%, 93%, 94%, 95%, 96%, 97%, 98%, 99%, or 100%) sequence identity to SEQ ID NO:1, 3, 5, or 7, and/or the light chain variable region comprises an amino acid sequence having at least 90% (e.g., 90%, 91%, 92%, 93%, 94%, 95%, 96%, 97%, 98%, 99%, or 100%) sequence identity to SEQ ID NO:2, 4, 6, or 8.

In some embodiments of any one of the fusion proteins described above, the heavy chain variable region of the anti-PD1 antibody comprises an amino acid sequence having at least 90% (e.g., 90%, 91%, 92%, 93%, 94%, 95%, 96%, 97%, 98%, 99%, or 100%) sequence identity to SEQ ID NO:1, 3, 5, or 7, and the light chain variable region comprises an amino acid sequence having at least 90% (e.g., 90%, 91%, 92%, 93%, 94%, 95%, 96%, 97%, 98%, 99%, or 100%) sequence identity to SEQ ID NO:2, 4, 6, or 8.

In some embodiments of any one of the fusion proteins described above, the anti-PD1 antibody comprises the following:
(a) The heavy chain variable region comprises an amino acid sequence as set forth in SEQ ID NO:1, and the light chain variable region comprises an amino acid sequence as set forth in SEQ ID NO:2.
(b) The heavy chain variable region comprises an amino acid sequence as set forth in SEQ ID NO:3, and the light chain variable region comprises an amino acid sequence as set forth in SEQ ID NO:4.
(c) The heavy chain variable region comprises an amino acid sequence as set forth in SEQ ID NO:5, and the light chain variable region comprises an amino acid sequence as set forth in SEQ ID NO:6.
(d) The heavy chain variable region comprises an amino acid sequence as set forth in SEQ ID NO:7, and the light chain variable region comprises an amino acid sequence as set forth in SEQ ID NO:8.

In some embodiments of any one of the fusion proteins described above, the anti-PD1 antibody comprises the heavy chain variable region as set forth in SEQ ID NO:1 and the light chain variable region as set forth in SEQ ID NO:2.

In some embodiments, disclosed is a fusion protein comprising a PD1 binding unit portion and an IL10 unit portion. The PD1 binding unit is an anti-PD1 antibody, and the IL10 unit portion is an IL10 polypeptide.
(a) The anti-PD1 antibody comprises a heavy chain variable region and a light chain variable region, the heavy chain variable region has at least 95% sequence identity to SEQ ID NO:1, and the light chain variable region has at least 95% sequence identity to SEQ ID NO:2.
(b) The anti-PD1 antibody comprises a heavy chain variable region and a light chain variable region, the heavy chain variable region has at least 95% sequence identity to SEQ ID NO:3, and the light chain variable region has at least 95% sequence identity to SEQ ID NO:4.
(c) The anti-PD1 antibody comprises a heavy chain variable region and a light chain variable region, the heavy chain variable region has at least 95% sequence identity to SEQ ID NO:5, and the light chain variable region has at least 95% sequence identity to SEQ ID NO:6.
(d) The anti-PD1 antibody comprises a heavy chain variable region and a light chain variable region, the heavy chain variable region has at least 95% sequence identity to SEQ ID NO:7, and the light chain variable region has at least 95% sequence identity to SEQ ID NO:8.
(e) The anti-PD1 antibody competitively binds to a PD1 antigen or binds to the same epitope as the anti-PD1 antibody described in (a), (b), (c), or (d).

In some embodiments of any one of the fusion proteins described above, the anti-PD1 antibody comprises the heavy chain variable region as set forth in SEQ ID NO:1 and the light chain variable region as set forth in SEQ ID NO:2.

In some embodiments of any one of the fusion proteins described above, the anti-PD1 antibody comprises a constant region.

In some embodiments of any one of the fusion proteins described above, for the anti-PD1 antibody, a heavy chain constant region of the antibody is selected from the group consisting of heavy chain constant regions of IgG1, IgG2, IgG3, and IgG4, and/or a light chain constant region of the antibody is selected from the group consisting of a κ or λ chain constant region.

In some embodiments of any one of the fusion proteins described above, for the anti-PD1 antibody, the species of the constant region is derived from mouse or human.

In some embodiments of any one of the fusion proteins described above, for the anti-PD1 antibody, the heavy chain constant region is a mouse IgG2a constant region, a mouse IgG1 constant region, a human IgG1 constant region, or a human IgG4 constant region, and/or the light chain constant region is a mouse κ constant region or a human κ constant region.

In some embodiments of any one of the fusion proteins described above, for the anti-PD1 antibody, the heavy chain constant region comprises an amino acid sequence having at least 90% (e.g., 90%, 91%, 92%, 93%, 94%, 95%, 96%, 97%, 98%, 99%, or 100%) sequence identity to SEQ ID NO:19, and/or the light chain constant region comprises an amino acid sequence having at least 90% (e.g., 90%, 91%, 92%, 93%, 94%, 95%, 96%, 97%, 98%, 99%, or 100%) sequence identity to SEQ ID NO:20.

In some embodiments of any one of the fusion proteins described above, for the anti-PD1 antibody, the heavy chain constant region comprises an amino acid sequence of SEQ ID NO:19, and the light chain constant region comprises an amino acid sequence of SEQ ID NO:20.

In some embodiments of any one of the fusion proteins described above, for the anti-PD1 antibody, the heavy chain of the anti-PD1 antibody comprises an amino acid sequence having at least 90% (e.g., 90%, 91%, 92%, 93%, 94%, 95%, 96%, 97%, 98%, 99%, or 100%) sequence identity to SEQ ID NO:17, and the light chain of the anti-PD1 antibody comprises an amino acid sequence having at least 90% (e.g., 90%, 91%, 92%, 93%, 94%, 95%, 96%, 97%, 98%, 99%, or 100%) sequence identity to SEQ ID NO:18.

In some embodiments of any one of the fusion proteins described above, the anti-PD1 antibody comprises the heavy chain as set forth in SEQ ID NO:17 and the light chain as set forth in SEQ ID NO:18.

In some embodiments of any one of the fusion proteins described above, the anti-PD1 antibody is a full-length antibody or is an antigen binding fragment selected from the group consisting of any one of F(ab')2, Fab'-SH, Fab', Fab, scFab, dsFv, (dsFv)2, Fv, and scFv.

In some embodiments of any one of the fusion proteins described above, the IL10 polypeptide is a natural IL10 polypeptide or a modified IL10 polypeptide.

In some embodiments of any one of the fusion proteins described above, the IL10 polypeptide is the modified IL10 polypeptide, the modified IL10 polypeptide is in a monomer form, and wherein relative to the natural IL10 polypeptide, the modified IL10 polypeptide comprises a spacer peptide inserted between any two adjacent amino acids, wherein the spacer peptide is a flexible peptide.

In some embodiments, one of the two adjacent amino acids is located at Position 115, Position 116, Position 117, Position 118, or Position 119.

In some embodiments, the spacer peptide is located between Position 116 and Position 117.

In some embodiments, the spacer peptide is located between Position 115 and Position 116.

In some embodiments, the spacer peptide is located between Position 117 and Position 118.

In some embodiments, the spacer peptide is selected from the group consisting of at least one of GGGSGG, GGGSG, (GS)n, (GGGS)n, (GSGGS)n, or (GGGGS)n, wherein n is any integer from 1 to 10.

In some embodiments, an amino acid sequence of the spacer peptide is as set forth in SEQ ID NO:21.

In some embodiments, relative to the natural IL10 polypeptide, the modified IL10 polypeptide has a deletion of n amino acids at an N-terminus, wherein n is any integer from 1 to 12.

In some embodiments, relative to the natural IL10 polypeptide, the modified IL10 polypeptide has a deletion of 2 amino acids at the N-terminus.

In some embodiments, relative to the natural IL10 polypeptide, the modified IL10 polypeptide has the spacer peptide insertion between the amino acids at Position 116 and Position 117, and has a deletion of 2 amino acids at the N-terminus.

In some embodiments, the natural IL10 polypeptide has an amino acid sequence as set forth in SEQ ID NO:23.

In some embodiments of any one of the fusion proteins described above, the IL10 polypeptide comprises an amino acid sequence having at least 90% (e.g., 90%, 91%, 92%, 93%, 94%, 95%, 96%, 97%, 98%, 99%, or 100%) sequence identity to SEQ ID NO:22 or 23.

In some embodiments of any one of the fusion proteins described above, the IL10 polypeptide has an amino acid sequence as set forth in SEQ ID NO:22.

In some embodiments of any one of the fusion proteins described above, the IL10 polypeptide is directly linked to the heavy chain or light chain of the anti-PD1 antibody or linked to the heavy chain or light chain of the anti-PD1 antibody via a linker peptide. In some embodiments, an N-terminus of the IL10 polypeptide is linked to a C-terminus of the light chain of the anti-PD1 antibody via a linker peptide.

In some embodiments, the fusion protein described in any one of the above comprises two identical first chains shown in Formula P1 and two identical second chains shown in Formula P2.
**P1:** [light chain of anti-PD1 antibody]-[linker peptide]-[IL10 polypeptide]
**P2**: heavy chain of anti-PD1 antibody

In some embodiments, in Formula P1, the linker peptide is a flexible linker peptide.

In some embodiments, the linker peptide is selected from the group consisting of at least one of (GGGGS)nG, (GGGGS)n, (GS)n, (GGGS)n, (GSGGS)n, or (GGGGSG)n, wherein n is any integer from 1 to 10.

In some embodiments, an amino acid sequence of the linker peptide is as set forth in SEQ ID NO:24.

In some embodiments, the fusion protein described in any one of the above comprises the first chain with an amino acid sequence having at least 90% (e.g., 90%, 91%, 92%, 93%, 94%, 95%, 96%, 97%, 98%, 99%, or 100%) sequence identity to SEQ ID NO:25, and the second chain with an amino acid sequence having at least 90% (e.g., 90%, 91%, 92%, 93%, 94%, 95%, 96%, 97%, 98%, 99%, or 100%) sequence identity to SEQ ID NO:17.

In some embodiments, the fusion protein described in any one of the above comprises two first chains with amino acid sequences as set forth in SEQ ID NO:25 and two second chains with amino acid sequences as set forth in SEQ ID NO:17.

In some embodiments, the fusion protein described in any one of the above has at least one of the following characteristics.
A. The fusion protein can specifically bind to human PD1; and optionally, the fusion protein can bind to the human PD1 with an EC50 value ≤10 nM (e.g., ≤10 nM, ≤5 nM, ≤4.50 nM, ≤3.00 nM, ≤2.00 nM, ≤1.00 nM, ≤0.9 nM, ≤0.8 nM, ≤0.7 nM, ≤0.6 nM, ≤0.5 nM, ≤0.4 nM, ≤0.3 nM, ≤0.2 nM, ≤0.1 nM, ≤0.05 nM, ≤0.01 nM, ≤0.005 nM, ≤0.001 nM, or less), where the EC50 value is determined by an FACS method. In some embodiments, the EC50 value is determined by a method in Example 3 of the present disclosure.
B. The fusion protein has low hematotoxicity or no hematotoxicity. In some embodiments, hematotoxicity is determined by the method in Example 3 of the present disclosure.
C. The fusion protein can improve killing activity of a CD8+T cell. In some embodiments, the hematotoxicity is determined by the method in Example 5 of the present disclosure.
D. The fusion protein can inhibit growth of a tumor. In some embodiments, the inhibition of the growth of the tumor is determined by a method in Example 6 of the present disclosure.

The present disclosure further provides a nucleic acid, which encodes the fusion protein described in any one of the above. In some embodiments, the nucleic acid is an isolated nucleic acid.

The present disclosure further provides a vector, which includes the nucleic acid described in any one of the above.

The present disclosure further provides a cell, which includes the nucleic acid or vector described in any one of the above.

The present disclosure further provides a pharmaceutical composition, which includes the fusion protein, nucleic acid, vector, or cell described in any one of the above. In some embodiments, the pharmaceutical composition further includes one or more pharmaceutically acceptable carriers, diluents, or excipients.

The present disclosure further provides an application of the fusion protein, nucleic acid, vector, cell, or pharmaceutical composition described in any one of the above in preparation of drugs for treating or preventing tumors or inflammatory diseases. In some embodiments, the tumors or inflammatory diseases are diseases with high human PD1 and/or PDL1 expression. In some embodiments, the tumors are tumors with high PDL1 expression. In some embodiments, the tumors are selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer. In some embodiments, the tumors are selected from the group consisting of non-small cell lung cancer, renal cell carcinoma, and gastric cancer.

In some embodiments, the present disclosure provides a method for treating diseases. The method includes a step of applying an effective dosage of the fusion protein, nucleic acid, vector, cell, or pharmaceutical composition described in any one of the above to a subject in need. In some embodiments, the diseases are tumors or inflammatory diseases. In some implementations, the tumors or inflammatory diseases are diseases with high human PD1 and/or PDL1 expression. In some embodiments, the tumors are tumors with high PDL1 expression. In some embodiments, the tumors are selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer. In some embodiments, the tumors are selected from the group consisting of non-small cell lung cancer, renal cell carcinoma, and gastric cancer.

In another aspect, the present disclosure further provides the fusion protein, nucleic acid, vector, cell, or pharmaceutical composition described in any one of the above for use as a drug. In some embodiments, the drug is used for treating diseases. In some embodiments, the diseases are tumors or inflammatory diseases. In some implementations, the tumors or inflammatory diseases are diseases with high human PD1 and/or PDL1 expression. In some embodiments, the tumors are tumors with high PDL1 expression. In some embodiments, the tumors are selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer. In some embodiments, the tumors are selected from the group consisting of non-small cell lung cancer, renal cell carcinoma, and gastric cancer.

In some embodiments, the treatments described in any one of the above further include applying other therapeutic drugs to the subject. In some embodiments, other therapeutic drugs are chemotherapeutic agents.

The present disclosure further provides a kit, which includes the fusion protein, nucleic acid, vector, cell, or pharmaceutical composition described in any one of the above. In some embodiments, the kit is used for detecting or determining human PD1.

The present disclosure further provides a method for preparing the fusion protein described in any one of the above. The method includes steps of culturing the cell described in any one of the above, and then obtaining the fusion protein through separation and purification.

The present disclosure provides a new anti-PD1-IL10 fusion protein. In some embodiments, the anti-PD1-IL10 fusion protein in the present disclosure may relieve immunosuppression of the PD-1/PD-L1 through the anti-PD1 antibody in one aspect, and, in another aspect, may be directly enriched on a surface of an exhausted CD8+T cell highly expressing the PD1. The CD8+T cell is hindered to further exhaust by the IL10 polypeptide, the killing activity of the CD8+T cell is improved, and apoptosis induced by activation is inhibited, thereby improving an anti-tumor immune response. In some implementations, compared to the natural IL10 having significant hematologic toxicity in cynomolgus monkeys, the anti-PD1-IL10 fusion protein in the present disclosure has no hematologic toxicity and liver injury during drug administration, such that the problem of hematologic toxicity in IL10 in clinical, which results in low dosages and limited therapeutic efficacy. Therefore, the treatment problem of patients with PD-1/PD-L1 resistance is solved, the anti-tumor immune response of a body is improved, and the survival rate of cancer patients is increased.

In some embodiments, the anti-PD1-IL10 fusion protein (e.g., R1153) in the present disclosure also has good stability. For example, the fusion protein has good stability by being placed for 7 days and 14 days at a high temperature of 40 °C, placed for 1 h, 2 h, and 4 h at low pH (3.5), placed for 7 days at room temperature, placed for 7 days at 4 °C, etc., respectively, and the binding activity of the anti-PD1-IL10 fusion protein is basically unaffected.

### Brief Description of the Drawings

In order to illustrate the technical solutions in the embodiments of the present disclosure more clearly, the drawings used in the embodiments will be briefly introduced below. It is to be understood that the following drawings only illustrate some embodiments of the present disclosure, which therefore should not be regarded as limitations to the scope. For those of ordinary skill in the art, other related drawings may also be obtained in accordance with these drawings without creative efforts.
Fig. 1 is a schematic structural diagram of an anti-PD1-IL10 fusion protein.
Fig. 2 is an electrophoregram of an anti-PD1-IL10 fusion protein.
Fig. 3 is a diagram of a binding activity detection result for an anti-PD1-IL10 fusion protein and a human PD1 antigen.
Fig. 4 is a diagram of a binding activity detection result for an anti-PD1-IL10 fusion protein and a human IL10R.
Fig. 5 is a diagram of an activity experiment result for IL10 of proteins (R1153 and R1152) detected by a reporter gene method.
Fig. 6 is a diagram of an activity experiment result for IL10 of proteins (R1153 and R1152+CHO-hPD1) detected by a reporter gene method.
Fig. 7 is a diagram of an activity experiment result for PD1 of proteins (R1153 and R1152) detected by a reporter gene method.
Fig. 8 is a diagram of results for an anti-PD1-IL10 fusion protein CD8+T cell apoptosis detection experiment.
Fig. 9 is a diagram of results for IFN-γ factor secretion detection in an anti-PD1-IL10 fusion protein CD8+T apoptosis experiment.
Fig. 10 is a diagram of results for an in-vivo drug efficacy detection experiment of an anti-PD1-IL10 fusion protein.
Fig. 11 is a diagram of results for an FACS binding experiment of a PD-1 terminus of an anti-PD1-IL10 fusion protein.
Fig. 12 is a diagram of results for an FACS binding experiment of a IL10 terminus of an anti-PD1-IL10 fusion protein.
Fig. 13 is a diagram of results for a fluorescence reporter detection activity experiment of a PD-1 terminus of an anti-PD1-IL10 fusion protein.
Fig. 14 is a diagram of results for a fluorescence reporter detection activity experiment of a IL10 terminus of an anti-PD1-IL10 fusion protein.
Fig. 15 is a diagram of results for a binding and blocking activity experiment of a PD-1 terminus of an anti-PD1-IL10 fusion protein.
Fig. 16 is a diagram of results for a binding activity experiment of an anti-PD1-IL10 fusion protein and induced exhausted CD8+T cells.
Fig. 17 is a diagram of detection results for inhibition of exhausted CD8+T apoptosis by an anti-PD1-IL10 fusion protein.
Fig. 18 is a diagram of detection results for functions of an anti-PD1-IL10 fusion protein on exhausted CD8+T cells.
Fig. 19 is a diagram of results for an impact of an anti-PD1-IL10 fusion protein on an IFN-γ secretion level in an SEB experiment.
Fig. 20 is a diagram of results for an impact of an anti-PD1-IL10 fusion protein on an IL-2 secretion level in an MLR experiment.
Fig. 21 is a diagram of results for an impact of an anti-PD1-IL10 fusion protein on an IFN-γ secretion level in an MLR experiment.
Fig. 22 is a diagram of results for a binding activity experiment of an anti-PD1-IL10 fusion protein and a C1q protein.
Fig. 23 is a diagram of results for a fluorescence reporter detection ADCC activity experiment of an anti-PD1-IL10 fusion protein.
Fig. 24 is a diagram of results for an in-vivo drug efficacy detection experiment of an anti-PD1-IL10 fusion protein in a CT26 model.
Fig. 25 is a diagram of results for an in-vivo drug efficacy detection experiment of an anti-PD1-IL10 fusion protein in a MC38 model.

### Detailed Description of the Embodiments

In order to make the objectives, technical solutions, and advantages of the embodiments of the present disclosure clearer, the technical solutions in the embodiments of the present disclosure are described below in a clear and complete manner. However, the description and embodiments should not be construed as limiting the scope of the present disclosure. If specific conditions are not indicated in the embodiments, the implementations are carried out in accordance with the conventional conditions or the conditions recommended by manufacturers. Reagents or instruments used are conventional products that may be purchased commercially if the manufacturers are not specified. Unless explicitly defined herein, technical and scientific terms used in the present disclosure shall have the meanings commonly understood by those skilled in the art to which the present disclosure belongs.

In the present disclosure, "a/an", "one", "the", "the above", and "the foregoing" include plural references unless the context clearly indicates otherwise. For example, "an antibody" refers to one antibody or more than one antibody. In addition, the terms "first" and "second" are used for descriptive purposes only and are not to be construed as indicating or implying relative importance or implicitly specifying the number of technical features indicated. The term "a plurality of" means at least two, such as 2, 3, and so on, unless explicitly stated otherwise.

Unless the context clearly indicates otherwise, the terms "including" or "having" in the specification and claims of the present disclosure shall be understood to mean "including" rather than in an exclusive or exhaustive sense, that is, they shall be understood to mean "including but not limited to." For example, "including A" means "including A, but not limited to A."

"Approximately" means within an acceptable error range of a specific value as determined by those skilled in the art, which will depend in part on how the value is measured or determined, that is, the limitations of a measurement system. In the context of specific determination, results, or embodiments, unless explicitly stated otherwise in the embodiments or elsewhere in the specification, "approximately" means within one standard deviation according to standard practice in the art, or within a range of up to 5%.

The term "and/or" refers to either one or a combination of two specific characteristics, which may or may not include another specific characteristic. For example, "A and/or B" means any of the following scenarios: A and B, A or B, A (alone) and B (alone); "A, B, and/or C" means any of the following scenarios: A, B, and C, A, B, or C, A or C, A or B, B or C, A and C, A and B, B and C, A (alone), B (alone), and C (alone).

Three-letter and single-letter codes of amino acids used in the present disclosure are described in J. Biol. Chem., 243, p. 3558 (1968).

The term "amino acid" refers to a naturally-existing amino acid and a synthetic amino acid, as well as an amino acid analogue and amino acid mimic that function in a manner similar to the naturally-existing amino acid. The naturally-existing amino acid includes an amino acid encoded by a genetic code and an amino acid modified by the same, such as hydroxyproline, γ-carboxyglutamic acid, and O-phosphoserine. Common naturally-existing amino acids include Alanine (Ala; A), Arginine (Arg; R), Asparagine (Asn; N), Aspartic acid (Asp; D), Cysteine (Cys; C); Glutamic acid (Glu; E), Glutamine (Gln; Q), Glycine (Gly; G); Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V). The amino acid analogue refers to a compound having the same basic chemical structure as the naturally-existing amino acid (i.e., α-carbon bonded to hydrogen, carboxyl, amino, and an R group), such as homoserine, norleucine, methionine sulfoxide, and methionine methylsulfide. The amino acid analogue typically has a modified R-group (e.g., norleucine) or a modified peptide backbone, but retains the same basic chemical structure as the naturally-existing amino acid. The amino acid mimic refers to a chemical compound that has a structure differing from a general chemical structure of amino acids, but functions in a manner similar to the naturally-existing amino acid.

The term "antibody" in the present disclosure is used in a broadest manner and encompasses various antibody structures, including but not limited to: a monoclonal antibody, a polyclonal antibody, a monospecific antibody, a multispecific antibody (e.g., a bispecific antibody, a trispecific antibody, a tetraspecific antibody), a mouse-derived antibody, a chimeric antibody, a humanized antibody, a human antibody, a full-length antibody, an antigen binding fragment (also referred to as an antigen-binding portion or an antibody fragment), etc., as long as they show the desired antigen-binding activity.

The "natural antibody" is a naturally-existing immunoglobulin molecule. For example, a natural IgG antibody is heterotetrameric glycoprotein with approximately 150, 000 daltons, and composed of two light chains and two heavy chains linked by disulfide bonds. From an N-terminus to a C-terminus of the IgG antibody, each heavy chain has a heavy chain variable region (VH, also referred to as a variable heavy domain or heavy chain variable domain), followed by a heavy chain constant region (CH, also referred to as a constant heavy domain). The heavy chain constant region includes three constant domains (CH1, CH2, and CH3); Similarly, from the N-terminus to the C-terminus, each light chain has a light chain variable region (VL, also referred to as a variable light domain or light chain variable domain), followed by a light chain constant region (CL, also referred to as a constant light domain). The antibodies are classified into five isotypes-IgA, IgD, IgE, IgG, and IgM based on whether the antibodies include heavy chains of α, δ, ε, γ, and µ. Isotypic antibodies may further be divided into different subclasses. For example, the IgG isotype includes four subclasses: IgG1 (γ1 heavy chain), IgG2 (γ2 heavy chain), IgG3 (γ3 heavy chain), and IgG4 (γ4 heavy chain). The IgA isotype is divided into two subclasses: IgA1 (α1 heavy chain) and IgA2 (α2 heavy chain), etc.

The terms "full-length antibody," "complete antibody," and "full antibody" in the present disclosure are used interchangeably herein to refer to antibodies having a structure substantially similar to that of the natural antibodies, or antibodies including heavy chains of an Fc region.

"Isolated" antibodies are antibodies that have been separated from the components in a natural environment. In some embodiments, the antibodies may be purified to purity greater than 90% or 99%. Purification and determination may be performed through methods such as electrophoresis (e.g., SDS-PAGE, isoelectric focusing (IEF), capillary electrophoresis) or chromatography (e.g., ion exchange or reverse-phase HPLC). For a review of methods for evaluating antibody purity, refer to, e.g., Flatman et al., J. Chromatogr. B 848:79-87 (2007).

The term "variable region" or "variable domain" refers to a domain in the heavy or light chain of an antibody that is involved in binding antigen. For the heavy chain variable region (VH) and light chain variable region (VL) of the natural antibody, each variable region includes four conserved framework regions (FR) and three hypervariable regions (also referred to as complementarity determining regions, HVR, or CDR).

The term "complementarity determining region", "hypervariable region", or "CDR" refers to a primary region within a variable domain that facilitates antigen binding; and "framework" or "FR" refers to a variable domain residue within the variable region excluding a CDR residue. The VH includes 3 CDR regions: HCDR1, HCDR2, and HCDR3; and the VL includes 3 CDR regions: LCDR1, LCDR2, and LCDR3. Each VH and VL typically consists of three CDRs and four FRs arranged in the following sequence (from an N-terminus to a carboxyl terminus): FR1-CDR1-FR2-CDR2-FR3-CDR3-FR4. The boundaries of an amino acid sequence of the CDRs may be determined using methods known to those skilled in the art, such as a "Kabat" numbering rule (referring to Kabat et al. (1991), "Sequences of Proteins of Immunological Interest", 5th Ed., Public Health Service, National Institutes of Health, Bethesda, MD), a "Chothia" numbering rule, an "ABM" numbering rule, a "contact" numbering rule (referring to Martin, ACR. Protein Sequence and Structure Analysis of Antibody Variable Domains[J]. 2001), an ImMunoGenTics (IMGT) numbering rule (Lefranc, M.P.et al., Dev. Comp. Immunol., 27, 55-77(2003); Front Immunol. 2018 Oct 16;9:2278), etc. A correspondence relationship between the numbering systems is well known to those skilled in the art.

The term "light chain" includes the light chain variable region (VL) and the light chain constant region (CL). The VL is located at the N-terminus of the light chain, and the CL is located at the C-terminus of the light chain. The light chain may be a κ chain, a λ chain, etc.

The term "heavy chain" includes the heavy chain variable region (VH) and the heavy chain constant region (CH). The heavy chain variable region is located at the N-terminus of the heavy chain, and the heavy chain constant region is located at the C-terminus of the heavy chain. The IgG antibody heavy chain constant region includes three constant domains: CH1, CH2, and CH3. The heavy chain may belong to any isotype, including IgG (including IgG1, IgG2, IgG3, and IgG4 subclasses), IgA (including IgA1 and IgA2 subclasses), IgD, IgM, and IgE.

The term "constant region" or "constant domain" refers to the carboxyl-terminal portions of the light chain and heavy chain. The portions do not directly participate in the binding of the antibody to the antigen. Compared to the VH and VL, the constant region has a relatively-conserved amino acid sequence.

The term "antigen binding fragment" refers to a portion of a complete antibody but differs from the complete antibody. The portion can specifically bind to the antigen that the complete antibody binds to. Examples of the antigen binding fragment include, but are not limited to: Fv, Fab, Fab', Fab'-SH, F(ab')2, dsFv, (dsFv)2, single-chain Fab (scFab), single-chain antibodies (e.g., scFv), a bispecific antibody, a linear antibody, and a multispecific antibody formed by the antigen binding fragment. "Fab" is a monovalent fragment composed of the VL, VH, CL, and CH1 structural domains; "Fv" is composed of the VH and the VL; "Fab" includes an Fab segment of partial hinge region; "F(ab')2" includes bivalent fragments of two Fab' fragments connected by a disulfide bond in the hinge region; "scFab" is a polypeptide composed of the VH, the CH1, the VL, the CL, and an linker, where the antibody domain and the linker have one of the following sequences from the N-terminus to the C-terminus: a) VH-CH1-linker-VL-CL, b) VL-CL-linker-VH-CH1, c) VH-CL-linker-VL-CH1, or d) VL-CH1-linker-VH-CL; "scFv" is a fusion protein including the light chain variable region and the heavy chain variable region, where the light chain variable region and the heavy chain variable region are connected via a peptide linker, the scFv can be expressed as a single-chain polypeptide and retains the specificity of the complete antibody from which the scFv is derived, and unless otherwise specified, the scFv here may have the VL and VH variable regions in any sequence, for example, the N-terminus to the C-terminus of the scFv polypeptide may include: a) VL-linker-VH or b) VH-linker-VL; "dsFv" is an Fv fragment stabilized by the disulfide bond; (dsFv)2 represents dimerized dsFv; and "Fab'-SH" indicates that a cysteine residue in the hinge region of a Fab' fragment carries a free thiol group.

The term "Fc region" refers to the C-terminal region of the antibody heavy chain, including both a natural antibody Fc region and an Fc region variant. In some implementations, the Fc region of the human IgG heavy chain extends from an amino acid residue at Position Cys226 to the carboxyl terminus. In some implementations, the Fc region of the human IgG heavy chain extends from Pro230 to the carboxyl terminus. The C-terminus of the Fc region may also be, for example, missing the C-terminus lysine of the Fc region (residue 447 according to an EU numbering system) or C-terminus glycine and lysine missing the Fc region (residues 446 and 447 according to the EU numbering system). In some implementations, the composition of the complete antibody may include an antibody population missing the K447 residue and/or the G446+K447 residues. In some implementations, the composition of the complete antibody may include an antibody population without missing the K447 residue and/or the G446+K447 residues. In some implementations, the composition of the complete antibody may include an antibody population having an antibody mixture with or without the K447 residue and/or the G446+K447 residues. The Fc region used for the antibody described in the present disclosure includes Fc regions of human IgG1, IgG2 (IgG2a, IgG2b), IgG3, and IgG4. Unless otherwise specified in the present disclosure, the amino acid residues in the Fc region or constant region are numbered according to the EU numbering system, also referred to as an EU index, as described in Kabat et al., Sequences of Proteins of Immunological Interest, 5th Edition, Public Health Service, National Institutes of Health, Bethesda, MD, 1991.

The term "chimeric" antibody means that a portion of the heavy chain and/or light chain in the antibody is derived from a specific species, while the remaining portion of the heavy chain and/or light chain is derived from another species.

The term "humanized" antibody refers to an antibody that retains the reactivity of a non-human antibody while having low immunogenicity in humans. For example, this may be implemented by retaining the CDR region of the non-human antibody and replacing the remaining portion of the antibody with its human counterpart (i.e., the framework region of the heavy chain variable region of the human antibody, the framework region of the light chain variable region, and the constant region of the human antibody).

The terms "human antibody", "humanized antibody", "fully human antibody", and "completely human antibody" are interchangeable and refer to antibodies of which variable region and constant region sequences are human sequences. The human antibody includes an antibody that is derived from a human gene but with altered features such as cysteine or glycosylation sites. The human antibody may be an antibody that is recombinantly produced in non-human cells. The human antibody includes antibodies obtained from transgenic mice with human immunoglobulin heavy chain and light chain gene loci.

The term "affinity" refers to the overall strength of a non-covalent interaction between a single binding site of a molecule (e.g., an antibody) and its binding ligand (e.g., an antigen). Unless otherwise specified, the term "affinity" in the present disclosure refers to internal binding affinity, reflecting a 1:1 interaction between members of a binding pair (e.g., antibody and antigen). The affinity of a molecule X to its ligand Y is typically represented by a dissociation constant (KD). The affinity may be measured using conventional methods known in the art. The term "kassoc" or "ka" refers to an association rate of a specific antibody-antigen interaction, and the term 'kdis' or "kd" refers to a dissociation rate of the specific antibody-antigen interaction. The term "KD" refers to the dissociation constant, which is obtained from a ratio of kd to ka (i.e., kd/ka), and typically expressed in molar concentration (M). A KD value of the antibody may be determined using methods known in the art. Methods for determining antibody KD include measuring surface plasmon resonance with a biosensing system, or measuring solution affinity by Solution Equilibrium Titration (SET).

The term "affinity-matured" antibody refers to an antibody that has one or more amino acid residue changes in one or more CDRs of the antibody, resulting in improved affinity of the antibody for the antigen compared to a parent antibody. In some implementations, the affinity-matured antibody has nanomolar or even picomolar affinity to a target antigen. The affinity-matured antibody may be generated using methods known in the art. Marks et al., Bio/Technology 10:779-783 (1992) recorded affinity maturation through rearrangement of the VH and VL structural domains. The following documents record random mutagenesis of CDR and/or framework residues: Barbas et al., PNAS, 91:3809-3813 (1994); Schier et al., Gene 169:147-155 (1995); Yelton et al., J. Immunol. 155:1994-2004 (1995); Jackson et al., J. Immunol. 154 (7):3310-9 (1995); and Hawkins et al., J. Mol. Biol. 226:889-896 (1992).

The term "effector function" refers to biological activity attributable to the antibody Fc region (a natural sequence Fc region or an Fc region with a mutated amino acid sequence). Examples of the antibody effector function include, but are not limited to: C1q binding and complement dependent cytotoxicity, Fc receptor binding, antibody dependent cell-mediated cytotoxicity (ADCC), phagocytosis, downregulation of cell surface receptors (e.g., B cell receptors); and B cell activation.

The term "monoclonal antibody" refers to a substantially homogeneous population of antibodies, that is, the amino acid sequences of the antibody molecules in the population are identical (except for the possible presence of a small number of natural mutations). In contrast, the polyclonal antibody typically includes different antibodies of which variable structural domains have different amino acid sequences, and are generally specific to different epitopes and/or different antigens. "Monoclonal" refers to antibodies derived from a substantially homogeneous population of antibodies. The monoclonal antibody may be produced by methods known in the art, such as by a hybridoma method (Kohler et al., (1975) Nature 256:495), by recombinant DNA methods (see, e.g., U.S. Patent No. 4, 816, 567), or by isolation from a phage antibody library (Clackson et al., 1991, Nature 352:624-628 and Marks et al., 1991, J. Mol. Biol. 222:581-597), or obtained using transgenic animals containing all or part of human immunoglobulin loci (see Presta (2005) J. Allergy Clin. Immunol. 116:731).

The term "antigen" refers to a protein capable of selectively binding to an antigen-binding protein (e.g., the antibody). The antigen may have one or more epitopes that interact with different antigen-binding proteins (e.g., the antibodies).

The term "epitope" refers to an area or region on the antigen that can specifically bind to the antibody. The epitope is typically composed of antigenic determinants of specific chemical groups with a certain structure. The epitope may be formed by continuous amino acid residues (linear epitope) or by non-continuous amino acid residues (conformational epitope), such as non-continuous amino acid residues with close spatial proximity due to the folding of the antigen. In some embodiments, the epitope includes at least 3, at least 4, at least 5, at least 6, at least 7, or 8-10 amino acid residues in a unique spatial conformation. The epitope may be determined by any method known in the art, such as a conventional immunoassay, an antibody competitive binding assay, or X-ray crystallography or related structural determination methods (e.g., nuclear magnetic resonance spectroscopy). The screening of the antibodies (i.e., those binding to the same epitope) that bind to specific epitopes may be achieved using methods known in the art, including but not limited to, for example, alanine scanning, peptide blotting (see Meth. Mol. Biol. 248(2004)443-463), peptide cleavage analysis, epitope excision, epitope extraction, chemical modification of the antigen (see Prot. Sci. 9(2000)487-496), and cross blocking (see "Antibodies, " Harlow and Lane (Cold Spring Harbor Press, Cold Spring Harb., NY)).

An antibody that "binds to the same epitope" as a reference antibody or an antibody that "competitively binds to" the reference antibody refers to an antibody that blocks 50% or more of the reference antibody's binding to the antigen in a competitive assay, or whose binding to the antigen is blocked by 50% or more by the reference antibody. In order to determine whether an antibody to be tested binds to the same epitope as the reference antibody, whether the reference antibody is allowed to bind to the antigen is detected under saturated conditions. For example, after removing excess reference antibody, the ability of the antibody to be tested to bind to the antigen is evaluated. If the antibody to be tested can bind to the antigen after the saturation binding of the reference antibody, the antibody to be tested binds to an epitope different from the reference antibody. However, if the antibody to be tested cannot bind to the antigen after the saturation binding of the reference antibody, the antibody to be tested may bind to the same epitope as the reference antibody. In order to confirm whether the antibody to be tested binds to the same epitope or is merely prevented from binding due to spatial constraints, conventional experiments (e.g., peptide mutagenesis and detection using ELISA, RIA, surface plasmon resonance, flow cytometry, or any other quantitative or qualitative method known in the art). If, in both settings (i.e., with each antibody acting as the saturated antibody in turn), only the saturated antibodies bind to the antigen, it may be concluded that the antibody to be tested and the reference antibody competitively bind to the antigen. In some embodiments, in competitive binding assays (see, e.g., Junghans et al., Cancer Res. 50 (1990) 1495-1502), if one excess antibody (e.g., 1-, 5-, 10-, 20-, or 100-fold) inhibits the binding of another antibody to the antigen by at least 50%, at least 75%, at least 90%, or even 99% or more, the two antibodies are considered to bind to the same or overlapping epitopes.

The term "specific binding" or "specifically bind" refers to non-random binding between two molecules. For example, the antibody binds to the antigen or epitope corresponding to the antibody with higher affinity than to other antigens or epitopes. Typically, the antibody binds to the antigen or epitope in the antigen with an equilibrium dissociation constant (Kd) of approximately 1×10⁻⁷ M or lower (e.g., approximately 1×10⁻⁸ M or lower, approximately 1×10⁻⁹ M or lower, approximately 1×10⁻¹⁰ M or lower, approximately 1×10⁻¹¹ M or lower, or approximately 1×10⁻¹² M or lower). In some implementations, the KD of the antibody binding to the antigen is 10%, 1%, or less of the KD of the antibody binding to a non-specific antigen (e.g., BSA, casein). The KD value may be measured using methods known in the art, such as by BIACORE^{®} surface plasmon resonance assay. The antibody that specifically binds to the antigen or epitope in the antigen may have cross-reactivity with other related antigens. For example, the antibody may have the cross-reactivity with corresponding antigens from other species such as humans or monkeys, including crab-eating macaque (Macaca fascicularis) (cynomolgus, cyno), chimpanzee (Pan troglodytes) (chimpanzee, chimp), or common marmoset (Callithrix jacchus) (marmoset).

The terms "anti-PD1 antibody" and "PD1-binding antibody" refer to antibodies capable of binding to a PD1 antigen with sufficient affinity. In one embodiment, a binding degree of the anti-PD1 antibody to a non-PD1 protein is less than approximately 10%, 1%, or less than that to the PD1, and the binding may be measured by the BIACORE^{®} surface plasmon resonance assay. In some implementations, the antibody that binds to the PD1 has the following dissociation constants (KD): <approximately 1 µM, <approximately 100 nM, <approximately 10 nM, <approximately 1 nM, <approximately 0.1 nM, <approximately 0.01 nM, or<approximately 0.001 nM or less. In some embodiments, the anti-PD1 antibody binds to a conservative PD1 epitope in the PD1 from different species.

The binding of the anti-PD1 antibody provided in the present disclosure to human PD1 may also be expressed as a "half-maximal effective concentration" (EC50) value. Generally, if the EC50 is smaller, the affinity is better, and it indicates that the antibody may bind to the antigen at a lower concentration. The EC50 value may be determined using binding assays known in the art, such as direct or indirect binding assays (e.g., an enzyme-linked immunosorbent assay (ELISA), a flow cytometric cell sorting, and other binding assays).

In some implementations, the anti-PD1 antibody of the present disclosure is R1149, R1150, R1151, or R1152.

The term "fusion protein" refers to a naturally-existing, synthetic, semi-synthetic, or recombinant protein molecule that includes all or part of two or more heterologous polypeptides linked by peptide bonds.

In some implementations, the fusion protein of the present disclosure is an anti-PD1-IL10 fusion protein (including a PD1 binding unit portion and an IL10 unit portion). The PD1 binding unit portion is an anti-PD1 antibody, and the IL10 unit portion is an IL10 polypeptide. In some implementations, the IL10 unit is linked to a C-terminus and/or N-terminus of a heavy chain and/or light chain of the anti-PD1 antibody, thereby building the anti-PD1-IL-10 fusion protein. The anti-PD1-IL10 fusion protein isolates the IL10 unit through steric hindrance of the structure of the anti-PD1. When the IL10 unit of the fusion protein is in a free state, a functional response cannot be triggered even when the IL10 unit binds to IL10Rα. After an antibody terminus of the anti-PD1-IL10 fusion protein binds to a target cell, a clustering effect gathers nearby IL10 unit-IL10Rα complexes, so as to further bind to IL10Rβ to form a complex and generate a downstream signal and triggering a biological function, such that IL10 molecules are prevented from interfering with the targeting property of the antibody before reaching a target site, thereby avoiding clinical side effects. Moreover, since the IL-10 unit is difficult to trigger the biological function, the anti-PD1-IL10 fusion protein does not impose the same administration dose limitations as natural dimer IL10 molecules, causing a significant increase in the administration dose of the fusion protein.

In some embodiments, the present disclosure provides an anti-PD1-IL10 fusion protein, including a PD1 binding unit portion and an IL10 unit portion. The PD1 binding unit portion is an anti-PD1 antibody and includes two identical light chains and two identical heavy chains, and the IL10 unit is linked to the heavy chain and/or light chain of the anti-PD1 antibody. The natural IL10 molecule is in a dimer form. The IL10 molecules of the fusion protein in some specific embodiments of the present disclosure are in a monomer form before the antibody binds to the target cell and cannot bind to an IL10 receptor, thereby avoiding interference with the binding of the antibody molecule to the target cell. After the antibody binds to the target cell, a large number of IL10 unit molecules gather, causing 2 adjacent IL10 units to function as a dimer and to bind to the IL10 receptor, triggering a normal biological response, and causing the targeting property and safety of the fusion protein to be significantly improved, thereby allowing drug efficacy to be fully achieved. According to some specific embodiments of the present disclosure, each antibody light chain of the anti-PD1 antibody includes a VL region and a CL region, and each antibody heavy chain includes a VH region, a CH1 region, a CH2 region, and a CH3 region. The N-terminus of the IL10 unit is linked to a C-terminus of the antibody light chain, or the C-terminus of the IL10 unit is linked to an N-terminus of the antibody light chain, or the N-terminus of the IL10 unit is linked to a C-terminus of the light chain VL region, the C-terminus of the IL10 unit is linked to an N-terminus of the light chain CL region, or the C-terminus of the IL10 unit is linked to an N-terminus of the antibody heavy chain, or the N-terminus of the IL10 unit is linked to a C-terminus of the heavy chain VH region, the C-terminus of the IL10 unit is linked to an N-terminus of the heavy chain CH1 region, or the N-terminus of the IL10 unit is linked to a C-terminus of the heavy chain CH2 region, or the C-terminus of the IL10 unit is linked to an N-terminus of the heavy chain CH3 region.

According to some specific embodiments of the present disclosure, the IL10 unit includes a natural IL10 unit, and the natural IL10 unit has an amino acid sequence show in SEQ ID NO:23.

The natural IL10 (also referred to as IL-10) is a cytokine having a multi-directional adjustment function, and its active form typically exists as a homodimer formed by two subunits linked by disulfide bonds. The natural IL-10 is primarily expressed and secreted by an immune cell such as monocyte, macrophage, and DC, playing a crucial role in inhibiting the secretion of inflammatory cytokines such as TNF-α, IL-6, etc. The IL10 receptor (IL10R) consists of two binding subunits (R1 and R2), forming an IL10/IL10Rα/IL10Rβ hexameric complex that mediates downstream signal transduction. The IL10 receptor is primarily expressed on cells such as monocyte/macrophage, DC, T cells, B cells, etc., achieves different functions in different cells. A naive CD8+T cell does not express the IL10 receptor. In a tumor tissue, if the activation degree of the CD8+T cell is higher, the IL10 receptor has a higher expression level, and the exhausted CD8+T cell shows a higher expression level compared to the activated CD8+T cell. Therefore, the IL10 has a bidirectional regulation effect in a tumor microenvironment. On one hand, antigen presentation is reduced by inhibiting inflammatory responses in Th1 cells, macrophages, and Th17 and down-regulating MHC expression in the macrophages and DC, thereby achieving a tumor-promoting effect, and on the other hand, an anti-tumor effect may be achieved by directly stimulating the activation of the CD8+T cell infiltrating in the tumor. According to some specific embodiments, the anti-PD1-IL10 fusion protein in the present disclosure may relieve immunosuppression of the PD-1/PD-L1 in one aspect, and, in another aspect, may be directly enriched on a surface of an exhausted CD8+T cell highly expressing the PD1. The CD8+T cell is hindered from further exhaust by the IL10 polypeptide, the killing activity of the CD8+T cell is improved, and apoptosis induced by activation is inhibited, thereby improving an anti-tumor immune response.

According to some specific embodiments of the present disclosure, the IL10 unit is an IL10 polypeptide that is obtained by modifying the natural IL10. The modified IL10 polypeptide is in a monomer form, and relative to the natural IL10 polypeptide, in the modified IL10 polypeptide, a spacer peptide is inserted between any two adjacent amino acids, where the spacer peptide is a flexible peptide. In some embodiments, one of the two adjacent amino acids is located at Position 115, Position 116, Position 117, Position 118, or Position 119. In some embodiments, the spacer peptide is located between Position 116 and Position 117. In some embodiments, the spacer peptide is located between Position 115 and Position 116. In some embodiments, the spacer peptide is located between Position 117 and Position 118. In some embodiments, the spacer peptide is selected from the group consisting of at least one of GGGSGG, GGGSG, (GS)n, (GGGS)n, (GSGGS)n, or (GGGGS)n, where n is any integer from 1 to 10. In some embodiments, an amino acid sequence of the spacer peptide is as set forth in SEQ ID NO:21.

In some embodiments, relative to the natural IL10 polypeptide, the modified IL10 polypeptide has a deletion of n amino acids at an N-terminus, wherein n is any integer from 1 to 12.

In some embodiments, relative to the natural IL10 polypeptide, the modified IL10 polypeptide has a deletion of 2 amino acids at the N-terminus.

For example, modification includes at least one of (i) or (ii): (i) the first 2 amino acids at the N-terminus of the natural IL10 unit are removed; or the spacer peptide is inserted between the amino acids at Position 116 and Position 117, where the spacer peptide is the flexible linker peptide, for example, the amino acid sequence of the spacer peptide is as set forth in SEQ ID NO:21. After the first 2 amino acids at the N-terminus of the natural IL10 unit are removed, the stability of the IL10 unit is improved. Moreover, after the spacer peptide is inserted between the amino acids at Position 116 and Position 117 of the IL10 unit, the IL10 unit molecule in the fusion protein becomes a closed-loop monomer molecule (two IL10 chains of the natural IL10 dimer are formed in a staggered manner, and after modification with the inserted spacer peptide, the IL10 monomer may independently form a structural domain, i.e., the closed-loop monomer molecule). The two IL10 units in the same fusion protein do not interfere with each other through cross-linking, etc.

According to some embodiments of the present disclosure, the IL10 unit is linked to the antibody heavy chain or light chain via a linker peptide. The linker peptide links the IL-10 monomer to the antibody heavy chain or light chain, such that after the antibody portion in the fusion protein binds to a corresponding target cell, the IL10 units of 2 adjacent fusion proteins form the dimers more easily, and bind to the IL10 receptor, thereby achieving a function. According to embodiments of the present disclosure, an N-terminus of the linker peptide is linked to the C-terminus of the antibody light chain, and a C-terminus of the linker peptide is linked to the N-terminus of the IL10 unit; or the N-terminus of the linker peptide is linked to the C-terminus of the IL10 unit, and the C-terminus of the linker peptide is linked to the N-terminus of the antibody light chain; or the N-terminus of the linker peptide is linked to the C-terminus of the IL10 unit, and the C-terminus of the linker peptide is linked to the N-terminus of the antibody heavy chain. According to embodiments of the present disclosure, the IL10 unit is linked to the heavy chain terminus or light chain terminus of the antibody via the linker peptide. According to some embodiments of the present disclosure, the N-terminus of the linker peptide is linked to the C-terminus of the antibody light chain, and the C-terminus of the linker peptide is linked to the N-terminus of the IL10 unit; or the N-terminus of the linker peptide is linked to the C-terminus of the IL10 unit, and the C-terminus of the linker peptide is linked to the N-terminus of the antibody light chain; or the N-terminus of the linker peptide is linked to the C-terminus of the IL10 unit, and the C-terminus of the linker peptide is linked to the N-terminus of the antibody heavy chain.

According to some embodiments of the present disclosure, the N-terminus of the linker peptide is linked to the C-terminus of the light chain VL region, and the C-terminus of the linker peptide is linked to the N-terminus of the IL10 unit; or the N-terminus of the linker peptide is linked to the C-terminus of the IL10 unit, and the C-terminus of the linker peptide is linked to the N-terminus of the light chain CL region; or the N-terminus of the linker peptide is linked to the C-terminus of the heavy chain VH region, and the C-terminus of the linker peptide is linked to the N-terminus of the IL10 unit; or the N-terminus of the linker peptide is linked to the C-terminus of the IL10 unit, and the C-terminus of the linker peptide is linked to the N-terminus of the heavy chain CH1 region; or the N-terminus of the linker peptide is linked to the C-terminus of the heavy chain CH2 region, and the C-terminus of the linker peptide is linked to the N-terminus of the IL10 unit; or the N-terminus of the linker peptide is linked to the C-terminus of the IL10 unit, and the C-terminus of the linker peptide is linked to the N-terminus of the heavy chain CH3 region.

According to some embodiments of the present disclosure, the N-terminus of the IL10 unit is linked to the C-terminus of the VL region of the light chain of the antibody via the linker peptide, and the C-terminus of the IL10 unit is linked to the N-terminus of the CL region of the light chain of the antibody via the linker peptide; or the N-terminus of the IL10 unit is linked to the C-terminus of the VH region of the heavy chain of the antibody via the linker peptide, and the C-terminus of the IL10 unit is linked to the N-terminus of the CH1 region of the heavy chain of the antibody via the linker peptide; or the N-terminus of the IL10 unit is linked to the C-terminus of the CH2 region of the heavy chain of the antibody via the linker peptide, and the C-terminus of the IL10 unit is linked to the N-terminus of the CH3 region of the heavy chain of the antibody via the linker peptide.

In some embodiments, the fusion protein of the present disclosure includes two identical first chains shown in Formula P1 and two identical second chains shown in Formula P2. P1: [light chain of anti-PD1 antibody]-[linker peptide]-[IL10 polypeptide], and P2: heavy chain of anti-PD1 antibody. In some implementations, in Formula P1, the linker peptide is a flexible linker peptide. For example, an amino acid sequence of the linker peptide is as set forth in SEQ ID NO:24.

The terms "linker peptide", "linker polypeptide", "connexon", "Linker", or "adapter" refer to a connecting unit connecting two polypeptide fragments, and typically has a degree of flexibility. The use of an linker does not cause the loss of an original function of a protein structural domain. The linker may be a peptide linker including one or more amino acids, typically about 1 to 30, 2 to 24, or 3 to 15 amino acids. In some embodiments, the linker is selected from the group consisting of (GxSy)z GmSn linker, where x, y, z, m, and n are independently selected from the group consisting of integers 0-6. Optionally, x is selected from the group consisting of integers 1-5, and y, z, m, and n are independently selected from the group consisting of integers 0-6 (e.g., when x=4, y=1, z=4, m=1, n=0, that is, (G4S1)4G1S0, it indicates the amino acid sequence: GGGGSGGGGSGGGGSGGGGSG). In some embodiments, the linker is selected from the group consisting of (GxS)zG linker, where x is selected from the group consisting of integers 1-5, and z is selected from the group consisting of integers 1-6. In some embodiments, the linker is a polypeptide as set forth in SEQ ID NO:21 or SEQ ID NO:24.

The term "nucleic acid" is used interchangeably with the term "polynucleotide" in the present disclosure, referring to deoxyribonucleotides or ribonucleotides in single-stranded or double-stranded forms and polymers thereof. The nucleic acid includes a nucleic acid containing a known nucleotide analogue or a modified backbone residue or linkage. The nucleic acid may be synthetic, naturally-existing, or non-natural, such as a non-natural nucleic acid having binding properties similar to that of a reference nucleic acid and metabolized in a manner analogous to the reference nucleic acid. The nucleic acid includes, but is not limited to, thiophosphate ester, aminophosphate ester, methylphosphonic acid ester, chiral-methylphosphonic acid ester, 2-O-methyl ribonucleotide, and a nucleic acid modified with peptide-nucleic acid (PNA). An "isolated" nucleic acid refers to a nucleic acid molecule that has been separated from components in a natural environment. The isolated nucleic acid includes the nucleic acid molecule contained in the cell below. The cell normally contains the nucleic acid molecule. However, the nucleic acid molecule exists outside the chromosome or at a chromosomal position different from its natural chromosomal position. A "nucleic acid encoding the anti-PD1 antibody" refers to one or more nucleic acid molecules encoding the heavy chain and light chain (or fragments thereof) of the antibody, including one or more nucleic acid molecules in a single vector or separate vectors, and one or more nucleic acid molecules that exist at one or more positions in a host cell. In some embodiments, the nucleic acid sequence further includes a conservatively-modified variant (e.g., degenerate codon substitution) and a complementary sequence, as well as an explicitly-specified sequence. For example, the degenerate codon substitution may be obtained by generating the following sequences, and the third position of one or more selected (or all) codons is replaced by a mixed base and/or deoxyinosine residue (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The terms "polypeptide" and "protein" are used interchangeably here and refer to a polymer of amino acid residues. The term is applicable to an amino acid polymer, where one or more amino acid residues are artificial chemical analogues of the corresponding naturally-existing amino acids, as well as to a naturally-existing amino acid polymer and a non-naturally-existing amino acid polymer.

The terms "identity" and "sequence identity" refer to the degree (percentage) to which the amino acids (amino acid sequence identity) or nucleotides (nucleic acid sequence identity) of two sequences in an equivalent position are the same when the two sequences are optimally aligned. In order to obtain the optimal alignment between two sequences, a gap may be introduced as necessary to achieve a maximum sequence identity percentage. The sequence identity percentage of an amino acid sequence may be determined using various methods known in the art, such as software commonly used in the art including BLAST, BLAST-2, ALIGN, MEGALIGN (DNASTAR), CLUSTALW, or CLUSTAL OMEGA, etc. Those skilled in the art may determine appropriate parameters for an aligned sequence, including any algorithms required to achieve maximum alignment of the full length of a comparison sequence.

The term "conservatively-modified variant" or "conservative substitution" of the amino acid refers to replacing an amino acid in a sequence with another amino acid having similar characteristics (e.g., charge, side-chain size, hydrophilicity/hydrophobicity, backbone configuration, rigidity, etc.) without changing the biological activity of the protein. Those skilled in the art recognize that, under normal circumstances, single amino acid substitution in a non-essential region of the polypeptide typically does not change biological activity (see, e.g., Watson et al., (1987) Molecular Biology of the Gene, The Benjamin/Cummings Pub. Co., p. 224 (4th Ed.)). The term "conservatively-modified variants" or "conservative substitutions" of the nucleic acid refer to those nucleic acids encoding identical or essentially identical amino acid sequences, as well as nucleic acids with essentially identical sequences in cases where the nucleic acids do not encode the amino acid sequences. Due to the degeneracy of a genetic code, any given protein may be encoded by a plurality of nucleic acids with identical functions. For example, codons GCA, GCC, GCG, and GCU all encode amino acid alanine. Therefore, at each position that the codon specifies the alanine, the codon may be changed to any corresponding codon without changing the encoded polypeptide. Such nucleic acid variants are "silent mutations", which are a type of conservatively-modified mutations. Each nucleic acid sequence encoding the polypeptide in the present disclosure includes every possible silent mutation of the nucleic acid. Each codon in the nucleic acid (except AUG-typically the sole codon for methionine; and TGG-typically the sole codon for tryptophan) may be modified to produce molecules with identical functions.

The term "vector" refers to a carrier capable of transporting a genetic element (e.g., the nucleic acid) linked to the vector. The vector may be used to transform, transduce, or transfect host cells, causing the genetic elements carried by the vector to be expressed in the host cells. Exemplarily, the vector includes: a plasmid; a phagemid; a cosmid; an artificial chromosome, such as a Yeast Artificial Chromosome (YAC), a Bacterial Artificial Chromosome (BAC) or a P1-derived Artificial chromosome (PAC); a phage, such as a λ phage or an M13 phage; and animal viruses, etc. In some implementations, the vector is a "plasmid", which is a circular double-stranded DNA ring and may be linked to an additional DNA segment. In some implementations, the vector is a viral vector, such as an adeno-associated viral vector (AAV or AAV2), and the DNA segment may be linked to a viral genome. The vector may include a plurality of elements for controlling expression, including a promoter sequence, a transcription initiation sequence, an enhancer sequence, a selection element, and a reporter gene. The vector may further include a replication origin. The vector may further include components that facilitate its entry into cells, including but not limited to viral particles, liposomes, or protein coats. The vector may be an expression vector. In some embodiments, the vector (e.g., expression vector) includes the nucleic acid sequence encoding the antibody of the present disclosure and a promoter (e.g., SV40, CMV, or EF-1α), and may further include at least one selective marker.

The term "expression vector" or "expression construct" refers to a vector that may be used to transform the host cells and includes nucleic acid sequences instructing and/or controlling (together with the host cells) the expression of one or more coding regions operatively linked to the vector. The expression vector may include, but is not limited to: sequences that influence or control transcription, translation, and, in the presence of introns, affect RNA splicing of the coding regions operatively linked to the vector.

The term "operatively linked" a connection relationship in which the components referred to by the term are allowed to perform their inherent functions under suitable conditions. For example, a control sequence that is "operatively linked" to a protein-coding sequence refers to a sequence that is linked to it and achieves the expression of the protein-coding sequence under conditions compatible with the transcriptional activity of the control sequence.

The terms "host cell", "host cell line", and "host cell culture" are interchangeable and refer to cells into which exogenous nucleic acids have been introduced, including the progeny of such cells. The host cell includes a "transformant" and a "transformed cell", including a primary transformed cell and the progeny derived therefrom, regardless of the number of passages. The progeny may not be identical to parental cells in a nucleic acid content but may instead contain mutations. The present disclosure includes mutant progeny having the identical function or biological activity as those screened or selected in initial transformed cells. The host cell includes prokaryotic and eukaryotic host cells, and the eukaryotic host cell includes, but is not limited to, a mammalian cell, an insect cell line, a plant cell, and a fungal cell. The mammalian host cell includes a human cell, a mouse cell, a rat cell, a dog cell, a monkey cell, a pig cell, a goat cell, a cattle cell, a horse cell, and a hamster cell, including but not limited to a Chinese Hamster Ovary (CHO) cell, NSO, an SP2 cell, a HeLa cell, a Baby Hamster Kidney (BHK) cell, a monkey kidney cell (COS), a human hepatocellular carcinoma cell (e.g., Hep G2), an A549 cell, a 3T3 cell, and an HEK-293 cell. The fungal cell includes yeast and filamentous fungal cells, including, for example, Pichia pastoris, Pichia finlandica, Pichia trehalophila, Pichia koclamae, Pichia membranaefaciens, Pichia minuta (Ogataea minuta or Pichia lindneri), Pichia opuntiae, Pichia thermotolerans, Pichia salictaria, Pichia guercuum, Pichia pijperi, Pichia stiptis, Pichia methanolica, Pichia genus, Saccharomyces cerevisiae, Saccharomyces genus, Hansenula polymorpha, Kluyveromyces genus, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens, Neurospora crassa, Pichia genus, any Saccharomyces genus, Hansenula polymorpha, any Kluyveromyces genus, Candida albicans, any Aspergillus genus, Trichoderma reesei, Chrysosporium lucknowense, any Fusarium sp., Yarrowia lipolytica, and Neurospora crassa.

The term "pharmaceutical composition" refers to a mixture containing one or more drugs (e.g., the antibody described in the present disclosure) and other components (e.g., physiological/pharmaceutically acceptable carriers and excipients).

The term "pharmaceutically acceptable carrier" refers to a component in a pharmaceutical preparation that is different from an active ingredient and is non-toxic to a subject. The pharmaceutically acceptable carrier includes, but is not limited to, a buffer agent, an excipient, a stabilizing agent, a preservative, etc.

The term "subject" or "individual" includes both humans and non-human animals. The non-human animals include all vertebrates (e.g., mammals and non-mammals), for example, non-human primates (e.g., cynomolgus monkeys), sheep, dogs, cattle, chickens, amphibians, and reptiles. Unless otherwise specified, the terms "patient" and "subject" may be used interchangeably in the present disclosure. Unless otherwise defined in the present disclosure, the term "cynomolgus monkey (cyno)" or "crab-eating macaque (cynomolgus)" refers to Macaca fascicularis. In some embodiments, the subject is a human.

"Application" or "administration" refers to the contact of an exogenous drug, therapeutic agent, diagnostic agent, or composition with the animal, human, subject, cell, tissue, organ, or biological fluid when it is applied to the animal, human, test subject, cell, tissue, organ, or biological fluid.

The term "sample" refers to a collection of fluid, cells, or tissue isolated from the subject, as well as fluid, cells, or tissue present in the subject. Exemplary samples include biological fluids such as blood, serum and serosal fluid, plasma, lymph, urine, saliva, cyst fluid, tears, excretions, sputum, mucosal secretions from tissues and organs, vaginal secretions, ascites, fluids in pleura, pericardium, peritoneum, abdominal cavity, and other body cavities, fluids collected via bronchial lavage; synovial fluid; liquid solutions in contact with the subject or biological sources, such as cell and organ culture media (including cell or organ conditioned media), lavage fluids, etc., tissue biopsy samples, fine needle aspiration samples, surgically-excised tissue, and organ cultures or cell cultures.

The term "treatment or treat" refers to a clinical intervention that attempts to change a natural process of the individual being treated, and may be implemented for prevention or during the course of a clinical pathological process. The expected treatment effect includes, but is not limited to, preventing the occurrence or recurrence of diseases, alleviating symptoms, mitigating or reducing any direct or indirect pathological consequences of the diseases, preventing metastasis, slowing disease progression, improving or alleviating disease status, and inducing remission or improving prognosis. In some embodiments, the antibody of the present disclosure is used for delaying the formation of the diseases or slowing the progression of the diseases.

The term "application" or "administration" refers to the contact of an exogenous drug with the animal, human, subject, cell, tissue, organ, or biological fluid when it is applied to the animal, human, test subject, cell, tissue, organ, or biological fluid.

The term "effective dose" generally refers to an amount sufficient to reduce the severity or frequency of symptoms, eliminate symptoms and/or potential causes, prevent the onset of symptoms and/or potential causes thereof, and ameliorate damage associated with the disease status (e.g., lung disease). In some embodiments, the effective dose may be a therapeutically effective dose or a preventive effective dose. The "therapeutically effective dose" refers to the amount sufficient to treat the disease status or symptoms, particularly those associated with the disease status, or to otherwise prevent, impede, delay, or reverse the progression of the disease status or any other undesirable symptoms related to the disease in any manner. The "preventive effective dose" refers to the amount that, when administered to the subject, produces a predetermined preventive effect, such as preventing or delaying the onset (or recurrence) of the disease status, or reducing the likelihood of the onset (or recurrence) of the disease status or related symptoms. Complete treatment or preventive effects may not occur after a single dose is administered, but may occur after a series of doses. Therefore, the effective dose for treatment or prevention may be administered in one or more doses. The "therapeutically effective dose" and "preventive effective dose" may depend on various factors, such as the disease status, age, gender, and body weight of an individual, as well as the ability of the therapeutic agent to trigger the desired response in the individual.

The terms "cancer" and "tumor" refer to diseases in mammals characterized by unregulated cell growth. The tumor includes a benign tumor and a malignant tumor. An early-stage tumor refers to a non-invasive or non-metastatic tumor, or a tumor that is classified as Stage 0, Stage I, or Stage II cancer. In some embodiments, the tumor is a tumor expressing PDL1, and examples of the cancer include, but are not limited to, lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer.

In some implementations, the antibody of the present disclosure is prepared or detected using conventional techniques known in the art of cell biology, molecular biology (including recombinant technology), microbiology, biochemistry, and immunology. The related technologies have been disclosed in the literature, such as Molecular Cloning: A Laboratory Manual (2nd edition, Sambrook et al., 1989); Oligonucleotide Synthesis (M.J.Gait, 1984); Animal Cell Culture (R.I.Freshney, 1987); Methods in Enzymology (Academic Press, Inc.); Handbook of Experimental Immunology (D.M.Weir and C.C.Blackwell); Gene Transfer Vectors for Mammalian Cells (J.M.Miller and M.P.Calos, 1987); Current Protocols in Molecular Biology (F.M.Ausubel et al., 1987); PCR:The Polymerase Chain Reaction (Mullis et al., 1994); and Current Protocols in Immunology (J.E.Coligan et al., 1991). The literature is incorporated herein by reference.

The characteristics and performance of the present disclosure are further described below in detail with reference to embodiments.

### Example 1 Construction of anti-PD1-IL10 fusion protein

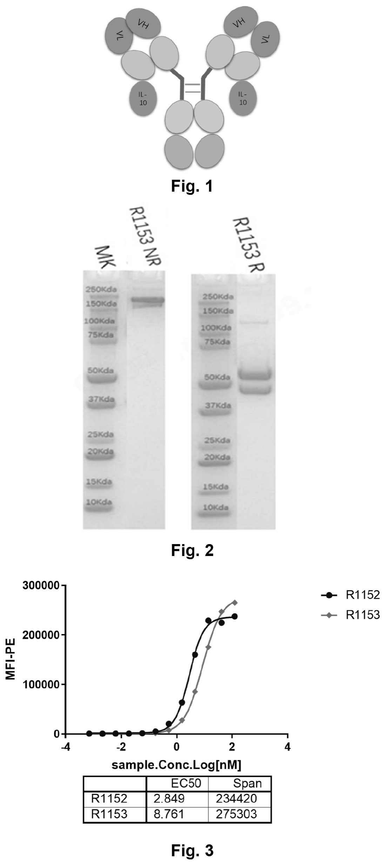

Exemplarily, an N-terminus of an IL10 monomer was linked to a C-terminus of a light chain of an anti-PD1 antibody, so as to construct the anti-PD1-IL10 fusion protein (a schematic structural diagram was shown in Fig. 1).

A variable region amino acid sequence of the anti-PD1 antibody was shown in Table 1 below.

**Table 1 Variable region sequence listing of anti-PD1 antibody**

| Antibody | VH | VL |
|---|---|---|
| R1152 | QVQLVQSGAEVKKPGASVKVSCKASGY TFTTYYMYWVRQAPGQGLEWMGGINPS NGGTNYAQKFQGRATMTVDTSTSTAYME LSSLRSEDTAVYYCTRRDSNYDGGFDYW GQGTTVTVSS (SEQ ID NO:1) | DIQMTQSPSSLSASVGDRVTITCRASKSVS TSGYSYMHWYQQKPGKAPKLLIYLAYHLE SGVPSRFSGSGSGTDFTLTISSLQPEDFAT YYCQHSWELPITFGQGTKLEIK (SEQ ID NO:2) |
| R1150 | QVQLVQSGAEVKKPGASVKVSCKASGY TFTTYYMYWVRQAPGQGLEWMGGINPS NGGTNFNEKFKSRVTMTVDKSTSTAYME LSSLRSEDTAVYYCTRRDSNYDGGFDYW GQGTTVTVSS (SEQ ID NO:3) | DIQLTQSPSSLSASVGDRATITCRASKSVS TSGYSYMHWYQQKPGKAPKLLIYLAYHLE SGVPSRFSGSGSGTDFTLTISSVQPEDFAT YYCQHSWELPITFGQGTKLEIK (SEQ ID NO:4) |
| R1151 | QVQLVQSGAEVKKPGASVKVSCKASGY TFTTYYMYWVRQAPGQGLEWIGGINPSN GGTNYAEKFKGRVTLTVDTSTSTAYMELS SLRSEDTAVYYCTRRDSNYDGGFDYWG QGTTVTVSS (SEQ ID NO:5) | DIQMTQSPSSLSASVGDRVTITCRASKSVS TSGYSYMHWYQQKPGKAPKLLIYLAYHLE SGVPSRFSGSGSGTDFTLTISSLQPEDFAT YYCQHSWELPITFGQGTKLEIK (SEQ ID NO:6) |
| R1149 | QVQLQQPGAELVKPGASVKLSCKASGYT FTTYYMYWVKQRPGQGLEWIGGINPSN GGTNFNEKFKSKATLTVDKSSSTAYMQL SSLTSEDSAVYYCTRRDSNYDGGFDYW GQGTTLTVSS (SEQ ID NO:7) | DIVLTQSPASLAVSLGQRATISCRASKSVST SGYSYMHWYQQKPGQPPKLLIYLAYHLES GVPARFSGSGSGTDFTLNIHPVEEEDAATY YCQHSWELPITFGSGTKLEIK (SEQ ID NO:8) |

Amino acid sequences of CDRs (defined by a Kabat numbering system) of anti-PD1 antibodies R1149, R1150, R1151, and R1152:
Amino acid sequence of HCDR1: TYYMY (SEQ ID NO:9).
Amino acid sequence of HCDR2: GINPSNGGTNYAQKFQG (SEQ ID NO:10); GINPSNGGTNFNEKFKS (SEQ ID NO:15); or GINPSNGGTNYAEKFKG (SEQ ID NO:16).
Amino acid sequence of HCDR3: RDSNYDGGFDY (SEQ ID NO:11).
Amino acid sequence of LCDR1: RASKSVSTSGYSYMH (SEQ ID NO:12).
Amino acid sequence of LCDR2: LAYHLES (SEQ ID NO:13).
Amino acid sequence of LCDR3: QHSWELPIT (SEQ ID NO:14).

The antibody VH in Table 1 above was linked to a human heavy chain constant region (SEQ ID NO:19), the antibody VL was linked to a human light chain constant region (SEQ ID NO:20), and the anti-PD1 antibodies R1149, R1150, R1151, and R1152 were obtained.

Exemplarily, the anti-PD1 antibody was R1152, and an amino acid sequence of R1152 was as follows.

Amino acid sequence (SEQ ID NO:17) of heavy chain of R1152 antibody:

QVQLVQSGAEVKKPGASVKVSCKASGYTFTTYYMYWVRQAPGQGLEWMGGINPSNGGTNY
AQKFQGRATMTVDTSTSTAYMELSSLRSEDTAVYYCTRRDSNYDGGFDYWGQGTTVTVSSASTK
GPSVFPLAPSSKSTSGGTAALGCLVKDYFPEPVTVSWNSGALTSGVHTFPAVLQSSGLYSLSSVVT
VPSSSLGTQTYICNVNHKPSNTKVDKRVEPKSCDKTHTCPPCPAPEAAGGPSVFLFPPKPKDTLMI
SRTPEVTCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQKSLSLSPGK.

Amino acid sequence (SEQ ID NO:18) of light chain of R1152 antibody:

DIQMTQSPSSLSASVGDRVTITCRASKSVSTSGYSYMHWYQQKPGKAPKLLIYLAYHLESGV
PSRFSGSGSGTDFTLTISSLQPEDFATYYCQHSWELPITFGQGTKLEIKRTVAAPSVFIFPPSDEQL
KSGTASVVCLLNNFYPREAKVQWKVDNALQSGNSQESVTEQDSKDSTYSLSSTLTLSKADYEKHK
VYACEVTHQGLSSPVTKSFNRGEC.

Amino acid sequence (SEQ ID NO:19) of heavy chain constant region of antibody:

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEAAGGPSVFLFPP
KPKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQK
SLSLSPGK.

Amino acid sequence (SEQ ID NO:20) of light chain constant region of antibody:


.

The IL10 monomer might be a natural IL10 or modified IL10 polypeptide. Exemplarily, the IL10 monomer was the modified IL10 polypeptide (a spacer peptide: GGGSGG (SEQ ID NO:21) was inserted between amino acid residues at Position 116 and Position 117 of the natural IL10 polypeptide), and at the same time, amino acid residues at Position 1 and Position 2 of an N-terminus of the natural IL10 polypeptide were deleted. An amino acid sequence of the IL10 monomer was as follows.

Amino acid sequence (SEQ ID NO:22) of modified IL10 polypeptide:

GQGTQSENSCTHFPGNLPNMLRDLRDAFSRVKTFFQMKDQLDNLLLKESLLEDFKGYLGCQ
ALSEMIQFYLEEVMPQAENQDPDIKAHVNSLGENLKTLRLRLRRCHRFLPCENGGGSGGKSAVE
QVKNAFNKLQEKGIYKAMSEFDIFINYIEAYMTMKIRN.

Amino acid sequence (SEQ ID NO:23) of natural IL10 polypeptide:

SPGQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENKSKAVEQVKN
AFNKLQEKGIYKAMSEFDIFINYIEAYMTMKIRN.

The above IL10 monomer was covalently linked to the C-terminus of the light chain of the anti-PD1 antibody R1152 via a linker peptide: GGGGSGGGGSGGGGSGGGGSG (SEQ ID NO:24), so as to obtain the anti-PD1-IL10 fusion protein R1153. The R1153 included two identical first chains and two identical second chains. An amino acid sequence of the R1153 was as follows.

Amino acid sequence (SEQ ID NO:25) of first chain of anti-PD1-IL10 fusion protein R1153:

DIQMTQSPSSLSASVGDRVTITCRASKSVSTSGYSYMHWYQQKPGKAPKLLIYLAYHLESG
VPSRFSGSGSGTDFTLTISSLQPEDFATYYCQHSWELPITFGQGTKLEIKRTVAAPSVFIFPPSDE
QLKSGTASVVCLLNNFYPREAKVQWKVDNALQSGNSQESVTEQDSKDSTYSLSSTLTLSKADY

EKHKVYACEVTHQGLSSPVTKSFNRGECGGGGSGGGGSGGGGSGGGGSGGQGTQSENSCTH
FPGNLPNMLRDLRDAFSRVKTFFQMKDQLDNLLLKESLLEDFKGYLGCQALSEMIQFYLEEVMPQ
AENQDPDIKAHVNSLGENLKTLRLRLRRCHRFLPCENGGGSGGKSKAVEQVKNAFNKLQEKGIYK
AMSEFDIFINYIEAYMTMKIRN.

Note: a bolded portion represented a light chain portion of the anti-PD1 antibody, a double-underlined portion represented a linker peptide portion, and a single-underlined portion represented an IL10 polypeptide portion.

An amino acid sequence of the second chain of the anti-PD1-IL10 fusion protein R1153 was identical to the amino acid sequence of the heavy chain of the R1152 antibody, both being SEQ ID NO:17.

Other control molecule sequences used in this example were as follows.

R0862: hIgG1 antibody containing an L234A/L235A mutation, also referred to in the present disclosure as Isotype control_hIgG1.8 or ISO-hIgG1.8 or Isotype control.

Heavy chain sequence (SEQ ID NO:26) of R0862:

QVQLVESGGGVVQPGRSLRLSCAASGFTFSSYGMHWVRQAPGKGLEWVAAIWYDGSNKYY
TDSVKGRFTISRDNSKNTLYLQMNSLRAEDTAVYYCARDLRGVMYFDLWGRGTLVTVSSASTKGP
SVFPLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPGK.

Light chain sequence (SEQ ID NO:27) of R0862:

EIVLTQSPATLSLSPGERATLSCRASQSVSRYLAWYQQKPGQAPRLLIYDASNRATGIPARFSG
SGSGTDFTLTISSLEPEDFAVYYCQLRNNWPLTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEV
THQGLSSPVTKSFNRGEC (SEQ ID NO:27).

R0676 included two identical peptide chains, and an amino acid sequence (SEQ ID NO:28) of one chain was as follows:

DKTHTCPPCPAPEAEGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGKGGGGSGGGGSGGGGSGGGGSSPGQGTQ
SENSCTHFPGNLPNMLRDLRDAFSRVKTFFQMKDQLDNLLLKESLLEDFKGYLGCQALSEMIQFY
LEEVMPQAENQDPDIKAHVNSLGENLKTLRLRLRRCHRFLPCENKSKAVEQVKNAFNKLQEKGIY
KAMSEFDIFINYIEAYMTMKIRN.

Heavy chain sequence (SEQ ID NO:29) of R0579:

QVQLKESGPGLVQPSQTLSLTCTVSGFSLTSYNVHWVRQPPGKGLEWMGGMRYNEDTSYN
SALKSRLSISRDTSKNQVFLKMNSLQTDDTGTYYCTRDAVYGGYGGWFAYWGQGTLVTVSSAKT
TPPSVYPLAPGSAAQTNSMVTLGCLVKGYFPEPVTVTWNSGSLSSGVHTFPAVLQSDLYTLSSSV
TVPSSTWPSQTVTCNVAHPASSTKVDKKIVPRDCGCKPCICTVPEVSSVFIFPPKPKDVLTITLTPKV
TCVVVAISKDDPEVQFSWFVDDVEVHTAQTKPREEQFNSTFRSVSELPIMHQDWLNGKEFKCRVN
SAAFPAPIEKTISKTKGRPKAPQVYTIPPPKEQMAKDKVSLTCMITNFFPEDITVEWQWNGQPAEN
YKNTQPIMDTDGSYFVYSKLNVQKSNWEAGNTFTCSVLHEGLHNHHTEKSLSHSPGAGGGGSG
GGGSGGGGSGGGGSGSPGQGTQSENSCTHFPGNLPNMLRDLRDAFSRVKTFFQMKDQLDNLL
LKESLLEDFKGYLGCQALSEMIQFYLEEVMPQAENQDPDIKAHVNSLGENLKTLRLRLRRCHRFLP
CENKSKAVEQVKNAFNKLQEKGIYKAMSEFDIFINYIEAYMTMKIRN.

Light chain sequence (SEQ ID NO:30) of R0579:

DTVLTQSPALAVSLGQRVTISCKASETVSSSMYSYIHWYQQKPGQQPKLLIYRASNLESGVPA
RFSGSGSGTDFTLTIDPVEADDVATYFCQQSWNPWTFGGGTKLELKRADAAPTVSIFPPSSEQLT
SGGASVVCFLNNFYPKDINVKWKIDGSERQNGVLNSWTDQDSKDSTYSMSSTLTLTKDEYERHN
SYTCEATHKTSTSPIVKSFNRNEC.

R0674 was composed of an Fc region and IL-10 WT fused at the C-terminus, and included two identical polypeptide chains, one of which was as follows:

Hinge-CH2-CH3-linker-IL10WT (SEQ ID NO:31):

DKTHTCPPCPAPEAAGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTPPVLDSDGSFFLYSRLT
VDKSRWQEGNVFSCSVMHEALHNHYTQKSLSLSLGKGGGGSGGGGSGGGGSGGGGSSPGQG
TQSENSCTHFPGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENKSKAVEQVKNAFNKLQEKG
IYKAMSEFDIFINYIEAYMTMKIRN.

### Example 2 Preparation of anti-PD1-IL10 fusion protein

A plasmid containing a target gene was constructed and prepared by using a conventional method. The plasmid containing the target gene formed a cationic complex with a transfection reagent PEI, and then introduced to a host cell Expi293; and during the period when the plasmid was in the cell, an exogenous gene on the plasmid underwent transcription and translation in the cell, thereby obtaining a target protein.

The Expi293 was cultured under conditions of 37 °C, 8% carbon dioxide, and 130 rpm, and before transfection, cell counting was performed, and 2E6 cells were inoculated in a 1 L shake flask, with a culture system being approximately 300 mL. A transfection complex was prepared for transfection: first, 750 µg of a target plasmid was added to a 50 mL centrifuge tube containing 15 mL of an Opti-MEM reagent, and the tube was gently mixed and marked as an A tube; 1.5 mg of the transfection reagent PEI was added to a 50 mL centrifuge tube containing 15 mL of an Opti-MEM reagent, and the tube was gently mixed, incubated for 5 min at room temperature, and marked as a B tube; the PEI diluent in the B tube was dropwise added to the DNA diluent in the A tube, the tube was gently mixed and incubated for 15 min at room temperature, and after incubation was completed, the PEI-target plasmid complex was added to the Expi293 cells and placed in a 37 °C shaker for continuous culture. A sample was collected after D5-D10.

An transiently transfected cell expression solution was centrifuged for 20 min at 9000 rpm, supernatant was collected, and then sterile filtration was performed using a 0.22 µM filter membrane. Protein A affinity chromatography was used for purification. A process included the following. An AKTA avant 150 chromatography device was used; at least a 5CV equilibration buffer (10mM PBS) was used to equilibrate a chromatography column (for example, MabSelectSuRe LX, GE); and a sample was loaded to the chromatography column, so as to cause a target protein to adsorb on the chromatography column and cause other impurities to penetrate and separate. After sample loading, the at least 5CV equilibration buffer (10mM PBS) was used to wash the chromatography column again; then an elution buffer (20mM NaAc, pH=3.4) was used to elute the target protein; a neutralizing buffer (1M Tris, pH8.0) was added in a collection tube in advance; and the adding volume of the neutralizing buffer was determined according to estimated content of an elution sample, and 10% elution volume was added generally.

Experimental results for anti-PD1-IL10 fusion protein expression were shown in Table 2. The experimental results showed that an transiently transfected expression of the R1153 reached 204.81 mg/L; experimental results for the sample detected by SEC-HPLC after one-step affinity purification were shown in Table 3. The experimental results showed that the purity of the target protein reached 94.60%. Furthermore, the sample was detected by SDS-PAGE, with an electrophoregram shown in Fig. 2. The experimental results showed the correct pairing of the heavy and light chains in the PD1-IL10 fusion protein.

**Table 2 Experimental results for anti-PD1-IL10 fusion protein expression**

| Sample | Protein content mg | Concentration (mg/mL) | Titer (mg/L) |
|---|---|---|---|
| R1153 | 1659 | 7.9 | 204.81 |

| | | | |
|---|---|---|---|
| Note: Titer referred to an transiently transfected expression concentration. | | | |

**Table 3 Experimental results of SEC-HPLC detection for anti-PD1-IL10 fusion protein sample**

| Sample | H.M.W% | Main% | L.M.W% |
|---|---|---|---|
| R1153 | 5.06 | 94.6 | 0.34 |

| | | | |
|---|---|---|---|
| Note: in the table, H.M.W% referred to High Molecular Weight%; L.M.W% referred to LOW Molecular Weight%; and Main% referred to main peak percentage. | | | |

### Example 3 Binding activity detection for anti-PD1-IL10 fusion protein

The binding activity at an antibody terminus and IL-10 monomer terminus of the anti-PD1-IL10 fusion protein was detected by flow cytometry.

6 g of BSA was weighed and dissolved in 200 mL 1XPBS, well mixed and placed on ice for later use; the molecules to be tested were diluted to an initial concentration of 125 nM (with a prepared concentration 250 nM) by using 3% BSA, a volume was 300 µL, and gradient dilution (100+200) had a total of 11 points; after the cells were counted, 50 µL of cell suspension was dispensed to a 96-well V-shaped plate with 2E+05/well, 50 µL of the molecules with different concentrations were added to the cells, and incubation was performed for 30 min at 2-8 °C; centrifugation was performed for 5 min at 350 xg, supernatant was discarded, 200 µL of 3% BSA was added per well; centrifugation was performed for 5 min at 350 xg, supernatant was discarded, a fluorescent antibody PE Mouse anti-human IgG Fc was prepared by using the 3%BSA (1:500x dilution), the fluorescent antibody was added to the corresponding 96-well V-shaped plate with 100 µL/well, and incubation was performed for 30 min at 2-8 °C; centrifugation was performed for 5 min at 350 xg, supernatant was discarded, the 3% BSA was used to wash the cells according to 200 µl/well; after centrifugation was performed for 5 min at 350 xg, supernatant was discarded, 1XPBS resuspension cells were added according to 100 µl/well; and loading was performed according to Standard Operating Procedures for the CytoFLEX Flow Cytometer, so as to perform FACS detection. CHO-hPD1 cells (CHO cell overexpressing human PD1) were used when the binding of the antibody terminus to the antigen was detected; and an H_IL10 Reporter 293 Cell Line (purchased from Genomeditech) was used to detect binding of the IL-10 terminus to the IL-10 receptor.

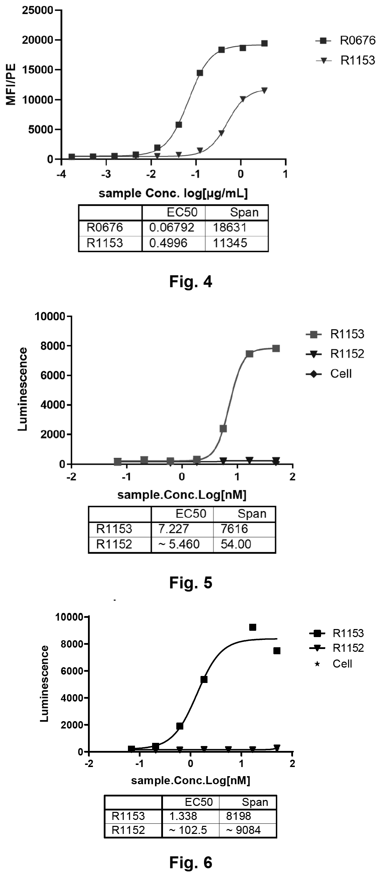

Experimental results were shown in Figs. 3 and 4. The experimental results indicated that compared to the PD1 monoclonal antibody R1152, the binding activity of the antibody terminus of the anti-PD1-IL10 fusion protein and the human PD1 antigen did not change significantly, indicating that the binding activity of the antibody terminus and the antigen thereof was not affected after the antibody and the IL10 monomer were fused into a fusion protein. Compared to an IL10-Fc fusion protein R0676 (SEQ ID NO:22, a dimer fusion protein constructed by directly linking the IL10 and an N-terminus of a human Fc constant region), the binding of the anti-PD1-IL10 fusion protein of the present disclosure to IL10R was significantly weaker.

### Example 4 Activity of anti-PD1-IL10 fusion protein detected by reporter gene method

### 4.1 Activity of IL10 terminus of anti-PD1-IL10 fusion protein detected by reporter gene method

Cell preparation: cultured HEK293-hIL10-Report gene cells (Genomeditech, with an catalog number GM-C07927) were taken and observed under a microscope, normal cell adhesion was shown, particles were bright, and the cells with moderate density might be used as effector cells for the experiment; the above cells were digested with trypsin, digestion was terminated, centrifugation was performed for 4 minutes at 350 xg to remove supernatant, resuspending was performed with 1% FBS-PBS, and then washing was performed; and the supernatant was discarded, a DMEM was used finally to resuspend the cells, the density of the cells was adjusted to 5E5/mL after the cells were counted, a 96-well plate was seeded at 50 µL per well, equivalent to 2.5E4 cells per well, and then an adhesion treatment was performed for 6 h in a 37 °C incubator. R0056FC2-6 cells (CHO-hPD1, CHO cells overexpressing the human PD1) were taken, centrifugation was performed, supernatant was discarded, the cells were counted, the density of the cells was adjusted to 1E6/mL, and the plate was seeded at 25 µL per well, equivalent to 2.5E4 cells per well.

Dilution: the molecules to be tested (R1152 or R1153) were diluted in an Assay Buffer (DMEM) to an initial concentration of 50 nM (dilution concentration of 200 nM), with a volume of 150 µL, a 3-fold gradient dilution (50+100), and 7 concentrations; and experiments were performed under both non-enriched and enriched conditions.

Under the non-enriched condition, the diluted molecule to be tested was directly added to the cells that were well subjected to the adhesion treatment, with 25 µL/well; under the enriched condition, the diluted molecule to be tested with a volume of 25 µL/well was mixed with an equal volume of target cells R0056FC2-6, then co-incubation was performed for an approximately 30 min, and then the mixture was carefully transferred to HEK293 which is subjected to the adhesion treatment for 6 h; 25 µL of the culture medium was additionally added to wells for Cell only, a final volume of all wells was ultimately 100 µL, and edge wells were sealed with 200 µL of sterile water; and the 96-well plate was continuously cultured for 16 h in the incubator.

A Bright-Lumi^{TM} firefly luciferase detection reagent was thawed on the second day and equilibrated to room temperature; after the cells were cultured to a time point, a cell culture plate was taken out and equilibrated to room temperature for 10 min (should not exceed 30 min), 100 µL of the Bright-Lumi^{TM} firefly luciferase detection reagent was added to each well, and incubation was performed for 5-10 min at room temperature with gentle shaking; and after the reaction system was completed, 100 µL was transferred from each well to a 96-well blank plate, a multimode reader was used, and a chemiluminescence mode was used to detect a signal.

Detection results were shown in Figs. 5 and 6. The experimental results indicated that the anti-PD1-IL10 fusion protein R1153 constructed in the present disclosure had high luciferase activity under a CHO-hPD1 enriched condition.

### 4.2 Activity of PD1 binding terminus of anti-PD1-IL10 fusion protein detected by reporter gene method

Cell preparation: the cells were counted, an appropriate amount of CHO-OKT3-hPDL1 cells (CHO cells overexpressing OKT3 and human PDL1) and Jurkat-luc-hPD1 cells (Jurkat cells overexpressing NFAT-luc and human PDL1) were taken, centrifugation was performed for 5 min at 300g, supernatant was discarded, washing was performed once by 1640+10%FBS culture medium, the density of the CHO-OKT3-hPDL1 cells was adjusted to 1E+06 cells/mL after resuspending was performed with the 1640+10%FBS culture medium, and the density of the Jurkat-luc-hPD1 cells was adjusted to 5E+06 cells/mL.

Dilution: the molecule to be tested was diluted to an initial concentration of 250 nM (dilution concentration of 500 nM) by using the 1640+10%FBS culture medium, with a volume of 100 µL, a 2-fold gradient dilution (50+50), and 10 concentrations.

According to an experimental design, 20 µL of the diluent of the molecule to be tested was added to each well of a 96-well blank cell culture flat-bottom plate, the CHO-OKT3-hPDL1 cells were added according to 4E+04 cell/well, that is, 40 µ/well, the Jurkat-luc-hPD1 cells were added according to 2E+05 cell/well, that is, 40 µ/well, and a certain volume of the culture medium was added to the corresponding wells, causing the total volume of all well to be 100 µL; 100 µL of the PBS was added to the outermost circle; the cell culture plate was placed in a CO₂ incubator and incubated for 6 h; the Bright-Lumi^{TM} firefly luciferase detection reagent was thawed in advance and equilibrated to room temperature; after the cells were cultured to a time point, a cell culture plate was taken out and equilibrated to room temperature for 10 min (should not exceed 30 min), 100 µL of the Bright-Lumi^{TM} firefly luciferase detection reagent was added to each well, and incubation was performed for 5-10 min at room temperature with gentle shaking; and chemiluminescent detection was performed through loading.

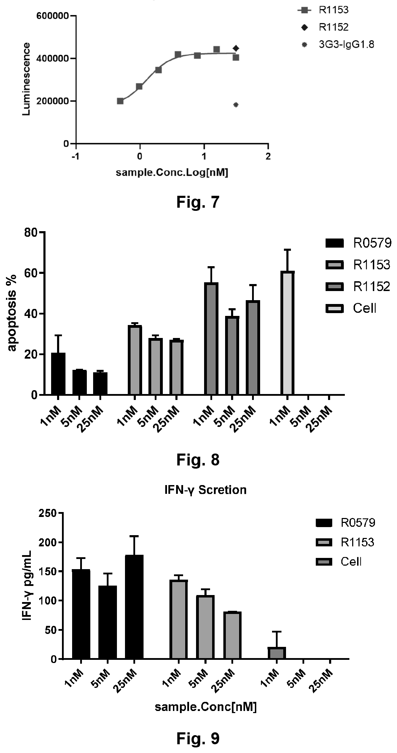

Experimental detection results were shown in Fig. 7. The fluorescent activity of the PD1 binding terminus of the anti-PD1-IL10 fusion protein of the present disclosure was basically consistent with the fluorescent activity of the anti-PD1 antibody R1152, indicating that after the anti-PD1 antibody and the IL10 monomer were fused into a fusion protein, the fluorescence activation activity of the antibody terminus was not affected.

### Example 5 Functional detection of exhausted CD8+T cells mediated by anti-PD1-IL10 fusion protein

Fresh human healthy donor blood was separated to obtain PBMC according to a standard operating procedure; CD8+T cells were separated from the PBMC by using a method provided by a CD8 microbeads kit from Miltenyi Biotec, and purity was determined by flow cytometry using an FITC anti-human CD8 antibody; on D0, the density of the CD8+T cells was adjusted to 1E6 cells/mL by using a 1640+10%FBS+100U/mL IL-2 culture medium, CD3/CD28 magnetic beads were simultaneously added according to a ratio of 1:1, after well mixing, the cells were inoculated into the 96-well flat-bottom plate according to 200 µL/well, equivalent to 2E5 cells per well, and the plate was placed at 37 °C and 5% CO₂ incubator for induction culture; and on D0, an initial expression level of the separated CD8+T cells was determined by using PE anti human IL10R (Biolegend, 308804), FITC anti human PD1 (Biolegend, 329904), and APC anti human TIM3 (Biolegend, 364804) flow cytometry antibodies.

On D1 (D1 indicated the first day of the experiment, D2 indicated the second day, and so on), PD-1-his (R2294, a human PD1 protein carrying a his label) protein was diluted with the DPBS to 5 µg/mL, and the protein was added to a 96-well flat-bottom plate according to a volume of 200 µL/well and incubated overnight at 4 °C; and on D2, the molecules to be tested (R1153 of the present disclosure, control molecules R0579 and R1152, and blank control group cells) were diluted by using the 1640+10%FBS culture medium to a final concentration of 50 nM and 5-fold dilution (3 gradients) for later use. Liquid in the 4 °C overnight plate was discarded and washed for twice with sterile DPBS, then the molecules to be tested with diluted gradients were added, and the mixture was incubated for 0.5 h-1 h in the 37 °C incubator. On D2, during incubation, all CD8+T cells in the 96-well flat-bottom plate were pipetted up and down to detach, collected, and mixed, the beads were removed using a magnet, then the cells were counted, a portion of cells was taken for surface marker expression analysis (including IL10R, PD1, and TIM3), the remaining cells were centrifuged, then the density was adjusted to 1.5E6 cells/mL by using the 1640+10%FBS culture medium, a required cell volume was transferred to a new centrifuge tube, the CD3/CD28 magnetic beads were added at a 1:1 ratio, well mixing was performed, and then the mixture was added to a plate for antibody incubation according to 100 µL (1.5E5 cells) cells per well, and was incubated for 4 d (days) in the 37 °C incubator. On D2, the remaining cells were centrifuged, then the density was adjusted to 5E5 cells/mL by using the 1640+10%FBS+100U/mL IL-2 culture medium, the CD3/CD28 magnetic beads were added at a 1:1 ratio, well mixing was performed, and then the mixture was re-inoculated into the 96-well flat-bottom plate according to the volume of 200 µL(1E5 cells)/well, and was placed at 37 °C for continuous culture stimulation.

Pre-coated plate and antibody incubation were continuously performed according to the above operations; markers were detected and induced CD8+T cells were added to the plate for antibody incubation, the plate was incubated for 4 d in the 37 °C incubator; on D6, apoptosis of cells incubated for 4 d since D2 was detected: the corresponding cells were pipetted up and down to detach and transferred to a V plate, centrifugation was performed at 350 xg, supernatant was discarded, washing was performed once with Annexin-V binding buffer (200µL/well), a staining solution was prepared by using Annexin-V binding buffer (mix 1 µL APC annexin-V and 1 µL Propidium Iodide Solution per well), the staining solution was added according to 100 µL/well and incubated for 15 min at room temperature in the dark, 100 µL of the Annexin-V binding buffer was additionally added to each well and well mixed, and finally, apoptosis was detected by flow cytometry. On D10, apoptosis for 4 d incubation since D6 was detected.

Cytokine detection: cell supernatant after 4d of incubation was collected, and IFN-γ was detected by using a Human IFN-γ uncoated ELISA kit (Invitrogen, 88-7316-77).

Detection results were shown in Figs. 8 and 9. Compared to the Cell group, under the enriched condition with the presence of the Pd-1-his protein, apoptosis of exhausted CD8+ T cells mediated by the anti-PD1-IL10 fusion protein of the present disclosure was significantly reduced, and IFN-γ secretion was lower than that of the positive control R0579.

### Example 6 In-vivo drug efficacy study on anti-PD1-IL10 fusion protein

Objective: the anti-tumor activity of the anti-PD1-IL10 fusion protein against CT-26 mouse colon cancer was observed, an isotype control group, a monotherapy control group, and a combination therapy group were established simultaneously.

Experimental materials: hPD1 Knock-in mice, female, 6-8 weeks old (Balb/c background, Source: GemPharmatech Co., Ltd., Animal Quality Certificate No. 320327230100208956); CT-26 cells (Shanghai Cell Bank, catalog No.: TCM37); RPMI 1640 culture medium (Gibco, 11875085), FBS (Gibco, 10091-148), 0.25% trypsin-EDTA (Gibco, 25200056), Penicillin-Streptomycin (Gibco, 15140122), and DPBS (Hyclone, SH30028.02).

Instruments and devices: Electronic balance (Shanghai Sunny Hengping Instrument, JA12002), vernier caliper (Shanghai Meinaite Industrial Co., Ltd., MNT-150T), microscope (Chongqing Opte Instrument Co., Ltd., BDS200), medical centrifuge (Hunan CENCE Laboratory Development Co., Ltd., L530R), digital constant temperature water bath (PRS Machinery Co., Ltd., HH-S), CO₂ incubator (Panasonic Healthcare Co., Ltd., Japan, MCO-18AC), biosafety cabinet (GuangZhou Rivers Laboratory Technology Co.,Ltd, ACZ-451), and cell counter (Shanghai Ruiyu Cell counter Co., Ltd., IC1000).

### Test method

Cell culture: the mouse colon cancer cells (CT-26) were cultured in an RPMI 1640 culture medium containing 10% fetal bovine serum (FBS) and 1% penicillin-streptomycin (1:1), and the culture medium was placed in a 37 °C 5% CO₂ incubator for culture.

Inoculation: CT-26 cells at a logarithmic growth phase were collected and washed twice with DPBS, and a cell concentration was regulated to 1X10⁶ cells/mL using DPBS. Female hPD1 mice were taken, and CT-26 cells were inoculated subcutaneously at a volume of 0.1 mL/mouse, equivalent to 1×10⁵/mouse.

Administration: the day of inoculation was recorded as Day 0 (D0). When an average tumor volume reached 80-100 mm³, mice might be randomly grouped based on tumor volume, ensuring that each group had an identical or similar average tumor volume. On Day 9 (D9) of the experiment, the mice were randomly grouped into 5 groups based on the tumor volume, with 7 mice in each group, and then the mice started to be administered (administration regimen shown in Table 4 below). The R1153 group received a dose of 10 mg/kg, while other groups received doses equivalent to the molar amount of R1153 (10 mg/kg).

**Table 4 Administration dose, administration route, and frequency of CT-26 tumor model**

| Group | Dosage (mg/kg) | Administration volume (µL/g) | Administration route | Administration frequency |
|---|---|---|---|---|
| Isotype control_hIgG1.8 | 8 | 10 | i.p. | Q3D*4 |
| R0676 | 5 | 10 | i.p. | Q3D*4 |
| R1152 | 7.9 | 10 | i.p. | Q3D*4 |
| R1153 | 10 | 10 | i.p. | Q3D*4 |
| R0676+R1152 | 5+7.9 | 10 | i.p. | Q3D*4 |

| | | | | |
|---|---|---|---|---|
| Note: i.p., intraperitoneal injection; Q3D, administer once every 3 days; if weight loss exceeded 15% during administration, the administration regimen would be adjusted. | | | | |

Recording: the tumor volume was measured and recorded from D9 onwards; and long and short diameters of the tumor were measured by using a vernier caliper twice per week. The tumor volume was calculated according to a formula: (1/2)×long diameter×(short diameter)² (shown in Table 5). When each mouse reached an experimental endpoint (weight loss exceeding 20% or tumor volume exceeding 2000 mm³ to reach a humane endpoint), and the mice were euthanized by CO₂ asphyxiation.

**Table 5 Anti-tumor activity of PD1-IL10 fusion protein in CT-26 mouse colon cancer model**

| Study on Reduction of tumor volume (mm³) in hPD1 Mice with CT-26 colon cancer by PD1-IL10 fusion protein | | | | | |
|---|---|---|---|---|---|
| Days after implantation | Isotype control | R0676 | R1152 | R1153 | R1152+R0676 |
| 9 | 86.97 | 84.87 | 85.25 | 84.10 | 85.95 |
| 13 | 140.22 | 161.88 | 142.71 | 88.35 | 150.96 |
| 16 | 245.15 | 154.80 | 169.60 | 76.29 | 125.59 |
| 20 | 433.94 | 127.15 | 157.22 | 38.02 | 79.61 |
| 23 | 700.08 | 180.18 | 207.69 | 29.79 | 69.3 |
| 27 | 1352.84 | 308.83 | 331.26 | 24.14 | 102.53 |
| TGI%, D27 | - | 82.31% | 80.57% | 104.74% | 98.69% |
| CR, D27 | 0/7 | 1/7 | 1/7 | 4/7 | 2/7 |

| | | | | | |
|---|---|---|---|---|---|
| Data computation, statistics, and analysis: | | | | | |

Independent sample T-tests might be used to compare two groups. For comparisons involving three or more groups, One-Way ANOVA was used. If the F-value indicated a significant difference, post-hoc analyses among a plurality of groups might be performed. Data were processed by using Prism GraphPad, and when p<0.05, it indicated a statistically significant difference. Tumor volume (TV) = 0.5×a×b2, where a and b represented the long and short diameters of the tumor, respectively. Tumor Growth Inhibition (TGI) (%) = [1-(Ti-T0)/(Vi-V0)]×100, where Ti was an average tumor volume of a treatment group on day i, T0 was an average tumor volume of the treatment group at the start of treatment, Vi was an average tumor volume of a solvent control group on Day i, and V0 was an average tumor volume of the solvent control group at the start of treatment.

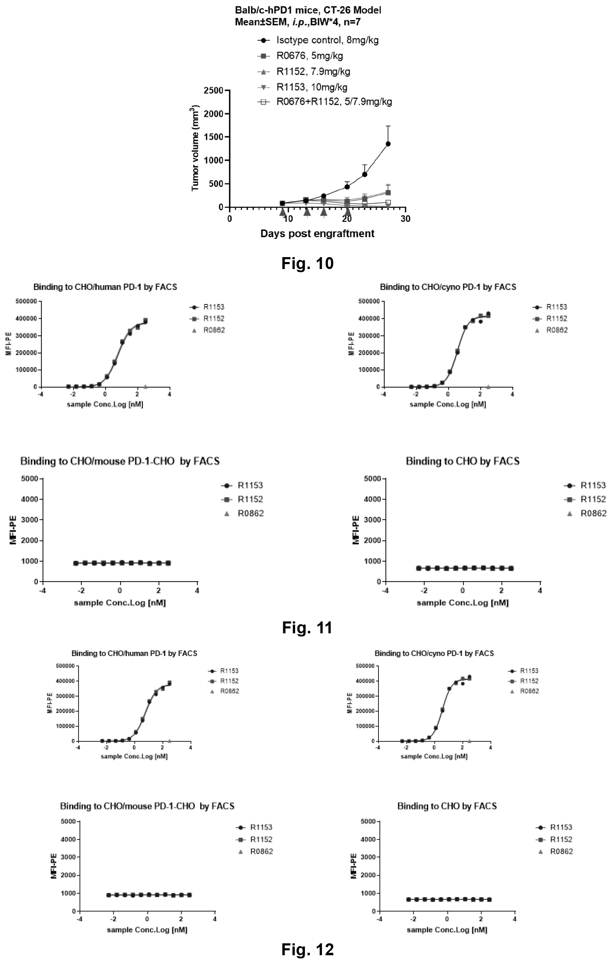

The experimental results were shown in Table 5 and Fig. 10. The experimental results showed that the anti-PD1-IL10 fusion protein R1153 (TGI=104.74%, CR: 4/7) showed significant antitumor efficacy in the hPD1 mouse CT-26 colon cancer model, markedly superior to both the PD1 monoclonal antibody R1152 (TGI=80.57%, CR: 1/7) and the IL10 fusion protein R0676 (TGI=82.31%, CR: 1/7) when used alone.

### Example 7 Early-stage safety assessment of anti-PD1-IL10 fusion protein

Test I: the test was intended to administer R0674 intravenously to cynomolgus monkeys. One week after the initial dose, a plurality of administrations were performed twice a week for a total of six doses (including the initial dose). Toxicity reactions induced by R0674 were observed and its toxicokinetic (pharmacokinetic) characteristics were investigated, thereby providing reference for subsequent test design. In the test, the R0674 included two groups, with doses of 1 mg/kg and 3 mg/kg, respectively. Furthermore, a solvent control group was additionally set. During the test, the following observations were performed on animals: death and near death, clinical observation, weight, food intake, body temperature, hematological and coagulation indicator detection, serum biochemical indicators, and immune functional detection. On D27, the animals were euthanized, and gross anatomy was performed. Major organs (adrenal glands, brain, epididymis, heart, kidneys, liver, spleen, testes, thymus, thyroid gland, and parathyroid glands) were weighed. Histopathological examination was performed on the liver, spleen, and kidneys.

During the experiments, no significant abnormalities were observed in animal death and near-death, clinical observation, weight, food intake, body temperature, blood coagulation, immune functional detection, and organ weights.

Hematology: compared with the solvent control group, the low-dose R0674 group (1 mg/kg) showed on D15: RBC↓ (-37.4%), HGB↓ (-37.0%), HCT↓ (-25.6%); and the high-dose group (3 mg/kg) at the same time point also showed RBC↓ (-13.6%), HGB↓ (-15.7%), and HCT↓ (-12.8%). The above abnormal indicators were considered to be related to test samples. On D28, the above abnormal indicators had basically returned to normal. (Note: "↑" indicated an increase in the corresponding value compared to the solvent control group, while "↓" indicated a decrease in the corresponding value compared to the solvent control group. For example, "RBC↓(-37.4%)" indicated that a RBC value decreased by 37.4% compared to the solvent group, and other cases followed the same pattern.

Serum biochemistry: compared with the solvent control group, the high-dose R0674 group (3 mg/kg) showed the following serum biochemical indicators on D23: ALT↑, AST↑, CK↓, BUN↑, and CREA↑.

Toxicokinetics (pharmacokinetics): [Exposure-accumulation relationship]: exposure to R0674 (AUC(0-t) and Cmax) increased with rising doses. Following D1 administration, the increase in animal exposure was proportional to the dose. After D22 administration (the third dose), the increase in animal exposure exceeded the proportional increase in dose. [Accumulation status]: After 22 days, no significant accumulation was observed in any dose group of R0674.

Cytokines: during the experiment, compared with the solvent control group, no abnormalities were observed in the cytokines IFN-γ, IL-17A, IL-1β, IL-4, IL-5, and TNF-α in the low-and high-dose R0674 groups before the first administration, 6 hours after the first administration, and 6 hours after administration on D22. Cytokine IL-6 was detected in all R0674 dose groups at 6 hours after the first administration and 6 hours after the administration on D22. However, it was also detected in the solvent control group, with a detection value slightly above a lower limit of quantification, which was considered to be a baseline fluctuation. Detected values of the cytokine IL-10 in both the low-and high-dose R0674 groups exceeded an upper limit of quantification 6 hours after the first administration.

To sum up, R0674 was administered intravenously to the cynomolgus monkeys once during Week 1 (D1, i.e., on the first day of the experiment), followed by twice a week (D8, D11, D15, D18, and D22) with a total of six doses at doses of 1 mg/kg and 3 mg/kg. During the test, no significant abnormalities related to the test sample were observed in the clinical observation, weight, food intake, body temperature, blood coagulation function, and immune function of the animals. R0674 (≥1 mg/kg) at D15 showed decreased red blood cell parameters (RBC↓, HGB↓, HCT↓), which were considered to be related to the test sample, but these parameters basically recovered by D28. High-dose R0674 group (3 mg/kg) showed ALT↑, AST↑, CK↓, BUN↑, and CREA↑. Moreover, gross anatomy observation indicated gray discoloration on both sides of the kidneys. Histopathological analysis indicated moderate tube cast in kidneys, moderate dilatation of renal tubules, mild tubular single-cell necrosis, mild tubular nephritis, slight mineralization of renal tubules, and mild interstitial mononuclear cell infiltration. Potentially test sample-related histopathological changes observed at the end of the administration period were seen in eosinophilic material deposition in a germinal center of the white pulp of the spleen in the R0674 dose group.

Test II: the test was intended to administer the anti-PD1-IL10 fusion protein R1153 intravenously to the cynomolgus monkeys once a week for 3 consecutive weeks, with a total of four doses (D1, D8, D15, and D22). The first day of administration was defined as D1. The triggered toxic reactions were observed and their pharmacokinetic characteristics were studied, thereby providing reference for subsequent test design. The test included 2 groups, Group 1 was the solvent control group, which received no administration on D1, D8, and D15, with a 10 mg/kg dose administered on D22. Group 2 was the test sample group, which received doses of 1 mg/kg, 3 mg/kg, 10 mg/kg, and 10 mg/kg on D1, D8, D15, and D22, respectively. During the test, the following observations were performed on animals: death and near death, clinical observation, weight, body temperature, hematological and coagulation indicator detection, serum biochemical indicators, and immune functional detection.

During the experiments, no significant abnormalities were observed in animal death and near-death, clinical observation, weight, body temperature, blood coagulation, and immune functional detection.

Hematology: No significant abnormalities observed.

Serum biochemistry: No significant abnormalities observed.

Lymphocyte subtype: No significant abnormalities observed.

Cytokines: No significant abnormalities observed.

To sum up, the cynomolgus monkeys were intravenously administered with the anti-PD1-IL10 fusion protein R1153 once a week at doses of 1 mg/kg on D1, 3 mg/kg on D8, 10 mg/kg on D15, and 10 mg/kg on D22. During the test, no test sample-related abnormalities were observed in the clinical observation, weight, body temperature, and blood coagulation and immune functional detection (immune lymphocyte subtypes and serum cytokines) of the animals. In Test I, the Fc-IL10 fusion protein R0674 showed significant abnormalities including decreased red blood cell parameters, elevated liver function indicators, and increased cytokine levels. These abnormalities did not occur following administration of R1153, indicating significantly improved safety for the anti-PD1-IL10 fusion protein R1153.

Appendix I: Inspection parameter indicators were shown in Tables 6-9.

**Table 6 Blood cell count (ADVIA^{®} 2120 series blood analyzer)**

| Abbreviation in English | Detection item | Unit | Detection method |
|---|---|---|---|
| WBC | White blood cell count | ×10⁹/L | Basophil granulocyte/polymorphonuclear method+flow cytometry+peroxidase staining method |
| Neut | Neutrophil | ×10⁹/L& % | Peroxidase staining method+basophil granulocyte/polymorphonuclear method |
| Lymph | Lymphocyte | ×10⁹/L& % | Peroxidase staining method+basophil granulocyte/polymorphonuclear method |
| Mono | Mononuclear cell | ×10⁹/L& % | Peroxidase staining method+basophil granulocyte/polymorphonuclear method |
| Eos | Eosinophil | ×10⁹/L& % | Peroxidase staining method+basophil granulocyte/polymorphonuclear method |
| Baso | Basophil granulocyte | ×10⁹/L& % | Peroxidase staining method+basophil granulocyte/polymorphonuclear method |
| RBC | Red blood cell count | ×10¹²/L | Two-dimensional laser streaming technology |
| HGB | Hemoglobin | g/L | Cyanide methemoglobin colorimetric method |
| HCT | Hematocrit | % | Calculation according to RBC and MCV |
| Retic | Reticulocyte count | ×10¹²/L&% | Laser flow cytometry technology+nucleic acid fluorescence staining method |
| MCV | Mean corpuscular volume | fL | Calculation |
| MCHC | Mean corpuscular hemoglobin concentration | g/L | Calculation |
| MCH | Mean corpuscular hemoglobin | pg | Calculation |
| PLT | Platelet count | ×10⁹/L | Two-dimensional laser streaming technology |

| | | | |
|---|---|---|---|
| Note: This study analyzed only the above measured indicators; and other indicators measured by this instrument were not analyzed. | | | |

**Table 7 Blood cell count (Sysmex^{®} XN series fully-automatic modular blood and body fluid analyzer)**

| Abbreviation in English | Detection item | Unit | Detection method |
|---|---|---|---|
| WBC | White blood cell count | ×10⁹/L | Semiconductor laser+flow cytometry |
| Neut | Neutrophil | ×10⁹/L&% | Semiconductor laser+flow cytometry |
| Lymph | Lymphocyte | ×10⁹/L&% | Semiconductor laser+flow cytometry |
| Mono | Mononuclear cell | ×10⁹/L&% | Semiconductor laser+flow cytometry |
| Eos | Eosinophil | ×10⁹/L&% | Semiconductor laser+flow cytometry |
| Baso | Basophil granulocyte | ×10⁹/L&% | Semiconductor laser+flow cytometry |
| RBC | Red blood cell count | ×10¹²/L | Impedance method |
| HGB | Hemoglobin | g/L | SLS-hemoglobin method |
| HCT | Hematocrit | % | Calculation |
| Retic | Reticulocyte count | ×10¹²/L&% | Semiconductor laser+flow cytometry |
| MCV | Mean corpuscular volume | fL | Calculation |
| MCHC | Mean corpuscular hemoglobin concentration | g/L | Calculation |
| MCH | Mean corpuscular hemoglobin | pg | Calculation |
| PLT | Platelet count | ×10⁹/L | Impedance method |

| | | | |
|---|---|---|---|
| Note: This study analyzed only the above measured indicators; and other indicators measured by this instrument were not analyzed. | | | |

**Table 8 Blood biochemical indicators (TBA-120FR fully-automatic biochemical analyzer)**

| Abbreviation in English | Detection item | Unit | Detection method |
|---|---|---|---|
| ALT | Alanine aminotransferase | U/L | Alanine substrate method |
| AST | Aspartate aminotransferase | U/L | Aspartate substrate method |
| TP | Total protein | g/L | Biuret method |
| Alb | Albumin | g/L | Bromocresol green method |
| Glb | Globulin | g/L | Calculation method |
| A/G | Albumin/globulin | - | Calculation method |
| TBil | Total bilirubin | µmol/L | Chemical oxidation method |
| LDH | Lactic dehydrogenase | U/L | Lactic substrate method |
| ALP | Alkaline phosphatase | U/L | NPP substrate-AMP buffer method |
| CK | Creatine kinase | U/L | Phosphocreatine substrate method |
| GGT | γ-glutamyltransferase | U/L | GCANA substrate method |
| UREA | Urea | mmol/L | Urease-glutamate dehydrogenase method |
| Cre | Creatinine | µmol/L | Creatine oxidase method |
| CHO | Total cholesterol | mmol/L | CHOD-PAP method |
| TG | Triglyceride | mmol/L | GPO-PAP method |
| Glu | Glucose | mmol/L | Hexokinase method |
| Ca | Calcium | mmol/L | Arsenazo III method |
| P | Phosphorus | mmol/L | Phosphomolybdate method |
| Na+ | Sodium ion concentration | mmol/L | Electrode method |
| K+ | Potassium ion concentration | mmol/L | Electrode method |
| Cl- | Chloride ion concentration | mmol/L | Electrode method |

| | | | |
|---|---|---|---|
| Table 9 Blood biochemical indicators (Beckman Coulter AU5800 fully-automatic biochemical analyzer) | | | |

| Abbreviation in English | Detection item | Unit | Detection method |
|---|---|---|---|
| ALT | Alanine aminotransferase | U/L | Lactic dehydrogenase method |
| AST | Aspartate aminotransferase | U/L | MDH method |
| TP | Total protein | g/L | Biuret method |
| Alb | Albumin | g/L | Bromocresol green method |
| Glb | Globulin | g/L | Calculation method |
| A/G | Albumin/globulin | - | Calculation method |
| TBil | Total bilirubin | µmol/L | Diazonium salt method |
| LDH | Lactic dehydrogenase | U/L | Lactic substrate method |
| ALP | Alkaline phosphatase | U/L | NPP substrate-AMP buffer method |
| CK | Creatine kinase | U/L | Enzyme coupling method |
| GGT | γ-glutamyltransferase | U/L | Rate method |
| UREA | Urea | mmol/L | Urease-glutamate dehydrogenase method |
| Cre | Creatinine | µmol/L | Creatine oxidase method |
| CHO | Total cholesterol | mmol/L | Enzyme method |
| TG | Triglyceride | mmol/L | GPO-POD method |
| Glu | Glucose | mmol/L | Hexokinase method |
| Ca | Calcium | mmol/L | Arsenazo III method |
| P | Phosphorus | mmol/L | Phosphomolybdate method |
| Na+ | Sodium ion concentration | mmol/L | Electrode method |
| K+ | Potassium ion concentration | mmol/L | Electrode method |
| Cl- | Chloride ion concentration | mmol/L | Electrode method |

Immune cell subtyping: a BD FACSCalibur flow cytometer and/or a BD LSR Fortessa^{TM} flow cytometer and/or a BD FACSCanto^{TM} II flow cytometer were used, the percentages of total T lymphocytes (CD45⁺CD3⁺) in peripheral blood, Th cells (CD45⁺CD3⁺CD4⁺), Tc cells (CD45⁺CD3⁺CD8⁺), Treg cells (CD45⁺CD3⁺CD4⁺CD25⁺Foxp3⁺), and mononuclear cells (CD14⁺) were detected, an absolute value was calculated in combination with blood cell counting results, and a ratio of CD3⁺CD4⁺/CD3⁺CD8⁺ was determined.

Cytokines: Interleukin-2 (IL-2), Interleukin-4 (IL-4), Interleukin-6 (IL-6), Interleukin-10 (IL-10), Tumor Necrosis Factor-α (TNF-α), and Interferon-γ (IFN-γ) in serum were detected with a commercialized kit (BioLegend^{®}) by using the BD FACSCalibur flow cytometer and/or the BD LSR Fortessa^{TM} flow cytometer and/or the BD FACSCanto^{TM} II flow cytometer.

### Example 8 Detection of binding activity of R1153 to CHO cells expressing human, monkey, and mouse PD-1 by FACS

CHO/human PD-1, CHO/cyno PD-1, and CHO/mouse PD-1 cells were taken out from liquid nitrogen, rapidly transferred to a 37 °C water bath for thawing, then gently agitated until only a small amount of ice crystals remained, and then transferred to a biosafety cabinet. 1 mL of cell suspension was pipetted into a centrifuge tube containing 10 mL of a fresh CD-CHO culture medium, and gently mixed through blowing, centrifugation was performed for 5 min at 300 g, supernatant was discarded, resuspending was performed with 10 mL of the fresh CD-CHO culture medium, and culture was performed for 2 days in a 37 °C 5% CO₂ incubator. Cell counting: cell counting was performed on the CHO/human PD-1, CHO/cyno PD-1, and CHO/mouse PD-1 cells to be tested, and the cells were centrifuged for 5 min at 300×g, resuspended with a FACS buffer, and adjusted to a density of 4E+06 cells/mL; and each cell line was added to a 96-well V-shaped plate with 50 µL/well, achieving the number of the cells in each well to be 2E+05. The diluted sample was added to a 96-well V-shaped plate with 50 µL/well, and incubated on ice in the dark for 30 min; the 96-well V-shaped plate was centrifuged at 300×g for 5 min, the supernatant was discarded, 200 µL of the FACS buffer was added for one wash, centrifugation was performed for 5 min at 300×g, and the supernatant was discarded; a diluted secondary antibody (1:500) was added to the 96-well V-shaped plate with 100 µL/well, and incubated on ice in the dark for 30 min; the 96-well V-shaped plate was centrifuged at 300×g for 5 min, the supernatant was discarded, 200 µL of the FACS buffer was added for one wash, centrifugation was performed for 5 min at 300×g, and the supernatant was discarded; 100 µL 1×PBS was added for resuspending, and the 96-well V-shaped plate was placed in a flow cytometer for detection; and a curve was plotted by using GraphPad Prism 8.3.0, and an EC₅₀ value was determined by using a four-parameter method.

**Table 10 Summary of FACS results for fusion protein PD-1 terminus**

| EC₅₀ (nM) | CHO/human PD-1 | CHO/cyno PD-1 | CHO/mouse PD-1 | CHO |
|---|---|---|---|---|
| R1153 | 6.045 | 3.587 | Not binding | Not binding |
| R1152 | 5.469 | 3.469 | Not binding | Not binding |
| R0862 | Not binding | Not binding | Not binding | Not binding |

Under the conditions of this experiment, the results were shown in Fig. 11 and Table 10. Both R1153 and the corresponding PD-1 parent antibody R1152 specifically bound to the CHO/human PD-1 cells and cross-reacted with the CHO/cyno PD-1 cells with comparable binding activity. However, the R1153 and the R1152 did not cross-react with the CHO/mouse PD-1 cells. The isotype control R0862 did not cross-react with any of the three cell types.

### Example 9 Detection of binding activity of R1153 to CHO cells expressing human, monkey, and mouse IL10R1 by FACS

Cell resuscitation: CHO/human IL10R1, CHO/cyno IL10R1, and CHO/mouse IL10R1 cells were taken out from a liquid nitrogen tank, rapidly transferred to a 37 °C water bath for thawing, then gently agitated until only a small amount of ice crystals remained, and then transferred to a biosafety cabinet. 1 mL of cell suspension was pipetted into a centrifuge tube containing 10 mL of a fresh CD-CHO culture medium, and gently mixed through blowing, centrifugation was performed for 5 min at 300 g, supernatant was discarded, resuspending was performed with 10 mL of the fresh CD-CHO culture medium, and culture was performed for 2 days in a 37 °C 5% CO₂ incubator. Cell counting: cell counting was performed on the CHO/human IL10R1, CHO/cyno IL10R1, and CHO/mouse IL10R1 cells to be tested, and the cells were centrifuged for 5 min at 300×g, resuspended with a FACS buffer, and adjusted to a density of 4E+06 cells/mL. Each cell line was added to a 96-well V-shaped plate with 50 µL/well, achieving the number of the cells in each well to be 2E+05. The diluted sample was added to a 96-well V-shaped plate with 50 µL/well, and incubated on ice in the dark for 30 min; the 96-well V-shaped plate was centrifuged at 300×g for 5 min, the supernatant was discarded, 200 µL of the FCM buffer was added for one wash, centrifugation was performed for 5 min at 300×g, and the supernatant was discarded; a diluted secondary antibody (1:400) was added to the 96-well V-shaped plate with 100 µL/well, and incubated on ice in the dark for 30 min; the 96-well V-shaped plate was centrifuged at 300×g for 5 min, the supernatant was discarded, 200 µL of the FACS buffer was added for one wash, centrifugation was performed for 5 min at 300×g, and the supernatant was discarded; 100 µL 1×PBS was added for resuspending, and the 96-well V-shaped plate was placed in a flow cytometer for detection; and a curve was plotted by using GraphPad Prism 8.3.0, and an EC₅₀ value was determined by using a four-parameter method.

**Table 11 Summary of FACS results for fusion protein IL10 terminus**

| EC₅₀ (nM) | CHO/human IL10R1 | CHO/cyno IL10R1 | CHO/mouse IL10R1 |
|---|---|---|---|
| R1153 | 8.861 | 4.652 | 2.320 |
| R1187 | 8.507 | 4.251 | 2.138 |
| R3361 | 8.665 | 5.029 | 2.457 |
| R0862 | Not binding | Not binding | Not binding |

R1187: ISO-hIgG1.8-IL10M, a heavy chain amino acid sequence was as set forth in SEQ ID NO:32, and a light chain amino acid sequence was as set forth in SEQ ID NO:33.

QVQLVESGGGVVQPGRSLRLSCAASGFTFSSYGMHWVRQAPGKGLEWVAAIWYDGSNKYY
TDSVKGRFTISRDNSKNTLYLQMNSLRAEDTAVYYCARDLRGVMYFDLWGRGTLVTVSSASTKGP
SVFPLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPGK
(SEQ ID NO:32)

EIVLTQSPATLSLSPGERATLSCRASQSVSRYLAWYQQKPGQAPRLLIYDASNRATGIPARFSG
SGSGTDFTLTISSLEPEDFAVYYCQLRNNWPLTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEV
THQGLSSPVTKSFNRGECGGGGSGGGGSGGGGSGGGGSGGQGTQSENSCTHFPGNLPNMLR
DLRDAFSRVKTFFQMKDQLDNLLLKESLLEDFKGYLGCQALSEMIQFYLEEVMPQAENQDPDIKA
HVNSLGENLKTLRLRLRRCHRFLPCENGGGSGGKSKAVEQVKNAFNKLQEKGIYKAMSEFDIFINY
IEAYMTMKIRN (SEQ ID NO:33)

R3361: ISO-hIgG1.8-wtIL10, a heavy chain amino acid sequence was as set forth in SEQ ID NO:34, and a light chain amino acid sequence was as set forth in SEQ ID NO:35.

QVQLVESGGGVVQPGRSLRLSCAASGFTFSSYGMHWVRQAPGKGLEWVAAIWYDGSNKYY
TDSVKGRFTISRDNSKNTLYLQMNSLRAEDTAVYYCARDLRGVMYFDLWGRGTLVTVSSASTKGP
SVFPLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPGAGG
GGSGGGGSGGGGSGGGGSGSPGQGTQSENSCTHFPGNLPNMLRDLRDAFSRVKTFFQMKDQ
LDNLLLKESLLEDFKGYLGCQALSEMIQFYLEEVMPQAENQDPDIKAHVNSLGENLKTLRLRLRRC
HRFLPCENKSKAVEQVKNAFNKLQEKGIYKAMSEFDIFINYIEAYMTMKIRN (SEQ ID NO:34)

EIVLTQSPATLSLSPGERATLSCRASQSVSRYLAWYQQKPGQAPRLLIYDASNRATGIPARFSG
SGSGTDFTLTISSLEPEDFAVYYCQLRNNWPLTFGGGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEV
THQGLSSPVTKSFNRGEC (SEQ ID NO:35)

Under the conditions of this experiment, as shown in Fig. 12 and Table 11, the R1153, the control antibody R1187, and the R3361 all specifically bound to the CHO/human IL10R1 cells, and showed cross-species binding activity with the CHO/cyno IL10R1 and CHO/mouse IL10R1 cells, with comparable binding activity. The isotype control molecule R0862 had no binding activity with any of the three cell types.

### Example 10 Detection of reporter gene activity of PD-1 terminus of R1153 by luciferase reporter gene method

Cell resuscitation: CHO-OKT3-hPD-L1 cell (i.e., overexpressing OKT3 and hPDL-1 in CHO cells) and Jurkat-NFAT-luc-hPD-1 cell (i.e., overexpressing NFAT-LUC and hPD1 in Jurkat cells) cryopreservation tubes were taken out from a liquid nitrogen tank and rapidly transferred to a 37 °C water bath for thawing, then gently agitated until only a small amount of ice crystals remained, and then transferred to a biosafety cabinet. 1 mL of a CHO-OKT3-hPD-L1 cell cryopreservation solution was pipetted and added to a centrifuge tube containing 5 mL of a CD-CHO fresh culture medium. 1 mL of a Jurkat-NFAT-luc-hPD-1 cell cryopreservation solution was pipetted and added to a centrifuge tube containing 5 mL of 5mL of a RPMI 1640+10% FBS fresh culture medium. Well mixing was performed, then centrifugation was performed for 5 min at 300×g, the supernatant was discarded, resuspending was performed by using 10 mL of a fresh culture medium, and finally, culture was performed for 2 days in a 37 °C 5% CO₂ incubator. Cell preparation: the cells were counted, an appropriate amount of CHO-OKT3-hPD-L1 and Jurkat-NFAT-luc-hPD-1 cells was taken, centrifugation was performed for 5 min at 300×g, the supernatant was discarded, washing was performed once by using the RPMI 1640+10% FBS culture medium, and counting was performed after resuspending was performed by using the RPMI 1640+10% FBS culture medium. The density of the CHO-OKT3-hPD-L1 cells was adjusted to 1E+06 cells/mL and the density of the Jurkat-NFAT-luc-hPD-1 cells was adjusted to 5E+06 cells/mL. 20 µL of the diluted antibody diluent to be tested was added to each well of a white 96-well plate. The CHO-OKT3-hPD-L1 cells were added based on 4E+04 cells/well, that is, 40 µL/well, and the Jurkat-NFAT-luc-hPD-1 cells were added based on 2E+05 cells/well, that is, 40 µL/well, sequentially. A specific volume of the culture medium was added to the corresponding wells to cause a total volume of 100 µL in all wells, and 100 µL of PBS was added to the blank wells; the cell culture plate was placed in a 37 °C 5% CO₂ incubator and incubated for 6 h; a Bright-Lumi^{TM} firefly luciferase detection reagent was thawed in advance and equilibrated to room temperature; after the cells were cultured to a time point, the cell culture plate was taken out and equilibrated to room temperature for 10 min, 100 µL of the Bright-Lumi^{TM} firefly luciferase detection reagent was added to each well, and incubation was performed for 5 min at room temperature; chemiluminescence detection was performed in a multimode reader; and a curve was plotted by using GraphPad Prism 8.3.0, and an EC50 value was determined by using a four-parameter method.

**Table 12 Summary of reporter gene results for fusion protein PD-1 terminus**

| Test sample name | EC₅₀ (nM) |
|---|---|
| R1153 | 5.068 |
| R1152 | 6.844 |
| R0862 | NA |

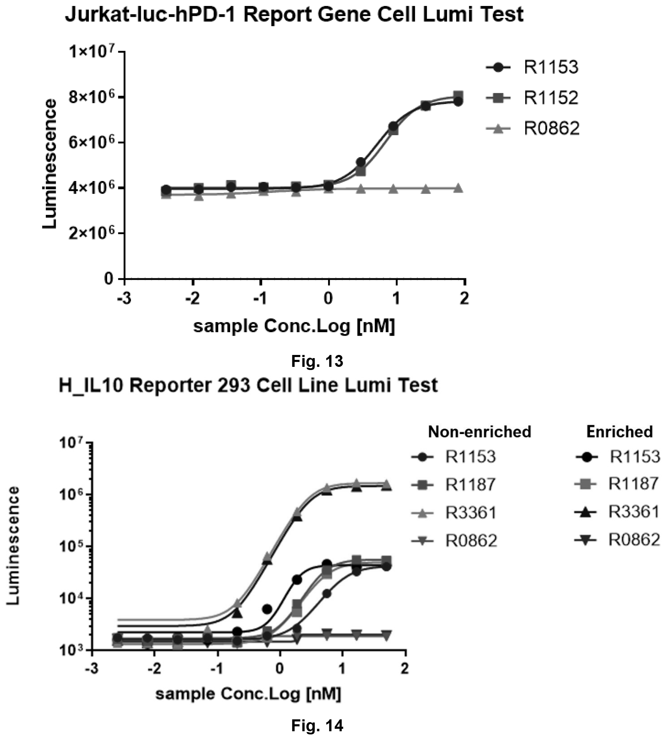

The test results showed that, as shown in Fig. 13 and Table 12, a reporter gene activity EC50 value for the antibody R1153 to be tested was 5.068 nM, and a reporter gene activity EC50 value for the PD-1 parent antibody R1152 was 6.844 nM. Both the R1153 and the corresponding PD-1 parent antibody R1152 had good reporter gene activity, with comparable activity. The isotype control molecule R0862 had no reporter gene activity.

### Example 11 Detection of reporter gene activity of IL10 terminus of R1153 by luciferase reporter gene method

Cell resuscitation: H_IL10 Reporter 293 Cell Line cell and CHO/human PD-1 cell cryopreservation tubes were taken out from a liquid nitrogen tank, rapidly transferred to a 37 °C water bath for thawing, then gently agitated until only a small amount of ice crystals remained, and then transferred to a biosafety cabinet. 1 mL of a CHO/human PD-1 cell cryopreservation solution was pipetted and added to a centrifuge tube containing 5 mL of a CD-CHO fresh culture medium. 1 mL of a H_IL10 Reporter 293 Cell Line cell cryopreservation solution was pipetted and added to a centrifuge tube containing 5mL of a DMEM+10% FBS fresh culture medium. Well mixing was performed, then centrifugation was performed for 5 min at 300×g, the supernatant was discarded, resuspending was performed by using 10 mL of a fresh culture medium, and finally, culture was performed for 2 days in a 37 °C 5% CO₂ incubator. The cultured H_IL10 Reporter 293 cell line was digested with 2 mL trypsin. After digestion was terminated with 5 mL of the DMEM+10% FBS culture medium, the cells were centrifuged at 300×g for 5 min, the supernatant was discarded, resuspending was performed by using 10 mL of 1% FBS-PBS, then centrifugation was performed for 5 min at 300×g, the supernatant was discarded, and finally, the cells were resuspended in the DMEM+10% FBS culture medium. The density of the H_IL10 Reporter 293 Cell Line cells was adjusted to 5E5/mL. A 96-well flat-bottom plate was seeded at 50 µL per well, equivalent to 2.5E4 cells/well; and then the plate was placed in a 37°C incubator and subjected to an adhesion treatment for 6 h. Antibody dilution: all antibody molecules were diluted to 200 nM by using the DMEM+10% FBS culture medium, with a 3-fold gradient dilution and 10 concentration gradients; CHO/human PD-1 cells were taken, centrifuged to remove the supernatant, counted, adjusted to a density of 1E6/mL, and spread based on 25 µL per well, equivalent to 2.5E4 cells/well.

Non-enrichment system: 25 µL candidate antibody+25 µL DMEM+10% FBS+50 µL H_IL10 Reporter 293 Cell Line. That is, the well-diluted antibody to be tested was directly added to the H_IL10 Reporter 293 Cell Line cells that is subjected to the adhesion treatment. Enrichment system: 25 µL candidate antibody+25 µL CHO/human PD-1+50 µL H_IL10 Reporter 293 Cell Line. First, the well-diluted antibody to be tested and target cell CHO/human PD-1 were mixed in a 37 °C 5% CO₂ incubator for co-incubation for approximately 30 min, and then carefully transferred to the H_IL10 Reporter 293 Cell Line cells that is subjected to the adhesion treatment.

The DMEM+10% FBS culture medium was additionally added to cause the final volume of all wells to be 100 µL; the blank wells were sealed with 100 µL of sterile water, and the 96-well plate was continuously incubated for 16 h in the 37 °C incubator; a Bright-Lumi^{TM} firefly luciferase detection reagent was thawed in advance and equilibrated to room temperature; the cell culture plate was taken out and equilibrated to room temperature for 10 min; 100 µL of the Bright-Lumi^{TM} firefly luciferase detection reagent was added to each well, and incubation was performed for 5 min at room temperature; after the reaction system was completed, 100 µL was transferred from each well to a 96-well plate; chemiluminescent detection was performed in a multimode reader; and a curve was plotted by using GraphPad Prism 8.3.0, and an EC₅₀ value was determined by using a four-parameter method.

**Table 13 Summary of reporter gene results for fusion protein IL10 terminus**

| EC₅₀ (nM) | Non-enriched | Enriched |
|---|---|---|
| R1153 | 9.237 | 1.811 |
| R1187 | 4.542 | 4.918 |
| R3361 | 2.805 | 2.917 |
| R0862 | NA | NA |

Under the conditions of this experiment, as shown in Fig. 14 and Table 13, the isotype control molecule R0862 had no activation activity under non-enriched or enriched conditions, indicating the reliability of the experimental system. Under the non-enriched and enriched conditions, the fluorescence reporter activity plateau values of the R1153 and R1187 were significantly lower than those of a wild-type IL10 control R3361. The EC50 values for R1187 were comparable under the non-enriched and enriched conditions, the EC50 values for R3361 were also comparable under the non-enriched and enriched conditions, and the EC50 value for the antibody R1153 to be tested under the enriched condition was significantly lower than that under the non-enriched condition, indicating an increase in the reporter gene activity of the R1153 after enrichment.

### Example 12 Detection of binding and blocking activity of PD-1 terminus of R1153 by FACS

Cell resuscitation: a CHO/human PD-1 cell cryopreservation tube was taken out from a liquid nitrogen tank, rapidly transferred to a 37 °C water bath for thawing, then gently agitated until only a small amount of ice crystals remained, and then transferred to a biosafety cabinet. 1 mL of cell suspension was pipetted into a centrifuge tube containing 10 mL of a fresh CD-CHO culture medium, and gently mixed through blowing, centrifugation was performed for 5 min at 300 g, supernatant was discarded, resuspending was performed with 10 mL of the fresh CD-CHO culture medium, and culture was performed for 2 days in a 37 °C 5% CO₂ incubator. Cell counting: cell counting was performed on the CHO/human PD-1 cell to be tested, and the cells were centrifuged for 5 min at 300×g, resuspended with a FACS buffer, and adjusted to a density of 4E+06 cells/mL. Each cell line was added to a 96-well V-shaped plate with 50 µL/well, achieving the number of the cells in each well to be 2E+05. The diluted sample was added to a 96-well V-shaped plate with 50 µL/well, and incubated on ice in the dark for 30 min. Binding experiment: the 96-well V-shaped plate was centrifuged at 300×g for 5 min, the supernatant was discarded, 200 µL of a FACS buffer was added for one wash, centrifugation was performed for 5 min at 300×g, and the supernatant was discarded; a diluted secondary antibody PE anti-human IgG Fc (1:500) was added to the 96-well V-shaped plate with 100 µL/well, and incubated on ice in the dark for 30 min. Blocking experiment: 1 µg/mL of a ligand human PD-L1-mFC protein was added at 50 µL/well, well mixed, and incubated on ice in the dark for 30 min; the 96-well V-shaped plate was centrifuged at 300×g for 5 min, the supernatant was discarded, 200 µL of the FACS buffer was added for one wash, centrifugation was performed for 5 min at 300×g, and the supernatant was discarded; the diluted secondary antibody PE anti-mouse IgG Fc (1:500) was added to the 96-well V-shaped plate with 100 µL/well, and incubated on ice in the dark for 30 min; the 96-well V-shaped plate was centrifuged at 300×g for 5 min, the supernatant was discarded, 200 µL of the FCM buffer was added for one wash, centrifugation was performed for 5 min at 300×g, and the supernatant was discarded; 100 µL 1×PBS was added for resuspending, and the 96-well V-shaped plate was placed in a flow cytometer for detection; and a curve was plotted by using GraphPad Prism 8.3.0, and an EC₅₀ value was determined by using a four-parameter method.

**Table 14 Summary of binding and blocking activity results for fusion protein PD-1 terminus**

| Sample | EC₅₀ (nM) | IC₅₀ (nM) |
|---|---|---|
| R1153 | 7.299 | 8.621 |
| R1152 | 7.751 | 8.344 |
| R0862 | Not binding | Not blocking |

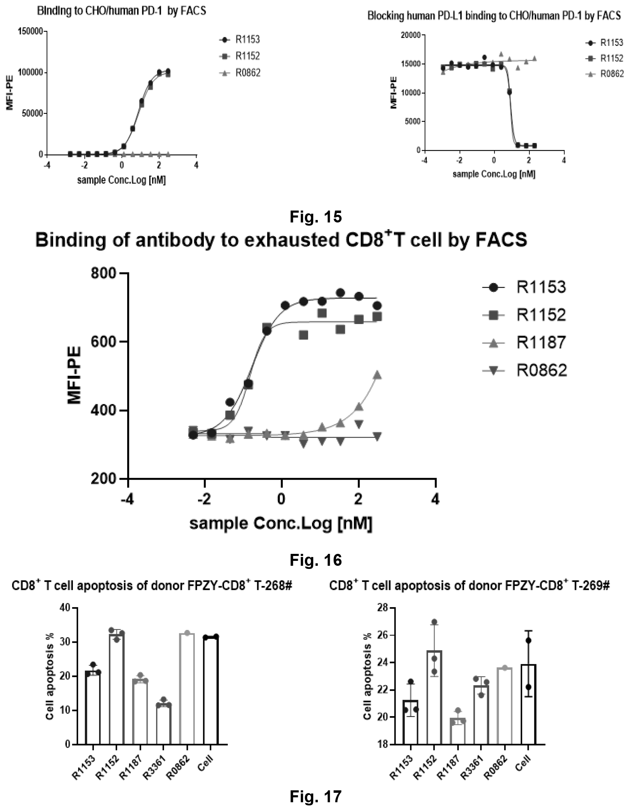

As shown in Fig. 15 and Table 14, the isotype control R0862 did not bind to the CHO/human PD-1 cells, and did not block the binding of the human PD-L1-mFC protein to the CHO/human PD-1 cells. The R1153 had strong binding activity to CHO/human PD-1 (EC50 = 7.299 nM), with binding capacity comparable to that of a reference antibody R1152 (EC50 = 7.751 nM). The R1153 had potent blocking activity against human PD-L1-mFC protein binding to CHO/human PD-1 cells (IC50 = 8.621 nM), with blocking capacity comparable to that of the reference antibody R1152 (IC50= 8.344 nM).

### Example 13 Detection of binding activity of R1153 to in vitro human exhausted CD8+T cells by FACS

PBMC cell separation: 100 mL of fresh human peripheral blood was placed in a biosafety cabinet, 15 mL of the human peripheral blood was added to each 50 mL centrifuge tube, then 15 mL of DPBS was added to each tube to reach a 1:1 dilution, and well mixing was performed; new 50 mL centrifuge tubes were prepared and each added with 15 mL of Lymphoprep^{TM} lymphocyte separation liquid, the diluted peripheral blood was slowly added to an upper layer of the separation liquid; centrifugation was performed for 30 min at 400×g at 4 °C, with an acceleration/deceleration rate set to 1/0, respectively; after centrifugation, a mononuclear cell layer was carefully pipetted to a clean 50 mL centrifuge tube, PBS was added to reach a volume of 45 mL, and centrifugation was performed for 10 min at 300×g at 4 °C; after the supernatant was discarded, 4 mL of ACK Lysing Buffer (human red blood cell lysate) was added to each tube, resuspended, and then allowed to stand for 10 min at room temperature; and additional PBS was added to reach a volume of 45 mL, cell counting was performed after well mixing, and centrifugation was performed for 5 min at 300×g simultaneously.

Separation and activation of CD8+T cell: the CD8+T cells were separated from fresh human PBMC according to the "Human CD8 Microbeads Kit Operating Manual". The density of the separated CD8+T cells was adjusted to 1E6 cells/mL by using a RPMI 1640+10 % FBS+100 U/mL IL-2 culture medium, the cells were inoculated into a 24-well plate with 1 mL per well, 5 µL of CD3/CD28 magnetic beads with the density of 2E+08 /mL was simultaneously added in a 1:1 ratio, and after well mixing, the plate was placed in a 37 °C 5% CO₂ incubator for continuous stimulation for 3 days.

Cell counting was performed on the induced CD8+T cells, the cells were centrifuged for 5 min at 300×g, resuspended with a FACS buffer, adjusted to a density of 4E+06 cells/mL, and added to a 96-well V-shaped plate with 50 µL/well, causing the number of the cells in each well to be 2E+05. The diluted sample was added to a 96-well V-shaped plate with 50 µL/well, and incubated on ice in the dark for 30 min; the 96-well V-shaped plate was centrifuged at 300×g for 5 min, the supernatant was discarded, 200 µL of the FACS buffer was added for one wash, centrifugation was performed for 5 min at 300×g, and the supernatant was discarded; a diluted secondary antibody (1:500) was added to the 96-well V-shaped plate with 100 µL/well, and incubated on ice in the dark for 30 min; the 96-well V-shaped plate was centrifuged at 300×g for 5 min, the supernatant was discarded, 200 µL of the FACS buffer was added for one wash, centrifugation was performed for 5 min at 300×g, and the supernatant was discarded; 100 µL 1×PBS was added for resuspending, and the 96-well V-shaped plate was placed in a flow cytometer for detection; and a curve was plotted by using GraphPad Prism 8.3.0, and an EC₅₀ value was determined by using a four-parameter method.

**Table 15 Summary of binding activity results for exhausted CD8+T cell induced by fusion protein**

| Sample | Binding EC₅₀ for exhausted CD8⁺T cell (nM) |
|---|---|
| R1153 | 0.1680 |
| R1152 | 0.1475 |
| R1187 | Weak binding |
| R0862 | Not binding |

Under the conditions of this experiment, the results were shown in Fig. 16 and Table 15, the isotype control ISO-hIgG1.8 did not bind to the induced exhausted CD8+T cells. The R1153 had good binding activity to the PD-1 parent antibody and the exhausted CD8+T cells (Values of 0.1680 nM and 0.1475 nM, respectively). The R1187 (i.e., ISO-hIgG1.8-IL10) weakly bound to the exhausted CD8+T cells.

### Example 14 Activation of exhausted CD8+T cell function and inhibition of apoptosis by R1153

PBMC cell separation: 100 mL of fresh human peripheral blood was placed in a biosafety cabinet, 15 mL of the human peripheral blood was added to each 50 mL centrifuge tube, then 15 mL of DPBS was added to each tube to reach a 1:1 dilution, and well mixing was performed; new 50 mL centrifuge tubes were prepared and each added with 15 mL of Lymphoprep^{TM} lymphocyte separation liquid, the diluted peripheral blood was slowly added to an upper layer of the separation liquid; centrifugation was performed for 30 min at 400×g at 4 °C, with an acceleration/deceleration rate set to 1/0, respectively; after centrifugation, a mononuclear cell layer was carefully pipetted to a clean 50 mL centrifuge tube, PBS was added to reach a volume of 45 mL, and centrifugation was performed for 10 min at 300×g at 4 °C; after the supernatant was discarded, 4 mL of ACK Lysing Buffer (human red blood cell lysate) was added to each tube, resuspended, and then allowed to stand for 10 min at room temperature; and additional PBS was added to reach a volume of 45 mL, cell counting was performed after well mixing, and centrifugation was performed for 5 min at 300×g simultaneously.

Separation and activation of CD8+T cell: the CD8+T cells were separated from fresh human PBMC according to the "Human CD8 Microbeads Kit Operating Manual". The density of the separated CD8+T cells was adjusted to 1E6 cells/mL by using a RPMI 1640+10 % FBS+100 U/mL IL-2 culture medium, the cells were inoculated into a 24-well plate with 1 mL per well, 5 µL of CD3/CD28 magnetic beads with the density of 2E+08 /mL was simultaneously added in a 1:1 ratio, and after well mixing, the plate was placed in a 37 °C 5% CO₂ incubator for continuous stimulation for 3 days.

Pre-coated plate: 5 µg/mL of a diluted R2294 protein was added to a 96-well flat-bottom plate based on a volume of 200 µL/well, and placed for 16 h at a 2 °C-8 °C fridge; an R2294-precoated plate was washed for three times with 1×PBS, and then the diluted sample was added to the pre-coated plate based on a volume of 100 µL/well and incubated for 60 min at 37 °C; the induced exhausted CD8+T cells were counted, centrifuged for 5 min at 300×g, resuspended by using an RPMI 1640+10 % FBS culture medium, and adjusted to a density of 2E+06 cells/mL, with total 3 mL; and 30 µL of CD3/CD28 magnetic beads with the density of 2E+08/mL was added in a 1:1 quantitative ratio and well mixed, then the mixture was added to a 96-well flat-bottom plate for sample incubation based on the volume of 100 µL/well, well mixed, and incubated for 4 days in a 37 °C 5% CO₂ incubator. Cytokine detection: cell supernatant after 4 days was collected and diluted 3 folds, then an IFN-γ secretion level was detected by using a Human IFN-γ uncoated ELISA kit according to a method in an instruction, and a diagram was plotted with GraphPad Prism 8.3.0. Apoptosis detection: the cells in the 96-well flat-bottom plate were transferred to a 96-well V-shaped plate and centrifuged for 5 min at 300×g, the supernatant was discarded, then washing was performed once with an Annexin-V binding buffer of 200 µL/well, a staining solution diluted by the Annexin-V binding buffer (1:100) was added to the 96-well V-shaped plate based on 100 µL/well, the staining solution contained APC annexin-V and Propidium Iodide Solution reagents and incubated for 15 min at room temperature in the dark; after incubation, 100 µL of the Annexin-V binding buffer was additionally added to each well and well mixed, and the 96-well V-shaped plate was placed in a flow cytometer for detection; and a curve was plotted by using GraphPad Prism 8.3.0.

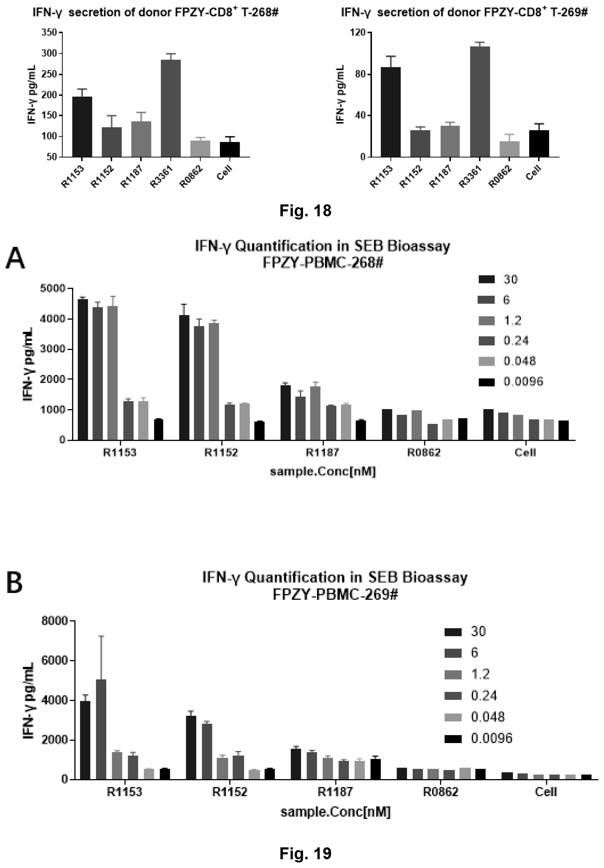

Under the conditions of this experiment, the results shown in Figs. 17 and 18, both the R1153 and R3361 inhibited apoptosis in the exhausted CD8+T cells and activate the function of the exhausted CD8+T cells. However, the isotype control R0862 and the PD-1 parent antibody R1152 had no effect on the function of exhausted CD8+T cells.

### Example 15 Study on the activity of R1153 in SEB experiments

Staphylococcal enterotoxin B (SEB) was a typical T-cell superantigen, which might trigger T-cell polyclonal activation. In order to investigate a T-cell activation function of R1153, this study used SEB superantigen to activate T cells and evaluated the effect of the R1153 on IFN-γ secretion.

PBMC cell separation and counting: 100 mL of fresh human peripheral blood was placed in a biosafety cabinet, 15 mL of the human peripheral blood was added to each 50 mL centrifuge tube, then 15 mL of DPBS was added to each tube to reach a 1:1 dilution, and well mixing was performed; new 50 mL centrifuge tubes were prepared and each added with 15 mL of Lymphoprep^{TM} lymphocyte separation liquid, the diluted peripheral blood was slowly added to an upper layer of the separation liquid; centrifugation was performed for 30 min at 400×g at 4 °C, with an acceleration/deceleration rate set to 1/0, respectively; after centrifugation, a mononuclear cell layer was carefully pipetted to a clean 50 mL centrifuge tube, DPBS was added to reach a volume of 45 mL, and centrifugation was performed for 10 min at 300×g at 4 °C; after the supernatant was discarded, 4 mL of ACK Lysing Buffer (human red blood cell lysate) was added to each tube; and the DPBS was additionally added to reach a volume of 45 mL, cell counting was performed after well mixing, and centrifugation was performed for 5 min at 300×g simultaneously.

An appropriate amount of PBMC cells was taken, centrifuged, then adjusted to a density of 1E6 cells/mL by using a complete culture medium (10 % FBS+X-VIVO), and added to a 96-well U-shaped plate with 100 µL per well; an antibody sample diluted by the 10% FBS+X-VIVO culture medium and 100 ng/mL SEB were added to the 96-well U-shaped plate with 50 µL per well; the 96-well plate was placed in a 5% CO₂ incubator and cultured for 3 days at 37 °C; 50 µL of the supernatant was pipetted from each well to a new 96-well plate and centrifuged, the supernatant was pipetted and diluted (8-fold dilution), and an IFN-γ secretion level was detected according to the Human IFN-γ uncoated ELISA kit instructions; and a bar graph was plotted by using GraphPad Prism 8.3.0.

Under the conditions of this experiment, as shown in Fig. 19, both the R1153 and R1152 could significantly increase IFN-γ secretion in different donors, indicating that they might promote T-cell activation and had activation abilities. The control antibody R1187 had a weak promotion effect on IFN-γ secretion. The isotype control molecule R0862 had no effect on T-cell activation.

### Example 16 Study on activity of R1153 in Mixed Lymphocyte Reaction (MLR) experiment

An MLR was commonly used for tissue typing before organ transplantation, so as to determine the degree of compatibility between major histocompatibility Human Leukocyte Antigens (HLA) of a receptor and a donor. When DC HLAs from different donors did not match T-cell TCR, T cells might be activated by these heterologous DCs, leading to cell division and the production of a wide variety of cytokines. In the field of drug development, it might be used to evaluate the effects of immunomodulatory drugs on the function of human primary T cells, thereby assessing the potential immunostimulatory or immunosuppressive effects of the drugs.

PBMC cell separation and counting: 100 mL of fresh human peripheral blood was placed in a biosafety cabinet, 15 mL of the human peripheral blood was added to each 50 mL centrifuge tube, then 15 mL of DPBS was added to each tube to reach a 1:1 dilution, and well mixing was performed; new 50 mL centrifuge tubes were prepared and each added with 15 mL of Lymphoprep^{TM} lymphocyte separation liquid, the diluted peripheral blood was slowly added to an upper layer of the separation liquid; centrifugation was performed for 30 min at 400×g at 4 °C, with an acceleration/deceleration rate set to 1/0, respectively; after centrifugation, a mononuclear cell layer was carefully pipetted to a clean 50 mL centrifuge tube, DPBS was added to reach a volume of 45 mL, and centrifugation was performed for 10 min at 300×g at 4 °C; after the supernatant was discarded, 4 mL of ACK Lysing Buffer (human red blood cell lysate) was added to each tube; and the DPBS was additionally added to reach a volume of 45 mL, cell counting was performed after well mixing, and centrifugation was performed for 5 min at 300×g simultaneously.

According to the operating instructions, MACS CD14 microbeads were used to positively select monocytes, the EasySep^{TM} Human T Cell Enrichment Kit was used to negatively select CD3 T cells from the remaining cells after positive selection, and the CD3T cells were frozen in liquid nitrogen for later use. The sorted monocytes were respectively inoculated in a T75 culture bottle based on the density of 5E+05/mL, with total 20 mL; GM-CSF and IL-4 with a final concentration of 50 ng/mL were additionally added to the inculated monocyte culture medium X-VIVO+10% FBS, and incubated for 3 days in a 37 °C 5% CO₂ incubator; after 3 days, half of the medium was replaced, 20 mL of cell suspension in the T75 culture bottle was pipetted into a 50 mL centrifuge tube and centrifuged for 5 min at 400×g, 10 mL of the supernatant was removed, then 10 mL of a fresh X-VIVO+10 % FBS culture medium was added and well mixed, and then the mixture was added to the T75 culture bottle.

The X-VIVO+10 % FBS culture medium containing the GM-CSF and IL-4 with the final concentration of 50 ng/mL was additionally added and continuously cultured for 3 days in the 37 °C 5% CO₂ incubator; LPS with a final concentration of 1 µg/mL was additionally added and continuously cultured for 24 hours in the 37 °C 5% CO₂ incubator; gentle pipetting was performed to resuspend adherent cells; the induced DCs were pipetted into a 50 mL centrifuge tube and centrifuged for 5 min at 400×g, the supernatant was discarded, the centrifuge tube was washed twice with 10 mL of fresh X-VIVO+10 % FBS culture medium, and finally, DC density was adjusted to 2E+05/mL by using the X-VIVO+10% FBS culture medium. The CD3 T cells of different donors were resuscitated, and the density of the CD3 T cells was adjusted to 2E+6/mL by using the X-VIVO+10% FBS culture medium.

MLR experiment: in a U-shaped 96-well plate system, 100 µL of 2E+5/mL DCs was added; and 50 µL of 2E+06/mL CD3T cells was added. A ratio of CD3 T:DC was 5:1; the DCs and the CD3 T cells were combined into FPZY-DC-195# (DCs separated from No.195 donor blood)+FPZY-CD3T-270# (T cells separated from No.270 donor blood), and FPZY-DC-270# (DCs separated from the No.270 donor blood)+FPZY-CD3T-195# (T cells separated from the No.195 donor blood); antibodies were added to the cells with 50 µL per well in a 96-well plate, well mixed, and incubated for 5 days in the 37 °C 5% CO₂ incubator; and on Day 3 and Day 5 after incubation, 50 µL of the supernatant was pipetted for cytokine detection. IL-2 detection: the D3 supernatant was pipetted and subjected to a 5-fold dilution; an IL-2 secretion level in the diluted supernatant was detected according to the Human IL-2 Uncoated ELISA kit instructions. IFN-γ detection: the D5 supernatant was pipetted and subjected to an 8-fold dilution, and an IFN-γ secretion level in the diluted supernatant was detected according to the Human IFN-γ uncoated ELISA kit instructions; and a bar graph was plotted by using GraphPad Prism 8.3.0.

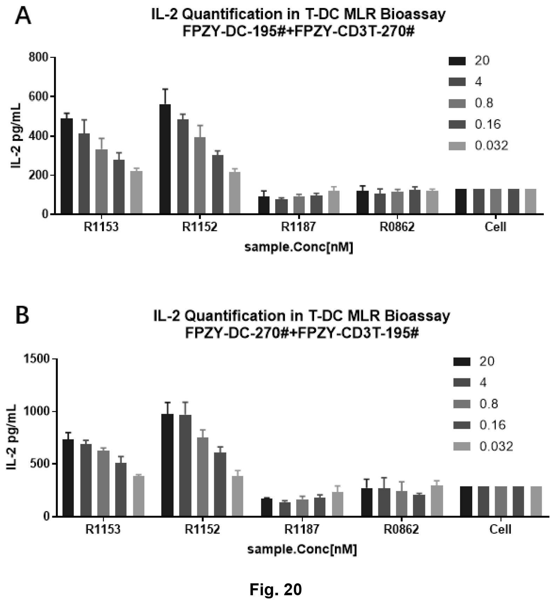

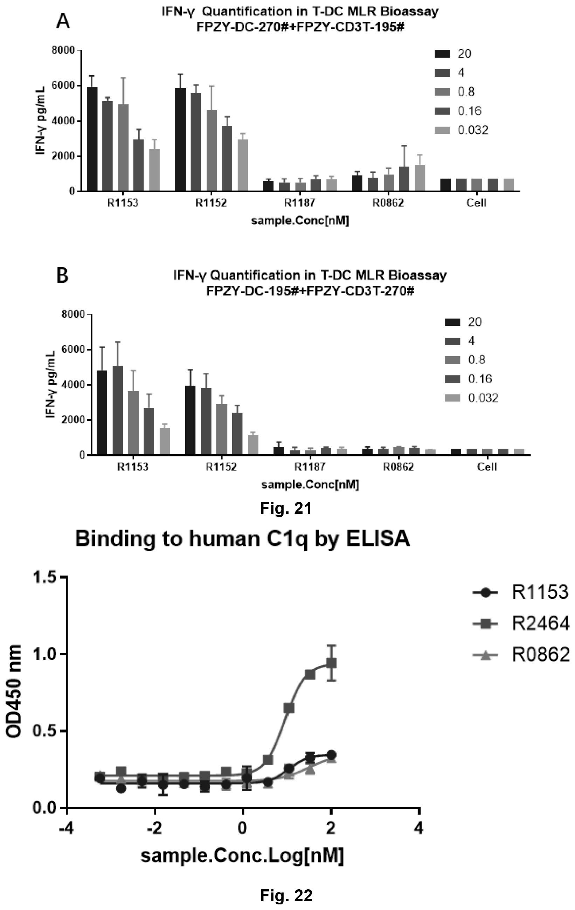

Under the conditions of this experiment, the results were shown in Figs. 20 and 21. For different donors, the results showed consistent trends. Both the R1153 and the corresponding PD-1 parent antibody R1152 significantly enhance IL-2 and IFN-γ secretion, indicating they might promote T-cell activation and had comparable activation abilities. However, the isotype control molecule R0862 and the control antibody R1187 had no effect on T-cell activation.

### Example 17 Detection of binding activity of R1153 and complement C1q protein by ELISA

The antibody molecules, gradient-diluted by a 50 mM CB buffer, were added to a 96-well ELISA plate at 100 µL/well, and the plate was sealed with a plate-sealing membrane and allowed to stand for 18 h at 2 °C-8 °C; and the plate was washed for three times with 200 µL of 1×PBST, and then dried by patting. A blocking solution (1% BSA) was added based on 200 µL/well and blocked at room temperature for 1.5 hours; the blocking solution was removed, the plate was washed twice with 200 µL of 1×PBST and dried by patting; 2 µg/mL of a Native human C1q protein was added based on 100 µL/well and incubated at room temperature for 1.5 hours, and the plate was washed for seven times with 200 µL of 1×PBST and dried by patting; 50 ng/mL of a Biotin Rabbit polyclonal to C1q secondary antibody was added based on 100 µL/well and incubated at room temperature for 1 hour, and the plate was washed for seven times with 200 µL of 1×PBST and dried by patting; SA-HRP diluted by 1×BSA (1:20, 000) was added based on 100 µL/well and incubated at room temperature for 40 minutes; and the plate was washed for seven times with 200 µL of 1×PBST and dried by patting. First, 50 µL of a color development solution A was added, followed by 50 µL of a color development solution B; the plate was sealed by using the plate-sealing membrane and incubated at 37 °C for 10 minutes; and a stop solution was added based on 100 µL/well to terminate the reaction. The absorbance was determined at 450 nm by immediately using a multimode reader; and a curve was plotted by using GraphPad Prism 8.3.0.

Under the conditions of this experiment, the results were shown in Fig. 22, Both the R1153 and the isotype control molecule R0862 had weak binding activity to the C1q protein, and had comparable binding activity. The positive control molecule hPD-1-hIgG1-IL10M had strong binding activity to the C1q protein.

### Example 18 Detection of ADCC activity of R1153 by luciferase reporter gene method

Cell resuscitation: CHO/human PD-1 cell and Jurkat-NFAT-luc-hCD16 cell cryopreservation tubes were taken out from a liquid nitrogen tank, rapidly transferred to a 37 °C water bath for thawing, then gently agitated until only a small amount of ice crystals remained, and then transferred to a biosafety cabinet. 1 mL of a CHO/human PD-1 cell cryopreservation solution was pipetted and added to a centrifuge tube containing 5 mL of a CD-CHO fresh culture medium. 1 mL of a Jurkat-NFAT-luc-hCD16 cell cryopreservation solution was pipetted and added to a centrifuge tube containing 5mL of a RPMI 1640+10% FBS fresh culture medium. Well mixing was performed, then centrifugation was performed for 5 min at 300×g, the supernatant was discarded, resuspending was performed by using 10 mL of a fresh culture medium, and finally, culture was performed for 2 days in a 37 °C 5% CO₂ incubator.

Cell preparation: the cells were counted, an appropriate amount of CHO/human PD-1 and Jurkat-NFAT-luc-hCD16 cells was taken, centrifugation was performed for 5 min at 300×g, the supernatant was discarded, washing was performed once by using the RPMI1640+10% FBS culture medium, and counting was performed after resuspending was performed by using the RPMI 1640+10% FBS culture medium. The density of the CHO/human PD-1 cells was adjusted to 2E+05 cells/mL and the density of the Jurkat-NFAT-luc-hCD16 cells was adjusted to 1E+06 cells/mL.

100 µL of the diluted antibody diluent to be tested was added to each well of a white 96-well plate. The CHO/human PD-1 cells were added based on 1E+04 cells/well, that is, 50 µL/well, and the Jurkat-NFAT-luc-hCD16 cells were added based on 5E+04 cells/well, that is, 50 µL/well, sequentially. A specific volume of the culture medium was added to the corresponding wells to cause a total volume of 200 µL in all wells, and 200 µL of PBS was added to the blank wells; the cell culture plate was placed in a 37 °C 5% CO₂ incubator and incubated for 16 h; a Bright-LumiTM firefly luciferase detection reagent was thawed in advance and equilibrated to room temperature; after the cells were cultured to a time point, the cell culture plate was taken out and equilibrated to room temperature for 10 min, and after gently mixing, 100 µL was pipetted from each well into a new white 96-well plate; 100 µL of the Bright-Lumi^{TM} firefly luciferase detection reagent was added to each well, and incubation was performed for 5 min at room temperature; chemiluminescence detection was performed in a multimode reader; and a curve was plotted by using GraphPad Prism 8.3.0, and an EC₅₀ value was determined by using a four-parameter method.

**Table 16 Summary of ADCC Activity of Fusion Protein Determined by Fluorescence Reporter Assay**

| Test sample name | EC₅₀ (nM) |
|---|---|
| R1153 | NA |
| R2464 | 0.2572 |
| R0862 | NA |

R2464: anti-hPD1-hIgG1-IL10M, a heavy chain amino acid sequence was as set forth in SEQ ID NO:42, and a light chain amino acid sequence was as set forth in SEQ ID NO:43.

QVQLVQSGAEVKKPGASVKVSCKASGYTFTTYYMYWVRQAPGQGLEWMGGINPSNGGTNY
AQKFQGRATMTVDTSTSTAYMELSSLRSEDTAVYYCTRRDSNYDGGFDYWGQGTTVTVSSASTK
GPSVFPLAPSSKSTSGGTAALGCLVKDYFPEPVTVSWNSGALTSGVHTFPAVLQSSGLYSLSSVVT
VPSSSLGTQTYICNVNHKPSNTKVDKRVEPKSCDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQKSLSLSPGK
(SEQ ID NO:42)

DIQMTQSPSSLSASVGDRVTITCRASKSVSTSGYSYMHWYQQKPGKAPKLLIYLAYHLESGV
PSRFSGSGSGTDFTLTISSLQPEDFATYYCQHSWELPITFGQGTKLEIKRTVAAPSVFIFPPSDEQL
KSGTASVVCLLNNFYPREAKVQWKVDNALQSGNSQESVTEQDSKDSTYSLSSTLTLSKADYEKHK
VYACEVTHQGLSSPVTKSFNRGECGGGGSGGGGSGGGGSGGGGSGGGGGSGGGGSGGGGS
GGGGSGGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENGGGSGGKS
KAVEQVKNAFNKLQEKGIYKAMSEFDIFINYIEAYMTMKIRN (SEQ ID NO:43)

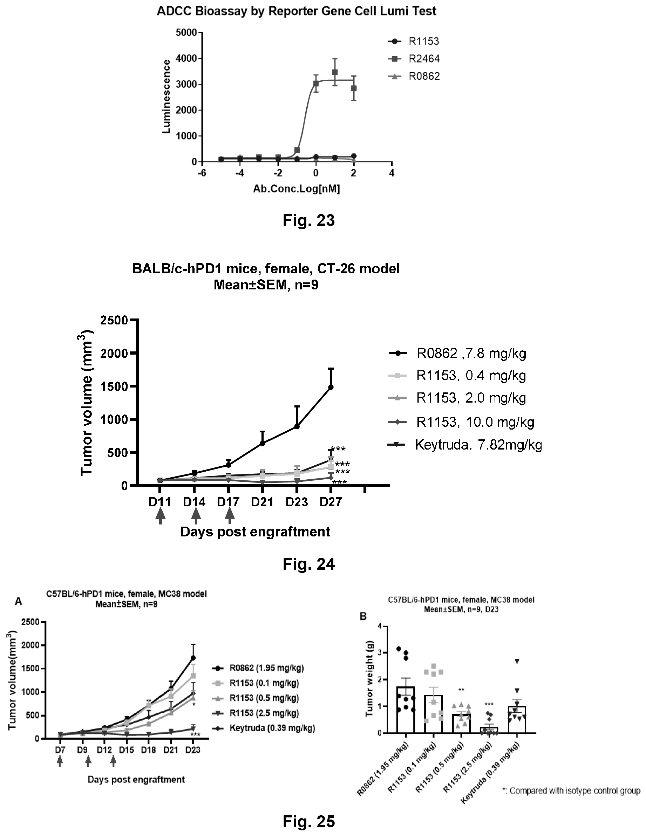

Under the conditions of this experiment, as shown in Fig. 23 and Table 16, the control molecule R2464 had good reporter gene activity, indicating the presence of ADCC activity. The antibody R1153 to be tested and the isotype control R0862 both had no luciferase activity, indicating the absence of ADCC activity.

### Example 19 In-vivo drug efficacy study on anti-PD1-IL10 fusion protein R1153 in CT-26 model

Objective: the anti-tumor activity of the PD1-IL10 fusion protein to CT-26 mouse colon cancer was observed.

Experimental materials: hPD1 Knock-in mice, female, 6-8 weeks old (Balb/c background, Source: Jiangsu GemPharmatech Co., Ltd., Animal Quality Certificate No. B202403040123); CT-26 cells (Shanghai Cell Bank, catalog No.: TCM37); RPMI 1640 culture medium (Gibco, 11875085), FBS (Gibco, 10091-148), 0.25% trypsin-EDTA (Gibco, 25200056), Penicillin-Streptomycin (Gibco, 15140122), DMSO (Sigma, D2650), and DPBS (Hyclone, SH30028.02).

Keytruda: pembrolizumab monoclonal antibody, a heavy chain amino acid sequence was as set forth in SEQ ID NO:36, and a light chain amino acid sequence was as set forth in SEQ ID NO:37.

QVQLVQSGVEVKKPGASVKVSCKASGYTFTNYYMYWVRQAPGQGLEWMGGINPSNGGTN
FNEKFKNRVTLTTDSSTTTAYMELKSLQFDDTAVYYCARRDYRFDMGFDYWGQGTTVTVSSASTK
GPSVFPLAPCSRSTSESTAALGCLVKDYFPEPVTVSWNSGALTSGVHTFPAVLQSSGLYSLSSVVT
VPSSSLGTKTYTCNVDHKPSNTKVDKRVESKYGPPCPPCPAPEFLGGPSVFLFPPKPKDTLMISRT
PEVTCVVVDVSQEDPEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSLSLSLGK
(SEQ ID NO:36)

EIVLTQSPATLSLSPGERATLSCRASKGVSTSGYSYLHWYQQKPGQAPRLLIYLASYLESGVP
ARFSGSGSGTDFTLTISSLEPEDFAVYYCQHSRDLPLTFGGGTKVEIKRTVAAPSVFIFPPSDEQLK
SGTASVVCLLNNFYPREAKVQWKVDNALQSGNSQESVTEQDSKDSTYSLSSTLTLSKADYEKHKV
YACEVTHQGLSSPVTKSFNRGEC (SEQ ID NO:37)

Instruments and devices: Electronic balance (Shanghai Sunny Hengping Instrument, JA12002), vernier caliper (Shanghai Meinaite Industrial Co., Ltd., MNT-150T), microscope (Chongqing Opte Instrument Co., Ltd., BDS200), medical centrifuge (Hunan CENCE Laboratory Development Co., Ltd., L530R), digital constant temperature water bath (PRS Machinery Co., Ltd., HH-S), CO₂ incubator (Panasonic Healthcare Co., Ltd., Japan, MCO-18AC), biosafety cabinet (Guangzhou Rivers Experimental Technology Co., Ltd., ACZ-451), and cell counter (Shanghai Ruiyu Cell counter Co., Ltd., IC1000)

### Test method

Cell culture: the mouse colon cancer cells (CT-26) were cultured in an RPMI 1640 culture medium containing 10% fetal bovine serum (FBS) and 1% penicillin-streptomycin (1:1), and the culture medium was placed in a 37 °C 5% CO₂ incubator for culture.

Inoculation: CT-26 cells at a logarithmic growth phase were collected and washed twice with DPBS, and a cell concentration was regulated to 1X10⁶ cells/mL using DPBS. Female Balb/c-hPD1 mice were taken, and CT-26 cells were inoculated subcutaneously at a volume of 0.1 mL/mouse, equivalent to 1×10⁵/mouse.

Administration: the day of inoculation was recorded as Day 0 (D0). When an average tumor volume reached 60-100 mm³, mice might be randomly grouped based on tumor volume, ensuring that each group had an identical or similar average tumor volume. On Day 11 (D11) of the experiment, the mice were randomly grouped into 5 groups based on the tumor volume, with 9 mice in each group, and then the mice started to be administered (administration regimen shown in Table 17 below).

**Table 17 Administration dose, administration route, and frequency of CT-26 tumor model**

| Group | Dosage (mg/kg) | Administration volume (µL/g) | Administration route | Administration frequency |
|---|---|---|---|---|
| R0862 | 7.8 | 10 | i.p. | Q3D*3 |
| R1153(anti-hPD1-IL10M) | 0.4 | 10 | i.p. | Q3D*3 |
| R1153(anti-hPD1-IL10M) | 2 | 10 | i.p. | Q3D*3 |
| R1153(anti-hPD1-IL10M)) | 10 | 10 | i.p. | Q3D*3 |
| Keytruda | 7.82 | 10 | i.p. | Q3D*3 |

| | | | | |
|---|---|---|---|---|
| Note: Dosing Volume, adjusted according to animal body weight (10µL/g); i.p., intraperitoneal injection; Q3D, administer once every 3 days; if weight loss exceeded 15% during administration, the administration regimen would be adjusted. | | | | |

Recording:
the tumor volume was measured and recorded from D11 onwards; and long and short diameters of the tumor were measured by using a vernier caliper twice per week. The tumor volume was calculated according to a formula: (1/2)×long diameter×(short diameter)² (shown in Table 18 and Fig. 24). When each mouse reached an experimental endpoint (weight loss exceeding 20% or tumor volume exceeding 2000 mm³ to reach a humane endpoint), and the mice were euthanized by CO₂ asphyxiation.

**Table 18 Anti-tumor activity of R1153 fusion protein in CT-26 mouse colon cancer model**

| Days after implantatio n | R0862 | R1153 0.4 mg/kg | R1153 2 mg/kg | R1153 10 mg/kg | Keytruda 7.82 mg/kg |
|---|---|---|---|---|---|
| 11 | 77.99±8.70 | 76.24±6.16 | 77.03±7.08 | 77.89±6.82 | 77.47±7.52 |
| 14 | 186.17±29.51 | 96.15±14.49 | 116.14±23.29 | 87.96±13.99 | 113.97±16.78 |
| 17 | 308.41±77.42 | 107.25±23.1 1 | 117.86±40.73 | 82.77±18.65 | 150.40±21.49 |
| 20 | 639.16±178.3 3 | 144.21±31.3 6 | 155.63±75.69 | 50.63±17.16 | 172.99±36.16 |
| 23 | 892.32±302.3 8 | 174.20±41.1 4 | 193.54±101.36 | 63.63±30.21 | 189.36±51.04 |
| 27 | 1269.49±203. 86 | 282.72±85.7 2 | 275.37±158.83 | 118.63±72.96 | 389.36±144.96 |

Data computation, statistics, and analysis:
Independent sample T-tests might be used to compare two groups. For comparisons involving three or more groups, One-Way ANOVA was used. If the F-value indicated a significant difference, post-hoc analyses among a plurality of groups might be performed. Data were processed by using Prism GraphPad, and when p<0.05, it indicated a statistically significant difference. Tumor volume TV = 0.5×a×b², where a and b represented the long and short diameters of the tumor, respectively. Tumor Growth Inhibition (TGI) (%) = [1-(Ti-T0)/(Vi-V0)]×100, where Ti was an average tumor volume of a treatment group on day i, T0 was an average tumor volume of the treatment group at the start of treatment, Vi was an average tumor volume of a solvent control group on Day i, and V0 was an average tumor volume of the solvent control group at the start of treatment.

Experiment results: the PD1-IL10 fusion protein R1153 showed significant anti-tumor activity, with TGI of 85.33%, 85.91%, and 97.11% in the low-, medium-, and high-dose groups, respectively; among the 9 mice, Complete Remission (CR) was observed in 2, 2, and 5 mice, respectively; and the Keytruda group, administered at an equivalent molar dose to the high-dose R1153 group, achieved the TGI of 77.84%, with 3 out of 9 mice having CR. (shown in Table 18 and Fig. 24).

### Example 20 In-vivo drug efficacy study on anti-PD1-IL10 fusion protein R1153 in MC38 model

Objective: the anti-tumor activity of the PD1-IL10 fusion protein to MC38 mouse colon cancer was observed.

Experimental materials: hPD1 Knock-in mice, female, 6-8 weeks old (C57BL/6 background, Source: Biocytogen (Jiangsu) Gene Cell counter Co., Ltd., Animal Quality Certificate No. 320726240100003000); MC38 cells (National Experimental Cell Resource Sharing Platform, resource No.: 1101MOU-PUMC000523); DMEM culture medium (Gibco, 11965-092), FBS (Gibco, 10091-148), 0.25% trypsin-EDTA (Gibco, 25200056), Penicillin-Streptomycin (Gibco, 15140122), glutamine (Gibco, 35050-061), sodium pyruvate (Gibco, 11360-070), and DPBS (Hyclone, SH30028.02).

Instruments and devices: Electronic balance (Shanghai Sunny Hengping Instrument, JA12002), vernier caliper (Shanghai Meinaite Industrial Co., Ltd., MNT-150T), microscope (Chongqing Opte Instrument Co., Ltd., BDS200), medical centrifuge (Hunan CENCE Laboratory Development Co., Ltd., L530R), digital constant temperature water bath (PRS Machinery Co., Ltd., HH-S), CO₂ incubator (Panasonic Healthcare Co., Ltd., Japan, MCO-18AC), biosafety cabinet (Guangzhou Rivers Experimental Technology Co., Ltd., ACZ-451), and cell counter (Shanghai Ruiyu Cell counter Co., Ltd., IC1000)

### Test method

Cell culture: the mouse colon cancer cells (MC38) were cultured in a DMEM culture medium containing 10% fetal bovine serum (FBS), 1% penicillin-streptomycin (1:1), 1% glutamine, and 1% sodium pyruvate, and the culture medium was placed in a 37 °C 5% CO₂ incubator for culture.

Inoculation: MC38 cells at a logarithmic growth phase were collected and washed twice with DPBS, and a cell concentration was regulated to 3X10⁶ cells/mL using DPBS. Female C57BL/6-hPD1 mice were used, and MC38 cells were inoculated subcutaneously at a volume of 0.1 mL/mouse, equivalent to 3X10⁵/mouse.

Administration: the day of inoculation was recorded as Day 0 (D0). When an average tumor volume reached 80-100 mm³, mice were randomly grouped based on tumor volume, ensuring that each group had an identical or similar average tumor volume. On Day 7 (D7) of the experiment, the mice were randomly grouped into 5 groups based on the tumor volume, with 9 mice in each group, and then the mice started to be administered (administration regimen shown in Table 19 below).

**Table 19 Administration dose, administration route, and frequency of MC38 tumor model**

| Group | Dosage (mg/kg) | Administration volume (µL/g) | Administration route | Administration frequency |
|---|---|---|---|---|
| R0862 | 1.95 | 10 | i.p. | Q3D*3 |
| R1153(anti-hPD1-IL10M) | 0.1 | 10 | i.p. | Q3D*3 |
| R1153(anti-hPD1-IL10M) | 0.5 | 10 | i.p. | Q3D*3 |
| R1153(anti-hPD1-IL10M)) | 2.5 | 10 | i.p. | Q3D*3 |
| Keytruda | 0.39 | 10 | i.p. | Q3D*3 |

| | | | | |
|---|---|---|---|---|
| Note: Dosing Volume, adjusted according to animal body weight (10µL/g); i.p., intraperitoneal injection; Q3D, administer once every 3 days; if weight loss exceeded 15% during administration, the administration regimen would be adjusted. | | | | |

Recording:
the tumor volume was measured and recorded from D7 onwards; and long and short diameters of the tumor were measured by using a vernier caliper twice per week. The tumor volume was calculated according to a formula: (1/2)×long diameter×(short diameter)² (shown in Table 20 and Fig. 25). When each mouse reached an experimental endpoint (weight loss exceeding 20% or tumor volume exceeding 2000 mm³ to reach a humane endpoint), and the mice were euthanized by CO₂ asphyxiation.

**Table 20 Anti-tumor activity of R1153 fusion protein in MC38 mouse colon cancer model**

| Days after | R0862 | R1153 | R1153 | R1153 | Keytruda |
|---|---|---|---|---|---|
| implantatio n | | 0.1 mg/kg | 0.5 mg/kg | 2.5 mg/kg | 0.39 mg/kg |
| 7 | 91.27±9.79 | 92.04±7.25 | 91.86±7.65 | 92.83±7.45 | 93.63±8.00 |
| 9 | 135.46±14.67 | 120.24±19.72 | 119.88±16.54 | 127.37±17.0 2 | 158.77±19.09 |
| 12 | 238.93±22.48 | 183.72±30.46 | 136.90±14.47 | 117.13±28.3 8 | 225.39±43.61 |
| 15 | 415.80±64.93 | 343.53±59.51 | 187.14±28.98 | 87.62±43.05 | 303.92±78.07 |
| 18 | 720.06±98.64 | 713.99±127.0 1 | 323.16±33.88 | 95.44±45.30 | 466.29±143.0 8 |
| 21 | 1078.95±155.7 0 | 910.63±157.5 8 | 558.34±106.0 2 | 139.88±61.9 4 | 643.64±157.4 9 |

Data computation, statistics, and analysis:
Independent sample T-tests might be used to compare two groups. For comparisons involving three or more groups, One-Way ANOVA was used. If the F-value indicated a significant difference, post-hoc analyses among a plurality of groups might be performed. Data were processed by using Prism GraphPad, and when p<0.05, it indicated a statistically significant difference. Tumor volume V = 0.5×a×b², where a and b represented the long and short diameters of the tumor, respectively. Tumor Growth Inhibition (TGI) (%) = [1-(Ti-T0)/(Vi-V0)]×100, where Ti was an average tumor volume of a treatment group on day i, T0 was an average tumor volume of the treatment group at the start of treatment, Vi was an average tumor volume of a solvent control group on Day i, and V0 was an average tumor volume of the solvent control group at the start of treatment.

Experiment results: the PD1-IL10 fusion protein R1153 showed a significant anti-tumor effect, with TGI of 23.56%, 52.06%, and 92.66% in the low-, medium-, and high-dose groups, respectively; tumor inhibition rates (IRTW%) were 17.85%, 59.12%, and 86.5%, respectively; among the 9 mice, Complete Remission (CR) was observed in 0, 0, and 4 mice, respectively; and the Keytruda group, administered at an equivalent molar dose to the medium-dose R1153 group, achieved the TGI of 46.37% and the IRTW% of 41.71%, with 0 out of 9 mice having CR. (shown in Table 20 and Fig. 25).

### Example 21 Impact of anti-PD1-IL10 fusion protein on tumor-infiltrating lymphocyte proportion

Objective: the mechanism of action of PD1-IL10 fusion protein in anti-tumor effects was studied.

Experimental materials: Balb/c mice, female, 6-8 weeks old (Source: Guangdong Charles River, Animal Quality Certificate No. 44829700019477); CT-26 cells (Shanghai Cell Bank, catalog No.: TCM37); RPMI 1640 culture medium (Gibco, 11875085), FBS (Gibco, 10091-148), 0.25% trypsin-EDTA (Gibco, 25200056), Penicillin-Streptomycin (Gibco, 15140122), DPBS (Hyclone, SH30028.02), tumor tissue digestive enzymes (Miltenyi Biotec, 130-096-730), Zombie NIR^{TM} Fixable Viability Kit (Biolegend, 423105), Brilliant Violet 510^{TM} anti-mouse CD45 Antibody (Biolegend, 103138) Alexa Fluor^{®} 700 anti-mouse CD3 (Biolegend, 100215), eBioscience^{TM} mouse regulatory T-cell staining kit (Invitrogen, 88-8111-40), PerCP/Cyanine5.5 anti-mouse Cd8a (Biolegend, 100712).

R0991(anti-mPD1-IL10M): having amino acid sequences as set forth in SEQ ID NO:38 (heavy chain) and SEQ ID NO:39 (light chain).

QVQLKESGPGLVQPSQTLSLTCTVSGFSLTSYNVHWVRQPPGKGLEWMGGMRYNEDTSYN

SALKSRLSISRDTSKNQVFLKMNSLQTDDTGTYYCTRDAVYGGYGGWFAYWGQGTLVTVSSAST
KGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPEAAGGPSVFLFPPKPKDTL
MISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYTQKSLSLSP
GK (SEQ ID NO:38)

DTVLTQSPALAVSLGQRVTISCKASETVSSSMYSYIHWYQQKPGQQPKLLIYRASNLESGVPA
RFSGSGSGTDFTLTIDPVEADDVATYFCQQSWNPWTFGGGTKLELKRTVAAPSVFIFPPSDEQLKS
GTASVVCLLNNFYPREAKVQWKVDNALQSGNSQESVTEQDSKDSTYSLSSTLTLSKADYEKHKVY
ACEVTHQGLSSPVTKSFNRGECGGGGSGGGGSGGGGSGGGGSGGQGTQSENSCTHFPGNLP
NMLRDLRDAFSRVKTFFQMKDQLDNLLLKESLLEDFKGYLGCQALSEMIQFYLEEVMPQAENQDP
DIKAHVNSLGENLKTLRLRLRRCHRFLPCENGGGSGGKSKAVEQVKNAFNKLQEKGIYKAMSEFD
IFINYIEAYMTMKIRN (SEQ ID NO:39)

anti-mPD1: with a subtype being hIgG1.8 and a number being R1049, a monoclonal antibody control of mouse anti-PD-1, a heavy chain amino acid sequence was as set forth in SEQ ID NO:40, and a light chain amino acid sequence was as set forth in SEQ ID NO:41.

One of heavy chain sequences of R1049 was as follows.

QVQLKESGPGLVQPSQTLSLTCTVSGFSLTSYNVHWVRQPPGKGLEWMGGMRYNEDTSYN
SALKSRLSISRDTSKNQVFLKMNSLQTDDTGTYYCTRDAVYGGYGGWFAYWGQGTLVTVSSAST
KGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPEAAGGPSVFLFPPKPKDTL
MISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYTQKSLSLSP
GK (SEQ ID NO:40)。

### One of light chain sequences of R1049 was as follows.

DTVLTQSPALAVSLGQRVTISCKASETVSSSMYSYIHWYQQKPGQQPKLLIYRASNLESGVPA
RFSGSGSGTDFTLTIDPVEADDVATYFCQQSWNPWTFGGGTKLELKRTVAAPSVFIFPPSDEQLKS
GTASVVCLLNNFYPREAKVQWKVDNALQSGNSQESVTEQDSKDSTYSLSSTLTLSKADYEKHKVY
ACEVTHQGLSSPVTKSFNRGEC (SEQ ID NO:41)

Instruments and devices: Electronic balance (Shanghai Sunny Hengping Instrument, JA12002), vernier caliper (Shanghai Meinaite Industrial Co., Ltd., MNT-150T), microscope (Chongqing Opte Instrument Co., Ltd., BDS200), medical centrifuge (Hunan CENCE Laboratory Development Co., Ltd., L530R), large capacity high-speed desk centrifuge (Hunan CENCE Laboratory Development Co., Ltd., H2050R), digital constant temperature water bath (PRS Machinery Co., Ltd., HH-S), CO₂ incubator (Panasonic Healthcare Co., Ltd., Japan, MCO-18AC), biosafety cabinet (Guangzhou Rivers Experimental Technology Co., Ltd., ACZ-451), cell counter (Shanghai Ruiyu Cell counter Co., Ltd., IC1000), and flow cytometer (Beckman, A00-1-1102).

### Test method

Cell culture: the mouse colon cancer cells (CT-26) were cultured in an RPMI 1640 culture medium containing 10% fetal bovine serum (FBS) and 1% penicillin-streptomycin (1:1), and the culture medium was placed in a 37 °C 5% CO₂ incubator for culture.

Inoculation: CT-26 cells at a logarithmic growth phase were collected and washed twice with DPBS, and a cell concentration was regulated to 1×10⁶ cells/mL using DPBS. Female Balb/c-hPD1 mice were taken, and CT-26 cells were inoculated subcutaneously at a volume of 0.1 mL/mouse, equivalent to 1×10⁵/mouse.

Administration: the day of inoculation was recorded as Day 0 (D0). When an average tumor volume reached 200-300 mm³, mice might be randomly grouped based on tumor volume, ensuring that each group had an identical or similar average tumor volume. On Day 16 (D16) of the experiment, the mice were randomly grouped into 4 groups based on the tumor volume, with 6 mice in each group, and then the mice started to be administered (administration regimen shown in Table 21 below).

**Table 21 Administration dose, administration route, and frequency of CT-26 tumor model**

| Group | Dosage (mg/kg) | Administration volume (µL/g) | Administration route | Administration frequency |
|---|---|---|---|---|
| R0862 | 7.8 | 10 | i.p. | Q3D*3 |
| R0991(anti-mPD1-IL10M) | 10 | 10 | i.p. | Q3D*3 |
| anti-mPD1 | 7.8 | 10 | i.p. | Q3D*3 |
| wt-IL10 | 5 | 10 | i.p. | Q3D*3 |

| | | | | |
|---|---|---|---|---|
| Note: Dosing Volume, adjusted according to animal body weight (10µL/g); i.p., intraperitoneal injection; Q3D, administer once every 3 days; if weight loss exceeded 15% during administration, the administration regimen would be adjusted. | | | | |

### TILs analysis

1) Materials: on the day following the final drug administration, the mice were euthanized, then tumors and spleens were taken, weighed, immersed in pre-cooled RPMI 1640, and labeled.
2) Tumor treatment: tumor scissors were used to cut the tumor tissue into small pieces, then the tumor tissues were ground into a paste using a glass plate, and red blood cell lysis was performed as needed.
3) Spleen treatment: the spleens were ground using a glass plate and passed through a cell filter.
4) Digestive juice preparation: 2.35 mL of a RPMI 1640 culture medium was added to a centrifuge tube, and then 100 µL of an enzyme D, 50 µL of an enzyme R, and 12.5 µL of an enzyme A were added, respectively. (For TILs analysis, it was recommended to reduce the amount of the enzyme R to 20%, e.g., adding 10 µL of an enzyme R to 2.5 mL of digestive juice)
5) Digestion: 1 g of the ground tissue was added to the prepared digestive juice and digested at 37 °C for 40 min, and an appropriate amount of 1640 culture medium was added to terminate digestion.
6) Filtration: the supernatant was removed from the digested tissue and placed in a cell strainer; and 5-10 mL of 1640 culture medium was then added to the tissue fragments for washing, and then the mixture was placed in the cell strainer for complete filtration.
7) Centrifugation: centrifugation was performed for 7 min at 350 xg, the supernatant was discarded, and digested cells might be obtained.
8) Red blood cell lysis: 2-3 mL of a red blood cell lysis solution was added to tumor and spleen samples and allowed to stand for 3-5 min, and then sufficient DPBS was added to terminate red blood cell lysis.
9) Counting: the cells were centrifuged for 5 min at 350 xg, resuspended, and then counted using a cell counter.
10) Blocking: an appropriate amount of the cells was taken from each cell suspension and centrifuged for 5 min at 350 xg, the supernatant was discarded, the cells were resuspended, and blocking was performed for 20-30 min by adding 2% serum.
11) Staining: after blocking, centrifugation was performed for 5 min at 350 xg, and the supernatant was discarded. Before staining, a live-or-dead dye was added, and the cells were well mixed, incubated at room temperature in the dark for 20-30 min, and then centrifuged for 5 min at 350 xg. A target mixed antibody was added to a sample tube, the corresponding antibody was added to a single-stain well, and the cells were well mixed.
12) Incubation: incubation was performed at 4 °C for 30 min, and then centrifugation was performed for 5 min at 350 xg.
13) Washing: the supernatant was discarded, washing was performed once, and centrifugation was performed for 5 min at 350 xg.
14) After the final washing, the supernatant was discarded, and the sample was gently flicked to completely dissociate the precipitate.
15) 200 µL of a Foxp3 fixation/membrane breaking working solution was added to each well. It better ensured that the cells were completely resuspended in the added solution, and incubated at 2-8 °C or room temperature in the dark for 40 min. (Mouse samples might be incubated in the dark at 2-8 °C for up to 18 hours).
16) The samples were centrifuged at room temperature at 300-400 g for 5 min, and then the supernatant was discarded.
17) 200 µL of a 1X membrane breaking solution was added to each well, the samples were centrifuged at 300-400 g for 5 min at room temperature, and then the supernatant was discarded.
18) The membrane breaking solution was washed again, with 200 µL/well; and the samples were centrifuged at 300-400 g for 5 min, and then the supernatant was discarded.
19) The precipitate was resuspended, and the volume was adjusted to approximately 100 µL using the 1X membrane breaking solution.
20) Without washing, a recommended amount of a directly-coupled antibody was added to detect an intracellular antigen, and incubated at room temperature in the dark for at least 30 min.
21) 200 µL of a 1X membrane breaking solution was added to each well, the samples were centrifuged at 300-400 g for 5 min at room temperature, and then the supernatant was discarded.
22) The membrane breaking solution was washed again, with 200 µL/well; and the samples were centrifuged at 300-400 g for 5 min, and then the supernatant was discarded.
23) The stained cells were resuspended in an appropriate volume of a flow cytometry staining solution, and analysis was performed by flow cytometry.

Data computation, statistics, and analysis:
Independent sample T-tests might be used to compare two groups. For comparisons involving three or more groups, One-Way ANOVA was used. If the F-value indicated a significant difference, post-hoc analyses among a plurality of groups might be performed. Data were processed by using Prism GraphPad, and when p<0.05, it indicated a statistically significant difference.

**Table 22 Proportion of various immune cell subgroups in tumor tissue**

| Group | mCD45+(%) | mCD8+(%) | mCD4+(%) |
|---|---|---|---|
| R0862 | 9.6±2.7 | 3.0 %±1.0 | 1.0 %±0.5 |
| wtIL10 | 27.6±10.2* | 6.1 %±1.7# | 0.9 %±0.7 |
| Anti-mPD1 | 15.4±5.9 | 4.5 %±1.5 | 1.6 %±0.7 |
| R0991 | 30.7±16.8** | 6.9 %±2.4## | 1.2 %±0.4 |

| | | | |
|---|---|---|---|
| Note: */#, compared with the R0862 (isotype control) group, the wtIL10 group showed a statistically significant difference, p<0.05; **/##, compared with the R0862 (isotype control) group, the R0991 group showed a statistically significant difference, p<0.01. | | | |

### Experimental results:

The R0991 might significantly increase the proportion of CD45+cells and CD8+T cells in tumor-infiltrating lymphocytes, with no apparent effect on CD4+T cells (see Table 22).

To sum up, the current clinical use of IL-10 protein had safety concerns. Clinical IL-10 drug molecules lacked targeting properties, making them prone to off target and resulting in adverse reactions. Most IL-10 antibody fusion proteins currently in development used antibody targeting to deliver IL10 to a local environment to achieve the effect. However, these fusion molecules often had a normal IL10 dimeric structure, and the side effects associated with the IL10 terminus remained a challenging issue.

The safety of the anti-PD1-IL10 fusion protein provided by the embodiments of the present disclosure was significantly improved. Compared to an IL10 dimer (Fc-IL-10), the anti-PD1-IL-10 fusion protein of the present disclosure showed no hematologic toxicity or liver injury during administration, facilitating the solving of the problems of limited therapeutic efficacy caused by low dosages due to high hematologic toxicity of IL10 treatment in clinical.

Moreover, the anti-PD1-IL10 fusion protein provided in the examples of the present disclosure could activate the function of the exhausted CD8+T cells, inhibit apoptosis, promote cell proliferation and cytokine secretion, improve cytotoxic activity, and show potent antitumor activity in the mouse tumor models. Therefore, the use of the anti-PD1-IL10 fusion protein provided in the examples of the present disclosure to reverse the exhausted CD8+T cells in a tumor microenvironment was a highly promising therapeutic strategy.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure shall fall within the scope of protection of the present disclosure.

### Industrial Applicability

The anti-PD1-IL10 fusion protein provided in the present disclosure has a good targeting effect, anti-tumor effect, and safety, and may be used to treat tumors or inflammatory diseases. Therefore, the anti-PD1-IL10 fusion protein provided in the present disclosure has excellent practical performance and wide market application prospects.

## Claims

1. A fusion protein, comprising a PD1 binding unit portion and an Interleukin-10 (IL10) unit portion, wherein the PD1 binding unit portion is an anti-PD1 antibody, and the IL10 unit portion is an IL10 polypeptide; the anti-PD1 antibody comprises a heavy chain variable region and a light chain variable region; and the heavy chain variable region comprises HCDR1, HCDR2, and HCDR3 in a variable region having at least 90% sequence identity to SEQ ID NO:1, 3, 5, or 7, and/or the light chain variable region comprises LCDR1, LCDR2, and LCDR3 in a variable region having at least 90% sequence identity to SEQ ID NO:2, 4, 6, or 8.

2. The fusion protein according to claim 1, wherein the anti-PD1 antibody is selected from:
A. the heavy chain variable region of the anti-PD1 antibody comprises the HCDR1, HCDR2, and HCDR3 in SEQ ID NO:1, and the light chain variable region of the anti-PD1 antibody comprises the LCDR1, LCDR2, and LCDR3 in SEQ ID NO:2;
B. the heavy chain variable region of the anti-PD1 antibody comprises the HCDR1, HCDR2, and HCDR3 in SEQ ID NO:3, and the light chain variable region of the anti-PD1 antibody comprises the LCDR1, LCDR2, and LCDR3 in SEQ ID NO:4;
C. the heavy chain variable region of the anti-PD1 antibody comprises the HCDR1, HCDR2, and HCDR3 in SEQ ID NO:5, and the light chain variable region of the anti-PD1 antibody comprises the LCDR1, LCDR2, and LCDR3 in SEQ ID NO:6;
or
D. the heavy chain variable region of the anti-PD1 antibody comprises the HCDR1, HCDR2, and HCDR3 in SEQ ID NO:7, and the light chain variable region of the anti-PD1 antibody comprises the LCDR1, LCDR2, and LCDR3 in SEQ ID NO:8;
optionally, the HCDR1, HCDR2, HCDR3, LCDR1, LCDR2, and LCDR3 are defined by a Kabat numbering system, or defined by an IMGT numbering system, or defined by a Chothia numbering system, or defined by a Contact numbering system, or defined by an AbM numbering system;
optionally, the HCDR1, HCDR2, HCDR3, LCDR1, LCDR2, and LCDR3 are defined by the Kabat numbering system;
optionally, wherein, in the heavy chain variable region of the anti-PD1 antibody, the HCDR1 comprises an amino acid sequence of SEQ ID NO:9, the HCDR2 comprises an amino acid sequence of SEQ ID NO:10, 15, or 16, and the HCDR3 comprises an amino acid sequence of SEQ ID NO:11; and the LCDR1 of the light chain variable region comprises an amino acid sequence of SEQ ID NO:12, the LCDR2 comprises an amino acid sequence of SEQ ID NO:13, and the LCDR3 comprises an amino acid sequence of SEQ ID NO:14; and
optionally, the anti-PD1 antibody comprises the HCDR1, HCDR2, and HCDR3 shown in SEQ ID NO:9, SEQ ID NO:10, and SEQ ID NO:11, respectively, as well as the LCDR1, LCDR2, and LCDR3 shown in SEQ ID NO:12, SEQ ID NO:13, and SEQ ID NO:14, respectively.

3. The fusion protein according to claim 1 or 2, wherein the anti-PD1 antibody is a humanized antibody, a mouse-derived antibody, or a chimeric antibody; and
optionally, the antibody is a full-length antibody or is an antigen binding fragment selected from any one of F(ab')2, Fab'-SH, Fab', Fab, scFab, dsFv, (dsFv)2, Fv, and scFv.

4. The fusion protein according to any one of claims 1 to 3, wherein the heavy chain variable region of the anti-PD1 antibody comprises an amino acid sequence having at least 90% sequence identity to SEQ ID NO:1, 3, 5, or 7, and/or the light chain variable region comprises an amino acid sequence having at least 90% sequence identity to SEQ ID NO:2, 4, 6, or 8;
optionally,
(a) the heavy chain variable region comprises an amino acid sequence of SEQ ID NO:1, and the light chain variable region comprises an amino acid sequence of SEQ ID NO:2;
(b) the heavy chain variable region comprises an amino acid sequence of SEQ ID NO:3, and the light chain variable region comprises an amino acid sequence of SEQ ID NO:4;
(c) the heavy chain variable region comprises an amino acid sequence of SEQ ID NO:5, and the light chain variable region comprises an amino acid sequence of SEQ ID NO:6; or
(d) the heavy chain variable region comprises an amino acid sequence of SEQ ID NO:7, and the light chain variable region comprises an amino acid sequence of SEQ ID NO:8; and
optionally, the anti-PD1 antibody comprises the heavy chain variable region shown in SEQ ID NO:1 and the light chain variable region shown in SEQ ID NO:2.

5. A fusion protein, comprising a PD1 binding unit portion and an IL10 unit portion, wherein the PD1 binding unit is an anti-PD1 antibody, and the IL10 unit portion is an IL10 polypeptide;
(a) the anti-PD1 antibody comprises a heavy chain variable region and a light chain variable region, the heavy chain variable region has at least 95% sequence identity to SEQ ID NO:1, and the light chain variable region has at least 95% sequence identity to SEQ ID NO:2;
(b) the anti-PD1 antibody comprises a heavy chain variable region and a light chain variable region, the heavy chain variable region has at least 95% sequence identity to SEQ ID NO:3, and the light chain variable region has at least 95% sequence identity to SEQ ID NO:4;
(c) the anti-PD1 antibody comprises a heavy chain variable region and a light chain variable region, the heavy chain variable region has at least 95% sequence identity to SEQ ID NO:5, and the light chain variable region has at least 95% sequence identity to SEQ ID NO:6;
(d) the anti-PD1 antibody comprises a heavy chain variable region and a light chain variable region, the heavy chain variable region has at least 95% sequence identity to SEQ ID NO:7, and the light chain variable region has at least 95% sequence identity to SEQ ID NO:8; or
(e) the anti-PD1 antibody is an antibody that competitively binds to a PD1 antigen or binds to the same epitope as the anti-PD1 antibody described in (a), (b), (c), or (d); and
optionally, the antibody comprises the heavy chain variable region shown in SEQ ID NO:1 and the light chain variable region shown in SEQ ID NO:2.

6. The fusion protein according to any one of claims 1 to 5, wherein the anti-PD1 antibody comprises a constant region;
optionally, a heavy chain constant region of the antibody is selected from heavy chain constant regions of IgG1, IgG2, IgG3, and IgG4, and/or a light chain constant region of the antibody is selected from a κ or λ chain constant region;
optionally, the species of the constant region is derived from mouse or human;
optionally, the heavy chain constant region comprises an amino acid sequence having at least 90% sequence identity to SEQ ID NO:19, and/or the light chain constant region comprises an amino acid sequence having at least 90% sequence identity to SEQ ID NO:20;
optionally, a heavy chain of the anti-PD1 antibody comprises an amino acid sequence having at least 90% sequence identity to SEQ ID NO:17, and a light chain of the anti-PD1 antibody comprises an amino acid sequence having at least 90% sequence identity to SEQ ID NO:18; and
optionally, the anti-PD1 antibody comprises the heavy chain shown in SEQ ID NO:17 and the light chain shown in SEQ ID NO:18.

7. The fusion protein according to any one of claims 1 to 6, wherein the IL10 polypeptide is a natural IL10 polypeptide or a modified IL10 polypeptide;
optionally, the modified IL10 polypeptide is in a monomer form, and compared to the natural IL10 polypeptide, in the modified IL10 polypeptide, a spacer peptide is inserted between any two adjacent amino acids, wherein the spacer peptide is a flexible peptide;
optionally, one of the two adjacent amino acids is located at Position 115, Position 116, Position 117, Position 118, or Position 119;
optionally, the spacer peptide is located between the Position 116 and the Position 117;
optionally, the spacer peptide is located between the Position 115 and the Position 116;
optionally, the spacer peptide is located between the Position 117 and the Position 118;
optionally, the spacer peptide is selected from at least one of GGGSGG, GGGSG, (GS)n, (GGGS)n, (GSGGS)n, or (GGGGS)n, wherein n is any integer from 1 to 10;
optionally, an amino acid sequence of the spacer peptide is shown in SEQ ID NO:21;
optionally, compared to the natural IL10 polypeptide, the modified IL10 polypeptide has a deletion of n amino acids at an N-terminus, wherein n is any integer from 1 to 12;
optionally, compared to the natural IL10 polypeptide, the modified IL10 polypeptide has a deletion of 2 amino acids at the N-terminus;
optionally, the IL10 polypeptide comprises an amino acid sequence having at least 90% sequence identity to SEQ ID NO:22 or 23; and
optionally, an amino acid sequence of the IL10 polypeptide is shown in SEQ ID NO:22.

8. The fusion protein according to any one of claims 1 to 7, wherein the IL10 polypeptide is directly linked to the heavy chain or light chain of the anti-PD1 antibody or linked to the heavy chain or light chain of the anti-PD1 antibody via a linker peptide;
optionally, an N-terminus of the IL10 polypeptide is linked to a C-terminus of the light chain of the anti-PD1 antibody via the linker peptide;
optionally, the fusion protein comprises two identical first chains shown in Formula P1 and two identical second chains shown in Formula P2;
P1: [light chain of anti-PD1 antibody]-[linker peptide]-[IL10 polypeptide] P2: heavy chain of anti-PD1 antibody;
optionally, in Formula P1, the linker peptide is a flexible linker peptide;
optionally, the linker peptide is selected from at least one of (GGGGS)nG, (GGGGS)n, (GS)n, (GGGS)n, (GSGGS)n, or (GGGGSG)n, wherein n is any integer from 1 to 10;
optionally, an amino acid sequence of the linker peptide is shown in SEQ ID NO:24;
optionally, the fusion protein comprises a first chain of an amino acid sequence having at least 90% sequence identity to SEQ ID NO:25 and a second chain of an amino acid sequence having at least 90% sequence identity to SEQ ID NO:17; and
optionally, the fusion protein comprises two first chains with amino acid sequences shown in SEQ ID NO:25 and two second chains with amino acid sequences shown in SEQ ID NO:17.

9. The fusion protein according to any one of claims 1 to 8, wherein the fusion protein has at least one of the following characteristics:
A. the fusion protein is able to specifically bind to human PD1; optionally, the fusion protein is able to bind to the human PD1 with an EC50 value ≤10 nM, wherein the EC50 value is determined by an FACS method;
B. the fusion protein has low hematotoxicity or no hematotoxicity;
C. the fusion protein is able to improve killing activity of a CD8+T cell;
D. the fusion protein is able to inhibit growth of a tumor; or
E. the fusion protein has good stability.

10. A nucleic acid, encoding the fusion protein according to any one of claims 1 to 9.

11. A vector, comprising the nucleic acid according to claim 10.

12. A cell, comprising the nucleic acid according to claim 10 or the vector according to claim 11.

13. A drug composition, comprising the fusion protein according to any one of claims 1 to 9, the nucleic acid according to claim 10, the vector according to claim 11, or the cell according to claim 12, wherein optionally, the drug composition further comprises one or more pharmaceutically acceptable carriers, diluents, or excipients.

14. Use of the fusion protein according to any one of claims 1 to 9, the nucleic acid according to claim 10, the vector according to claim 11, the cell according to claim 12, or the drug composition according to claim 13 in preparation of drugs for treating or preventing tumors or inflammatory diseases, wherein
optionally, the tumors are tumors with high PD-L1 expression;
optionally, the tumors are selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer; and
optionally, the tumors are selected from non-small cell lung cancer, renal cell carcinoma, and gastric cancer.

15. A method for treating diseases, comprising applying an effective dosage of the fusion protein according to any one of claims 1 to 9, the nucleic acid according to claim 10, the vector according to claim 11, the cell according to claim 12, or the drug composition according to claim 13 to a subject in need, wherein
optionally, the diseases are tumors or inflammatory diseases;
optionally, the tumors or inflammatory diseases are diseases with high human PD1 and/or PDL1 expression;
optionally, the tumors are tumors with high PDL1 expression;
optionally, the tumors are selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer; and
optionally, the tumors are selected from non-small cell lung cancer, renal cell carcinoma, and gastric cancer.

16. A kit, comprising the fusion protein according to any one of claims 1 to 9, the nucleic acid according to claim 10, the vector according to claim 11, the cell according to claim 12, or the drug composition according to claim 13; and
optionally, the kit is used for detecting or determining human PD1.

17. A method for preparing the fusion protein according to any one of claims 1 to 9, wherein the method comprises steps of culturing the cell according to claim 12, and then obtaining the fusion protein through separation and purification.
